(19) **Europäisches Patentamt**
**European Patent Office**
**Office européen des brevets**

(11) **EP 2 734 233 B1**

(12) **EUROPEAN PATENT SPECIFICATION**

(45) Date of publication and mention
of the grant of the patent:
**01.11.2017 Bulletin 2017/44**

(21) Application number: **12814244.5**

(22) Date of filing: **18.07.2012**

(51) Int Cl.:
*A61K 39/395* [(2006.01)]          *C12Q 1/68* [(2006.01)]
*C12Q 1/02* [(2006.01)]

(86) International application number:
**PCT/US2012/047240**

(87) International publication number:
**WO 2013/012947 (24.01.2013 Gazette 2013/04)**

(54) **METHODS OF PREDICTING HOST RESPONSIVENESS TO CANCER IMMUNOTHERAPIES BY EX VIVO INDUCTION OF LEUKOCYTE-FUNCTION-ASSOCIATED MRNAS**

VERFAHREN ZUR VORHERSAGE DES ANSPRECHENS EINES WIRTES AUF KREBSIMMUNTHERAPIEN DURCH EX-VIVO-INDUKTION VON MIT LEUKOZYTENFUNKTIONEN ASSOZIIERTEN MRNAS

MÉTHODES DE PRÉVISION DE LA SENSIBILITÉ D'UN HÔTE AUX IMMUNOTHÉRAPIES DU CANCER PAR INDUCTION EX VIVO D'ARNM ASSOCIÉS AUX FONCTIONS LEUCOCYTAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.07.2011 US 201161509030 P**

(43) Date of publication of application:
**28.05.2014 Bulletin 2014/22**

(73) Proprietors:
• **Hitachi Chemical Co., Ltd.**
**Chiyoda-ku**
**Tokyo 100-6606 (JP)**
• **Hitachi Chemical Co. America, Ltd.**
**San Jose, California 95131 (US)**
• **Shin Yokohama Kato Clinic**
**Kanagawa 222-0033 (JP)**

(72) Inventors:
• **MITSUHASHI, Masato**
**Irvine, CA 92614 (US)**
• **KATO, Yoichi**
**Yokohama**
**Kanagawa 222-0033 (JP)**

(74) Representative: **Vossius & Partner**
**Patentanwälte Rechtsanwälte mbB**
**Siebertstrasse 3**
**81675 München (DE)**

(56) References cited:
**WO-A1-2006/116721      WO-A1-2006/133399**
**WO-A1-2009/083653      WO-A1-2011/084333**
**WO-A2-2009/070442      US-A1- 2006 008 807**
**US-A1- 2007 292 448     US-A1- 2009 011 410**
**US-A1- 2009 111 128**

• **MITSUHASHI ET AL: "Ex vivo simulation of leukocyte function: Stimulation of specific subset of leukocytes in whole blood followed by the measurement of function-associated mRNAs", JOURNAL OF IMMUNOLOGICAL METHODS, ELSEVIER SCIENCE PUBLISHERS B.V.,AMSTERDAM, NL, vol. 363, no. 1, 15 December 2010 (2010-12-15), pages 95-100, XP027536029, ISSN: 0022-1759 [retrieved on 2010-10-15]**

**Description**

BACKGROUND

Field of the Invention

The present invention is defined by the claims. Embodiments of the present invention relate generally to methods for characterizing and/or predicting the responsiveness of an individual to certain therapeutic agents. More specifically, embodiments disclosed herein relate to a method for enabling a medical professional to recommend a cancer immunotherapy to a subject as defined in the claims.

Description of the Related Art

The primary therapeutic regimens for many cancers is surgery, chemotherapy, radiation, or combinations thereof. Unfortunately, these techniques are not well-suited to certain patients. A patient may be too ill to undergo the stresses of surgery or chemotherapy. Other cancer patients may have tumors that are inoperable or are non-responsive to chemotherapeutic agents or radiation therapy. Recently, cancer immunotherapy methods have entered the scope of feasible treatments for a variety of cancers.

Cancer patients are often faced with a short window of time after diagnosis in which a given therapy may be effective. If this window has closed, a patient's life expectancy and/or quality of life may be significantly decreased. The closure of this window may also render some treatment options moot, while making others that would have been less desirable become first line treatment regimens. In other words, time is of the essence for most cancer patients, and knowing if a certain therapy or combination of therapies would be effective against a cancer would increase the likelihood that a patient could be successfully treated. For example, WO 2011/084333 describes methods for predicting the efficacy of anti-cancer therapeutic regimen based on the immune function of a subject. WO 2006/133399 discloses a method for detecting significant introduction of a variety of TNF superfamily subtype and chemokine mRNA in blood leukocytes when whole blood is exposed to heat-aggregated IgG or anti-T-cell receptor antibodies as a model of leukocyte function associated mRNAs. WO 2006/116721 relates to the use of the method described in WO 2006/133399 for assessing the potential effectiveness of a dietary component against cancer or an autoimmune disorder. WO 2009/070442 is directed to a method for determining whether a rheumatoid arthritis patent is likely to respond to an anti-TNF therapy. WO 2009/083653 provides methods for determining or predicting efficacy of treatments of these diseases by comparing mRNA expression levels of different markers including FOXP3, GITR, GATA3, IFN-gamma and IL-5. Mitsuhashi (2010), Journal of Immunological Methods, 363:95-100 describes the ex vivo simulation of leukocyte function. In more detail, the stimulation of a specific subset of leukocytes in whole blood followed by the measurement of function-associated mRNAs is described. Thus there is still a need for methods to determine which individuals are likely to be responsive to a particular cancer therapy. Moreover, there is also a need for monitoring the ongoing efficacy of cancer therapy in an individual receiving the therapy on an ongoing basis.

SUMMARY

The present invention is defined by the claims. Several methods described herein as well as the claims address the need for methods for predicting responsiveness of patients to cancer immunotherapy. In accordance with the present invention there is provided a method for enabling a medical professional to recommend a cancer immunotherapy to a subject comprisingexposing a first sample of whole blood to a first leukocyte-activating agent in a solvent and exposing a second sample of whole blood to a second leukocyte-activating agent in said solvent, said exposing being for an amount of time sufficient for said leukocyte-activating agents to alter the expression of three or more leukocyte-function-associated mRNAs, wherein said first and second leukocyte-activating agents are associated with immune functions involved in cancer immunotherapy and wherein said leukocyte-function-associated mRNA is selected from the group consisting of interferon-gamma, tumor necrosis factor superfamily-1, tumor necrosis factor superfamily-2, tumor necrosis factor superfamily-5, interleukin-10, transforming growth factor-beta, CTL-associated protein 4, programmed cell death 1, forkhead box P3, granulocyte macrophage-colony stimulating factor, vascular endothelial growth factor, interleukin-8, CCL chemokine-8, CXCL chemokine-3, and interleukin 2; exposing a third sample of whole blood to said solvent without said first or second leukocyte-activating agents for said amount of time; quantifying the expression of said three or more leukocyte-function- associated mRNAs by measuring the amount of mRNA encoding said three or more leukocyte-function-associated mRNAs in said first, second and third whole blood samples; predetermining a parameter by multivariate analysis for each combination of said first and second leukocyte-activating agents and said three or more leukocyte-function-associated mRNAs using a plurality of patients in one or more of a plurality of groups with known clinical responsiveness to cancer immunotherapy; calculating a predictor value for each individual for each of said clinical response groups; and using said predetermined values as a comparative reference for said predictor values to categorize said subject within said clinical response group and enable said medical professional to recommend a cancer immunotherapy for said subject. Also described herein is a method for enabling a medical professional to recommend a cancer immunotherapy to a subject comprising obtaining at least a first, a second, and a third sample of whole blood from the subject, exposing the first sample of whole blood to a first leukocyte-activating agent in a solvent and exposing the second sample of whole blood to a second leukocyte-activating agent in said solvent, said exposing being for an amount of time sufficient for said leukocyte-activating agents to alter the expression of three or more leukocyte-function-associated mRNAs, wherein said first and second leukocyte-activating agents are associated with immune functions involved in cancer immunotherapy, exposing the third sample of whole blood to said solvent without said first or second leukocyte-activating agents for said amount of time, quantifying the expression of said three or more leukocyte-function-associated mRNAs by measuring the amount of mRNA encoding said three or more leukocyte-function-associated mRNAs in said first, second and third whole blood samples, predetermining a parameter by multivariate analysis for each combination of said first and second leukocyte-activating agents and said three or more leukocyte-function-associated mRNAs using a plurality of patients in one or more of a plurality of groups with known clinical responsiveness to cancer immunotherapy, calculating a predictor value for each individual for each of said clinical response groups, and using said predetermined values as a comparative reference for said predictor values to categorize said subject within said clinical response group and enable said medical professional to recommend a cancer immunotherapy for said subject.

There is also described herein a method for enabling a medical professional to recommend a cancer immunotherapy to a subject comprising exposing a first sample of whole blood obtained from a subject to a first leukocyte-activating agent in a solvent and exposing a second sample of whole blood obtained from the subject to a second leukocyte-activating agent in the solvent for an amount of time sufficient for the leukocyte-activating agents to alter the expression of three or more leukocyte-function-associated mRNAs that are associated with immune functions involved in cancer immunotherapy, exposing a third sample of whole blood obtained from the subject to the solvent without the first or second leukocyte-activating agents for the amount of time, and quantifying the expression of the three or more leukocyte-function-associated mRNAs by measuring the amount of mRNA encoding the three or more leukocyte-function-associated mRNAs in the first, second and third whole blood samples. A parameter may be determined by mathematical analysis for each combination of the first and second leukocyte-activating agents and the three or more leukocyte-function-associated mRNAs using a plurality of patients in one or more of a plurality of groups with known clinical responsiveness to cancer immunotherapy. Additionally, a predictor value is calculated for each individual for each of the clinical response groups, and the predictor values are compared with the predetermined values in order to categorize the subject within the clinical response group to enable the medical professional to recommend a cancer immunotherapy for the subject.

There is also provided herein a method for enabling a medical professional to recommend a cancer immunotherapy to a subject comprising obtaining at least a first, a second, and a third sample of whole blood from the subject, exposing the first sample of whole blood to a first leukocyte-activating agent in a solvent and exposing the second sample of whole blood to a second leukocyte-activating agent in the solvent, the exposing being for an amount of time sufficient for the leukocyte-activating agents to alter the expression of three or more leukocyte-function-associated mRNAs, wherein the first and second leukocyte-activating agents are associated with immune functions involved in cancer immunotherapy, exposing the third sample of whole blood to the solvent without the first or second leukocyte-activating agents for the amount of time, quantifying the expression of the three or more leukocyte-function-associated mRNAs by measuring the amount of mRNA encoding the three or more leukocyte-function-associated mRNAs in the first, second and third whole blood samples, predetermining a parameter by multivariate analysis for each combination of the first and second leukocyte-activating agents and the three or more leukocyte-function-associated mRNAs using a plurality of patients in one or more of a plurality of groups with known clinical responsiveness to cancer immunotherapy, calculating a predictor value for each individual for each of the clinical response groups, and indicating to the medical professional when the subject is categorized in any of the clinical response groups, thereby enabling the medical professional to recommend a cancer immunotherapy for the subject.

There is also described herein an ex vivo method for characterizing the potential responsiveness of an individual to cancer immunotherapy comprising, exposing a first sample of whole blood to a first leukocyte-activating agent in a solvent and exposing a second sample of whole blood to a second leukocyte-activating agent in the solvent, the exposing being for an amount of time sufficient for the leukocyte-activating agents to alter the expression of three or more leukocyte-function-associated mRNAs, exposing a third sample of whole blood to the solvent without the first or second leukocyte-activating agents for the amount of time, quantifying the expression of the three or more leukocyte-function-associated mRNAs by measuring the amount of mRNA encoding the three or more leukocyte-function-associated mRNAs in the first, second and third whole blood samples, and characterizing the potential responsiveness of the individual to the immunotherapy by predetermining a parameter by multivariate analysis for each combination of the first and second leukocyte-activating agents and the three or more leukocyte-function-associated mRNAs using a plurality of patients in one or more of a plurality of groups with known clinical responsiveness to cancer immunotherapy, calculating a predictor value for each individual for each of the clinical response groups, comparing the predictor values with the predetermined values in order to categorize the individual within the clinical response group; and characterizing an individual as a potential responder to cancer immunotherapy when the individual is categorized in any of the clinical response groups. The first and second leukocyte-activating agents may be associated with immune functions involved in cancer immunotherapy.

Additionally, there are described herein methods for advising a subject to undertake a cancer immunotherapy regime, comprising ordering a test of the subject's blood, the test comprising obtaining at least a first and a second sample of whole blood from the subject, exposing the first sample of whole blood to a first leukocyte-activating agent in a solvent and exposing the second sample of whole blood to a second leukocyte-activating agent in the solvent, the exposing being for an amount of time sufficient for the leukocyte-activating agents to alter the expression of three or more leukocyte-function-associated mRNAs, wherein the first and second leukocyte-activating agents are associated with immune functions involved in cancer immunotherapy; exposing a third sample of whole blood to the solvent without the first or second leukocyte-activating agents for the amount of time, quantifying the expression of the three or more leukocyte-function-associated mRNAs by measuring the amount of mRNA encoding the three or more leukocyte-function-associated mRNAs in the first, second and third whole blood samples, predetermining a parameter by multivariate analysis for each combination of the first and second leukocyte-activating agents and the three or more leukocyte-function-associated mRNAs using a plurality of patients in one or more of a plurality of groups with known clinical responsiveness to cancer immunotherapy, calculating a predictor value for each individual for each of the clinical response groups, comparing the predictor values with the predetermined values in order to categorize the individual within the clinical response group, and advising the subject to undergo a cancer immunotherapy when the subject is categorized in any of the clinical response groups.

There are also described herein methods for treating a subject having cancer based on the subject's diagnostic responsiveness to a cancer immunotherapy regime, comprising, ordering a test of the subject's blood, the test comprising obtaining at least a first and a second sample of whole blood from the subject, exposing the first sample of whole blood to a first leukocyte-activating agent in a solvent and exposing the second sample of whole blood to a second leukocyte-activating agent in the solvent, the exposing being for an amount of time sufficient for the leukocyte-activating agents to alter the expression of three or more leukocyte-function-associated mRNAs, wherein the first and second leukocyte-activating agents are associated with immune functions involved in cancer immunotherapy, exposing a third sample of whole blood to the solvent without the first or second leukocyte-activating agents for the amount of time, quantifying the expression of the three or more leukocyte-function-associated mRNAs by measuring the amount of mRNA encoding the three or more leukocyte-function-associated mRNAs in the first, second and third whole blood samples, predetermining a parameter by multivariate analysis for each combination of the first and second leukocyte-activating agents and the three or more leukocyte-function-associated mRNAs using a plurality of patients in one or more of a plurality of groups with known clinical responsiveness to cancer immunotherapy, calculating a predictor value for each individual for each of the clinical response groups, comparing the predictor values with the predetermined values in order to categorize the individual within the clinical response group, and administering to the subject a cancer immunotherapy when the subject is categorized in any of the clinical response groups.

Additionally, there are described herein methods of treating a cancer patient, the method comprising: a) obtaining at least a first, a second, and a third sample of whole blood from the patient, b) having an assay conducted on the whole blood samples from the patient, the assay comprising: i) exposing the first sample of whole blood to a first leukocyte-activating agent in a solvent and exposing the second sample of whole blood to a second leukocyte-activating agent in the solvent for an amount of time sufficient for the leukocyte-activating agents to alter the expression of three or more leukocyte-function-associated mRNAs, wherein the first and second leukocyte-activating agents are associated with immune functions involved in cancer immunotherapy; ii) exposing the third sample of whole blood to the solvent without the first or second leukocyte-activating agents for the amount of time, iii) quantifying the expression of the three or more leukocyte-function-associated mRNAs by measuring the amount of mRNA encoding the three or more leukocyte-function-associated mRNAs in the first, second and third whole blood samples; iv) predetermining a value by multivariate analysis for each combination of the first and second leukocyte-activating agents and the three or more leukocyte-function-associated mRNAs using a plurality of patients in one or more of a plurality of groups with known clinical responsiveness to cancer immunotherapy; and v) calculating a predictor value for each individual for each of the clinical response groups, c) obtaining the predictor values and predetermined values, d) comparing the predictor values with the predetermined values in order to categorize the individual within any of the groups with known clinical responsiveness to cancer immunotherapy; and e) administering a cancer immunotherapy to the patient if the comparison results in categorization of the patient in any of the clinical response groups. A nonimmunologically-based cancer therapy may be administered to the patient if the comparison results in categorization of the patient outside the clinical response groups.

There are also described herein ex vivo methods for characterizing the potential responsiveness of an individual to cancer immunotherapy comprising, exposing a first sample of whole blood to a first leukocyte-activating agent in a solvent and exposing a second sample of whole blood to a second leukocyte-activating agent in the solvent, the exposing being for an amount of time sufficient for the leukocyte-activating agents to alter the expression of three or more leukocyte-function-associated mRNAs, wherein the first and second leukocyte-activating agents are associated with immune functions involved in cancer immunotherapy, exposing a third sample of whole blood to the solvent without the first or second leukocyte-activating agents for the amount of time, quantifying the expression of the three or more leukocyte-function-associated mRNAs by measuring the amount of mRNA encoding the three or more leukocyte-function-associated mRNAs in the first, second and third whole blood samples, characterizing the potential responsiveness of the individual to the immunotherapy, wherein the characterization comprises: predetermining a parameter by multivariate analysis for each combination of the first and second leukocyte-activating agents and the three or more leukocyte-function-associated mRNAs using a plurality of patients in one or more of a plurality of groups with known clinical responsiveness to cancer immunotherapy, calculating a predictor value for each individual for each of the clinical response groups; and comparing the predictor values with the predetermined values in order to categorize the individual within the clinical response group, wherein categorization in any of the clinical response groups resulting in an individual being characterized as a responder to cancer immunotherapy.

The quantifying may comprise amplifying the three or more leukocyte-function-associated mRNAs using RT-PCR. The quantifying may comprise amplifying the three or more leukocyte-function-associated mRNAs using real time RT-PCR with primers specifically designed for amplification the three or more leukocyte-function-associated mRNAs. The quantifying may comprise measuring the induction of the leukocyte-function-associated mRNAs using northern blot.

The three or more leukocyte-function-associated mRNAs may be associated with different functional immune categories.

The cancer immunotherapy may comprise a dendritic cell vaccine. The cancer immunotherapy may comprise one or more therapeutic antibodies.

The whole blood samples may be treated with an anti-coagulant, which in some embodiments, comprises heparin.

The exposing may be performed at a temperature from about 30°C and about 42°C. Other temperatures may also be used (including ranges of temperatures). For example, the exposing may be performed at a temperature of about 37°C.

The amount of time of the exposing may be less than about 6 hours, though, longer times may also be used. For example, the amount of time may be from about 1 to about 4 hours.

The groups with known clinical responsiveness to cancer immunotherapy may be selected from the group consisting of are stable disease, partial response, overall survival, and regression free survival. Other intermediate groups may also be included (e.g., low grade progression, high grade progression, etc.).

The at least two different leukocyte-activating agents may be selected from the group consisting of phytohemaglutinin, heat-aggregated IgG, zymosan, interleukin 2, interferon alpha-2-beta, monoclonal antibody against the alpha/beta chain of the human T cell receptor, and picibanil. The at least two different leukocyte-activating agents may comprise phytohemaglutinin in combination with one or more of interferon alpha-2-beta, heat-aggregated IgG, zymosan, and a monoclonal antibody against the alpha/beta chain of the human T cell receptor. The at least two different leukocyte-activating agents may also comprise interleukin-2 in combination with one or more of interferon alpha-2-beta, heat-aggregated IgG, zymosan, phytohemaglutinin, and a monoclonal antibody against the alpha/beta chain of the human T cell receptor.

The leukocyte-function-associated mRNA may be selected from the group consisting of interferon-gamma, tumor necrosis factor superfamily-1, tumor necrosis factor superfamily-2, tumor necrosis factor superfamily-5, interleukin-10, transforming growth factor-beta, CTL-associated protein 4, programmed cell death 1, forkhead box P3, granulocyte macrophage-colony stimulating factor, vascular endothelial growth factor, interleukin-8, CCL chemokine-8, CXCL chemokine-3, and interleukin 2.

The multivariate analysis may comprise multivariate discriminant analysis. In several examples wherein the subject's potential responsiveness is determined, the methods further comprise administering a cancer immunotherapy to the subject, if indicated as appropriate by the predicted responsiveness of the subject.

Additionally, there is also described herein an ex vivo method for characterizing the potential responsiveness of an individual to cancer immunotherapy comprising, exposing a first sample of whole blood to a first leukocyte-activating agent in a solvent and exposing a second sample of whole blood to a second leukocyte-activating agent in the solvent, the exposing being for an amount of time sufficient for the leukocyte-activating agents to alter the expression of three or more leukocyte-function-associated mRNAs, wherein the first and second leukocyte-activating agents are associated with immune functions involved in cancer immunotherapy, exposing a third sample of whole blood to the solvent without the first or second leukocyte-activating agents for the amount of time, quantifying the expression of the three or more leukocyte-function-associated mRNAs by measuring the amount of mRNA encoding the three or more leukocyte-function-associated mRNAs in the first, second and third whole blood samples, and characterizing the potential responsiveness of the individual to the immunotherapy. The characterization may comprise predetermining a parameter by multivariate discriminant analysis for each combination of the first and second leukocyte-activating agents and the three or more leukocyte-function-associated mRNAs using a plurality of patients in one or more of a plurality of groups with known clinical responsiveness to cancer immunotherapy, calculating a predictor value for each individual for each of the clinical response groups and comparing the predictor values with the predetermined values in order to categorize the individual within the clinical response group, wherein categorization in any of the clinical response groups resulting in an individual being characterized as a responder to cancer immunotherapy.

The three or more leukocyte-function-associated mRNAs may be associated with different functional immune categories. In one example, the cancer immunotherapy comprises a dendritic cell vaccine, however in other examples, responsiveness to other immunotherapies is also predicted.

The whole blood samples may be treated with an anti-coagulant. In one example, the anti-coagulant comprises heparin.

The exposing may be performed at a temperature from about 30°C and about 42°C. In several example, the exposing is performed at a temperature of about 37°C.

The amount of time may be less than about 6 hours. In one example, the amount of time is from about 1 to about 4 hours.

The groups with known clinical responsiveness to cancer immunotherapy selected from the group consisting of may be stable disease, partial response, overall survival, and regression free survival.

The at least two different leukocyte-activating agents may be selected from the group consisting of phytohemaglutinin, heat-aggregated IgG, zymosan, interleukin 2, interferon alpha-2-beta, monoclonal antibody against the alpha/beta chain of the human T cell receptor, and picibanil. The at least two different leukocyte-activating agents may also comprise phytohemaglutinin in combination with one or more of interferon alpha-2-beta, heat-aggregated IgG, zymosan, and a monoclonal antibody against the alpha/beta chain of the human T cell receptor.

The at least two different leukocyte-activating agents may comprise interleukin-2 in combination with one or more of interferon alpha-2-beta, heat-aggregated IgG, zymosan, phytohemaglutinin, and a monoclonal antibody against the alpha/beta chain of the human T cell receptor.

The leukocyte-function-associated mRNA may be selected from the group consisting of interferon-gamma, tumor necrosis factor superfamily-1, tumor necrosis factor superfamily-2, tumor necrosis factor superfamily-5, interleukin-10, transforming growth factor-beta, CTL-associated protein 4, programmed cell death 1, forkhead box P3, granulocyte macrophage-colony stimulating factor, vascular endothelial growth factor, interleukin-8, CCL chemokine-8, CXCL chemokine-3, and interleukin 2.

BRIEF DESCRIPTION OF THE FIGURES

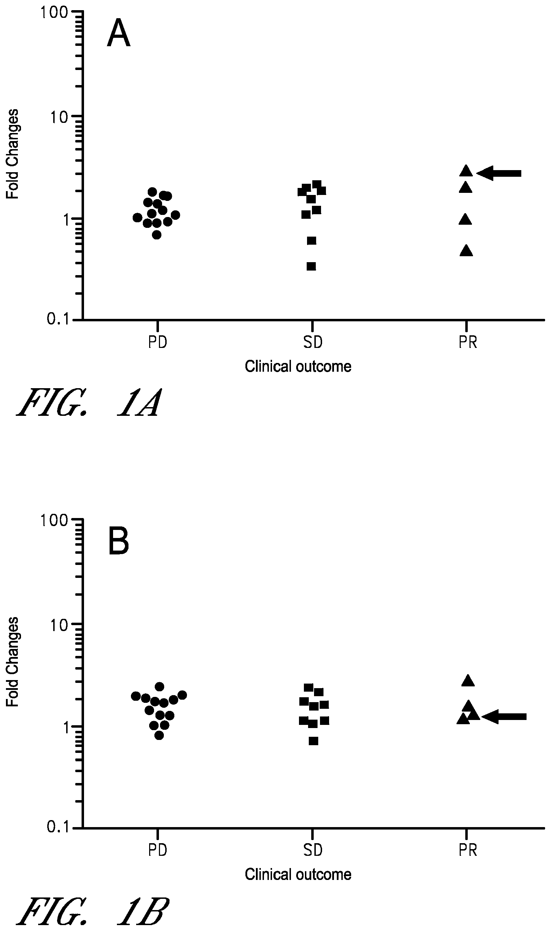

Figures 1A-1K depict ex vivo mRNA induction and clinical outcome. A-D: zymosan-induced ACTB (A), B2M (B), IFNG (C), and IL10 (D). E-H: PHA-induced ACTB (E), B2M (F), TNFSF2 (G), and IL10 (H). I-K: rl2-induced ACTB (I), B2M (J), and TNFSF2 (K), respectively. Fold changes (Y-axis) was calculated using the values of PBS as baseline. After therapy, clinical outcome (PD, SD, and PR) (x-axis) was determined as described below. Statistical analysis was performed by Student's t-test using the log values of fold changes. An arrow indicated the same patient for comparison among graphs (A-H).

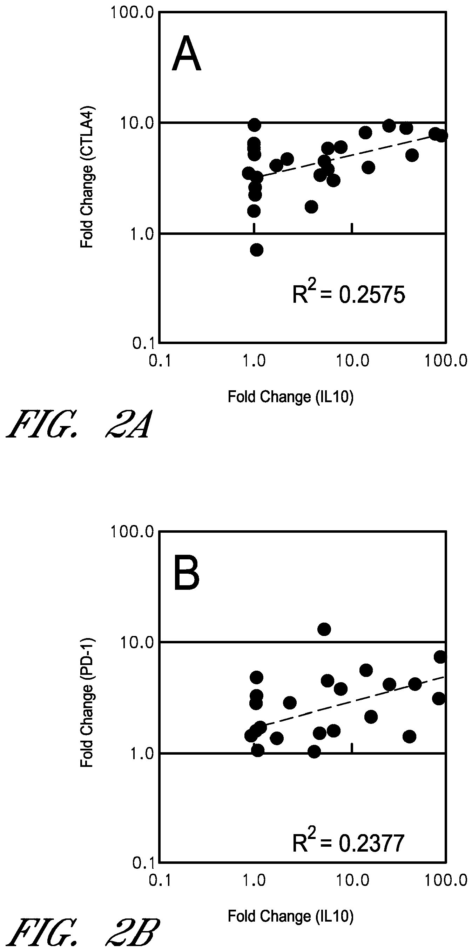

Figures 2A-2F depict mRNA-to-mRNA interaction. The fold increase of PHA-induced IL10 (x-axis) derived from 26 patients was compared with that of CTLA4 (A), PD-1 (B), FOXP3 (C), IFNG (D), IL2 (E), and TNFSF2 (F), respectively. A regression line is shown (dotted line) and $r^2$ is shown in each panel.

DETAILED DESCRIPTION

As discussed above, surgery, radiation, and chemotherapy are the typical first-line treatments for most cancers. As such, cancer immunotherapy is usually the second line of treatment. Cancer immunotherapy is the use of the immune system of a cancer patient to reject the cancer by stimulating the patient's immune system to attack the malignant tumor cells (and spare the normal cells of the patient). One mode of cancer immunotherapy employs immunization of the patient (e.g., by administering a cancer vaccine) to train the patient's immune system to recognize and destroy tumor cells. Another approach uses the administration of therapeutic antibodies, thereby recruiting the patient's immune system to destroy tumor cells. Cell-based immunotherapy is another approach, which involves immune cells such as the Natural killer Cells (NK cells), Lymphokine Activated killer cell (LAK), Cytotoxic T Lymphocytes (CTLs), Dendritic Cells (DC), etc., which are either (1) activated in vivo by administering certain cytokines (e.g., interleukins) or, 2) isolated, enriched and transfused to the patient to fight the cancer.

The keystone of the function of the immune system is the ability to discriminate between self and non-self. However, many kinds of tumor cells are more or less tolerated by the patient's own immune system, as they are the patient's own cells (e.g., they are "self"), that happen to be growing and dividing without proper regulatory control.

Despite the "self" nature of tumor cells, many kinds of tumor cells display unusual or unique antigens that are either inappropriate for a particular cell type and/or its environment, or are typically present only during a certain period in the organisms' development (e.g., fetal antigens in an adult tissue). Examples of such antigens include the glycosphingolipid GD2, which is normally only expressed at a significant level on the outer surface membranes of neuronal cells. However, GD2 is expressed on the surfaces of a wide range of tumor cells including neuroblastoma, medulloblastomas, astrocytomas, melanomas, small-cell lung cancer, osteosarcomas and other soft tissue sarcomas. Thus, markers with ectopic expression patterns similar to those of GD2 may present convenient tumor-specific targets for immunotherapies. Other kinds of tumor cells display cell surface receptors that are rare or absent on the surfaces of healthy cells, and which are responsible for activating cellular signal transduction pathways that cause the unregulated growth and division of the tumor cell. Such receptors may also present attractive targets for cancer immunotherapies.

Although state of the art cancer immunotherapies (e.g., antibody against cancer antigens, dendritic cell (DC) vaccine, infusion of in vitro activated cells, and/or inhibition of immune suppressor function, etc.) appear to demonstrate remarkable efficacy, such therapies are not necessarily beneficial for every patient. This is because it is unclear prior to the initiation of therapy which patients will respond to the therapy and which will not. If a positive response to an immunotherapy method could be accurately and confidently predicted, patients may choose immunotherapy even the treatment is experimental and expensive.

In fact, in order to discover appropriate immunological biomarkers, the international taskforce has recently published recommendations compiled from meetings organized by Society for Immunotherapy of Cancer (SITC) with US Food and Drug Administration (FDA), US National Cancer Institute (NCI), in collaboration with 10 organizations with representation of 20 countries.

There are currently four major approaches undertaken to develop predictive strategies for cancer therapies. First, identification of single nucleotide polymorphism (SNP) or mutations are used to characterize the genetic makeup of each of a patient's cells. While this is promising in terms of personalized medicine, its applicability may be of limited value in personalized cancer therapies because patients' immunological functions may be altered by chemotherapy and radiation. Second, in order to characterize unique subsets of leukocytes, immunohistochemical staining and flow cytometry are used to identify differentially expressed surface markers. Third, using microarray chip technologies, disease-specific gene expression profiles are extensively studied in whole blood or isolated leukocytes. However, the peripheral blood leukocytes used in the second and third approaches described above are generally in a resting state. In contrast, one leukocytes are attracted to (e.g., migrate to) the site of a cancer, they are fully and specifically activated. The activation is dependent on the type of leukocytes, the type of cancer antigens, and also the local environment of the cancer. Thus, the characterization of resting stage leukocytes by their surface markers or gene expression signature may not be directly linked to the function at cancer sites. Fourth, in order to assess cellular function, leukocytes are first isolated from peripheral blood, and cultured with or without specific stimuli for a period of time (typically ranging between two days to several weeks) to identify the functional changes (protein synthesis and secretion, apoptosis, cell proliferation, surface marker changes, etc.). However, such assays are complex, lengthy, and expensive, and as such, are not widely applicable as a routine diagnostic test. Thus, each of the above methodologies has some sort of limitation that makes them less than ideal for diagnostic use. In contrast to the above approach, several examples describes herein provide a simple, efficient, and accurate diagnostic assay for predicting the likelihood that a subject will respond to cancer immunotherapy.

General and Sample Types

In several examples described herein, methods are described herein for the *ex vivo* characterization of a subject's (e.g., a cancer patient's) responsiveness to administration of cancer immunotherapy. The methods may involve collection of peripheral whole blood from a potentially responsive individual and stimulation of the whole blood with one or more leukocyte-activating agents. At least two different leukocyte-activating agents may be used. Detection of changes in the mRNA encoding one or more leukocyte-function-associated mRNAs (e.g., a marker of leukocyte function) may be related to the individual's potential responsiveness to administration of the agents. The variation in response of one marker (or one marker class) may be insufficient to accurately predict responsiveness. In such examples, two, three, or more markers are quantified and analyzed in order to characterize responsiveness.

Also methods are described herein for the monitoring of the ongoing responsiveness of an individual to the administration of cancer immunotherapy. Peripheral whole blood may be collected from an individual before and after administration of a cancer immunotherapy regimen, and evaluated for changes in expression of a panel of leukocyte-function-associated mRNAs. The changes in expression are then used to evaluate the individual's ongoing responsiveness to, and the efficacy of, cancer immunotherapy.

Blood may be collected from mammals, such as humans. Whole blood samples from a subject may be used in the assay. Multiple experimental protocols directed to determining expression screening of markers responsive to a drug have been done in isolated leukocyte preparations. Such isolated populations are often preferred because the variety of lymphocytes in whole blood may preclude detection of induction of a specific mRNA in a small subset of lymphocytes. Moreover, with numerous complex biochemical interactions between the multiple types of lymphocytes, there is the possibility that use of a whole blood preparation may inhibit or modify the induction and measurement reactions. Furthermore, certain stimulatory agents (e.g., leukocyte-activating agents) which may be used herein, may interact with plasma proteins or plasma factors, and thereby exhibit decreased or reduced activity. However, when used as presented in several examples as described herein, whole blood unexpectedly produces reproducible, accurate, and physiologically relevant results that allow the characterization of the future or ongoing responsiveness of an individual to a cancer immunotherapy. However, while whole blood may be emplyoed, in other examples, blood cells separated from plasma may also be used, as well as isolated leukocyte preparations. The whole blood may be heparinized. The use of whole blood makes the procedure simple (e.g., minimally invasive), reproducible (consistent laboratory conditions and equipment are used), physiological, and easily converted to standardized methods. The collected whole blood may be stored at 4° C until the stimulation protocol.

A plurality of whole blood samples may be obtained in order to test a panel of multiple types of leukocyte-activating agents. Different solvents may be used, so samples are subdivided (or additional samples are collected) in order to properly balance the control samples versus the treated samples. The whole blood samples exposed to various stimuli may be followed by quantification of leukocyte-function-associated mRNAs. Methods related to quantification are discussed in more detail below.

Methods

The collected blood sample may be combined with one, two, three or more leukocyte-activating agents. Duplicate samples may also combined with a control agent (e.g., one that induces little or no response in the blood samples). The control agent ma be the same solvent used to carry the leukocyte-activating agent(s). The control agent may be phosphate-buffered saline (PBS). Other inert control agents may be used, such as DMSO.

On the same day of blood collection, a small volume of whole blood may be combined with one, two, three or more leukocyte-activating agents. Corresponding volumes of each whole blood sample may be combined with the appropriate control agent.

Samples may be incubated with the agents (e.g., the leukocyte-activating agents or the control agents) within a range of temperatures that approximate a physiological temperature. For example, in several examples, the incubations are performed at a temperature ranging from about 30°C to about 42°C, including about 30°C to about 32°C, about 32 °C to about 34 °C, about 34°C to about 36°C, about 36°C to about 38 °C, about 38°C to about 40 °C, about 40°C to about 42°C, and overlapping ranges thereof. The incubation may occur at about 37°C for a period of time.

The period of time for which the incubation occurs varies depending on the particular markers being evaluated and the particular leukocyte-activating agents being used. However, several examples use incubation times of about 6 hours. Incubation times of less than 6 hours may be used, including 5, 4, 3, 2, and 1 hours. The incubation time may also range from about 1 to about 4 hours. Longer time may optionally be used in other examples. After incubation, all blood samples are stored frozen at -80°C until analysis. Advantageously, by quantifying early responder mRNAs, which are induced within a couple of hours, lengthy and complex culture steps used in other methodologies are eliminated.

Markers and Quantification

Specific markers may be evaluated. As used herein, the term "markers" shall be given its ordinary meaning as used in molecular biology and shall also refer to mRNA(s) that are associated with a certain type or class of function, in particular, leukocyte function For example, a particular RNA might be associated with the function of chemotaxis, while another might be associated with antigen presentation. Markers may be evaluated based on their commonly accepted function, though in some examples, a single marker may be representative of more than a single class or function. Non-limiting examples of markers that are associated with certain immune functions are shown in Table 2. Additional markers from the same (or additional) categories of immune function are used in other examples.

Specific subsets of leukocytes may be stimulated in blood samples by adding agents which are known to react with specific subset of leukocytes. The diverse array of immune functions, such as antigen presentation, cytotoxic T cell (CTL) activities, immune suppressor function, chemotaxis, angiogenesis, extravasation, etc., can therefore be assessed by measuring corresponding mRNAs from a stimulated blood sample according to the methods disclosed herein. The disclosed methods may be capable of predicting responders and non-responders for immunotherapy for advanced cancers.

To determine mRNA levels, the erythrocytes and blood components other than leukocytes may be removed from the whole blood sample. Whole blood may be used without removal or isolation of any particular cell type. The leukocytes may be isolated using a device for isolating and amplifying mRNA. Examples of this device are described in more detail in United States Patent Nos. 7,745,180, 7,968,288, and 7,939,300 and United States Patent Application Nos.: 11/376,018, 11/803,594, and 11/803,663.

In brief, certain examples of the device comprise a multi-well plate that contains a plurality of sample-delivery wells, a leukocyte-capturing filter underneath the wells, and an mRNA capture zone underneath the filter which contains immobilized oligo(dT). The device may also contain a vacuum box adapted to receive the filter plate to create a seal between the plate and the box, such that when vacuum pressure is applied, the blood is drawn from the sample-delivery wells across the leukocyte-capturing filter, thereby capturing the leukocytes and allowing non-leukocyte blood components to be removed by washing the filters. Other means of drawing the blood samples through out of the sample wells and through the across the leukocyte-capturing filter, such as centrifugation or positive pressure, may also be used. In other examples of the device, leukocytes may be captured on a plurality of filter membranes that are layered together. The captured leukocytes may then lysed with a lysis buffer, thereby releasing mRNA from the captured leukocytes. The mRNA is then hybridized to the oligo(dT)-immobilized in the mRNA capture zone. Further detail regarding the composition of lysis buffers that may be used herein can be found in United States Patent Application No.: 11/376,018. cDNA may be synthesized from oligo(dT)-immobilized mRNA. The cDNA may be then amplified using real time PCR with primers specifically designed for amplification of infection-associated markers. Primers that are used in such examples are shown in Table 2. Further details about the PCR reactions used in some examples are also found in United States Patent Application No.: 11/376,018.

After the completion of PCR reaction, the various mRNA (as represented by the amount of PCR-amplified cDNA detected) for one or more leukocyte-function-associated markers are quantified. Quantification may be calculated by comparing the amount of mRNA encoding one or more markers to a reference value. The reference value may be expression level of a gene that is not induced by the stimulating agent, e.g., a house-keeping gene. Beta-actin may be used as the reference value. Numerous other house-keeping genes that are well known in the art may also be used as a reference value. A house keeping gene may be used as a correction factor, such that the ultimate comparison is the induced expression level of one or more leukocyte-function-associated markers as compared to the same marker from a non-induced (control) sample. The reference value may be zero, such that the quantification of one or more leukocyte-function-associated markers is represented by an absolute number. Two, three, or more leukocyte-function-associated markers may be quantifiedThe quantification may be performed using real-time PCR and the data may be expressed in terms of fold increase (versus an appropriate control).

Mathematical analysis may be used to evaluate the data related to quantification of leukocyte-function-associated markers. A multi-variable analysis may be used. A multivariate discriminant analysis may be used. Other types of analysis may be used in order to evaluate the quantification data. The evaluation may allow the characterization (e.g., prediction) of an individual's potential responsiveness to a cancer immunotherapy regime. Multivariate analysis is particularly advantageous because of the complex immune interactions that are involved in cancer development, progression and therapy. As discussed herein, multiple classes or categories of function are required for a cancer immunotherapy to be effective. For example, an individual that has elevated cytotoxic function is likely to be non-responsive to immunotherapy treatments if that individual has little or no chemotactic function, as the cytotoxic cells will not be directed to the site of cancer lesion.

Thus, the various functional classes may be used in the multivariate analysis to allow grouping (e.g., categorization) of an individual into a certain predictive group. The groups may be effectively binary, e.g., survivor versus non-survivor. However, in several examples, the groups correlate with responsiveness to a cancer immunotherapy. The groups may be partial responder (which includes a complete responder), stable disease, and progressive disease.

After stimulation of blood samples and quantification (e.g., by calculating fold change) of each gene of interest after exposure to each stimulus of interest, the results are categorized in order to characterize (e.g., predict) each individual's responsiveness to cancer immunotherapy. As a non-limiting example, the groups (for example, PR, SD, PD), may be used to characterize responsiveness.

The quantified data (e.g., fold change of each individual gene due to each individual stimulus) may optionally be converted, for example by taking the log(10) of the fold change, in order to normalize the distribution of the data prior to further analysis. Alternatively, a non-parametric analysis can be performed by assigning values to certain levels of fold increase in RNA expression. For example, a value of zero can be representative of no mRNA induction (e.g., fold increase < 3) and a value of 1 can be representative of mRNA induction (fold increase ≥ 3). Alternative values, or values in between those listed above, may also be used.

The quantified data may then processed by discriminant analysis, which allows prediction of an individual's group membership (e.g., responsiveness) based on the combination of variables (change in the amount of certain leukocyte-function associated mRNA and stimulants). As discussed in Example 1, because clinical progression was known for each individual (see Table 1), the methods disclosed herein allow for the prediction of group membership (e.g., responsiveness) when only the mRNA quantification data is known. Standard, art accepted methods for discriminant analysis were used to generate parameters for each group (e.g., PD, SD, PR) and each combination of mRNA of interest and stimulant agent. As discussed above, however, in some examples other types of multi-variable analysis are used (e.g., non-discriminant analysis). A non-limiting example of this is shown in Table 3 (parameters are shown in Table 3A). A prediction of group membership can be thereafter be made utilizing these parameters and the fold change data for each gene of interest and each stimulant. A non-limiting example of this calculation is provided below:

$$\text{Predictor Value} = \text{Parameter Constant for Group 1} + [\text{Parameter value}_{(\text{Gene X and stimulant A})}] + [\text{Parameter value}_{(\text{Gene X and stimulant B})}] + [\text{Parameter value}_{(\text{Gene Y and stimulant A})}] + [\text{Parameter value}_{(\text{Gene Y and stimulant B})}] + [\ldots]$$

The pattern in the example formula above is thus repeated until each combination of gene of interest with each stimulant agent is accounted for, and repeated for each of the groups. Thereafter, the calculated Predictor Value is then used to make the prediction of group membership for that individual. In this example (and Example 1) the largest Predictor Value of each of the three groups is used to predict membership of the individual within that group. For example if the Predictor Value is 13 for PR, 10 for SD, and 5 for PD, that individual would be categorized as a member of the PR group, and thereby be characterized as likely to be responsive to cancer immunotherapy.

The methods described herein may be used to monitor an individual's responsiveness to ongoing cancer immunotherapy. A first blood sample may be obtained from the individual. The first blood sample may be obtained prior to the administration of any immunotherapy treatment to the individual. The individual may have received a treatment in the past, and might again in the future. The first blood sample may be obtained at a time between two administrations of immunotherapy, such as just prior to an administration. A second blood sample is obtained from the individual at a time after the administration of immunotherapy. This time may be several hours, though in other examples, the time is several weeks, and in some examples up to several months. Additional samples may be taken serially over the course of several months. The blood samples obtained from the individual are then frozen until expression analysis, which is performed as described above. Evaluation of expression levels of leukocyte-function-associated markers can thus be used to monitor the progress (i.e., efficacy) of immunotherapy, as discussed above. Maintenance of the disease status categorization of a patient over time (e.g., PR at time 1 and PR at time 2) may indicate that the patient is responsive to therapy. Changes in category (e.g., PR to SD, PR to PD or SD to PD) indicate that the patient's responsiveness to the therapy is diminished.

In several examples of the methods described herein, artifacts introduced by the analysis itself are eliminated as much as possible, which aids in a more accurate characterization of the complex immune function in each individual. The *ex vivo* assay systems and methods described herein minimize technical variation associated with blood manipulation, mRNA purification, cDNA synthesis, and PCR. Because of the analysis approach presented herein, e.g., that of analysis based on combinations of stimulators of certain immune functions and analysis of leukocyte-function-associated markers associated with certain functions, the predictive methods are widely applicable to a variety of different cancer types and different treatment modalities. For example, dendritic cell therapy is but one example of many types of immunotherapy available (others are disclosed above). Because the methods disclosed herein analyze a wide variety of classes of immune function, as well as multiple specific mRNAs within each class, the power of the predictive methods extends to virtually all types of cancer, and virtually all types of immunotherapy. This is because the assessment employs analysis of multiple markers of multiple immune functions in each patient, which is therefore relevant to all types of cancer.

Applications of the Disclosed Methods

The methods and procedures described herein are useful for a variety of applications. For example, the methods can be used as an ex vivo method for characterizing the potential responsiveness of an individual to cancer immunotherapy. The methods provided can also enable a medical professional to recommend a cancer immunotherapy to a subject based on a calculated likelihood of responding to a cancer immunotherapy. Additionally the methods can be used for advising a subject to undertake a cancer immunotherapy regime. Moreover, the methods can also be used to treat a cancer patient. While alternative methods (such as functional leukocyte assays, cytokine profiling, protein analysis, etc.) could be used to attempt to achieve the characterization of potential responders, data supported clinical recommendations and/or treatment of cancer patients, the methods disclosed herein provide an unexpectedly high degree of accuracy in identifying potential immunotherapy responders. As such, the methods disclosed herein are particularly advantageous for not only identifying potential immunotherapy responders, but also for enabling medical professionals to recommend a particular therapy, advise a cancer patient regarding the likely success of an immunotherapy, and/or proceed with treatment of a cancer patient. The methods described herein therefore open up new opportunities to provide specific therapies to individuals, which will assist in improving the efficacy of therapy, improving patient outcomes, and reducing medical costs. The methods decribed herein may be used in other applications as well, as those listed above are non-limiting examples.

EXAMPLES

Specific embodiments will be described with reference to the following examples which should be regarded in an illustrative rather than a restrictive sense.

Example 1- *Ex vivo* stimulation of leukocyte-function associated mRNAs

Twenty-six (26) patients having a variety of cancer types were recruited as shown in Table 1. These subjects were given dendritic cell therapy as the type of cancer immunotherapy. However, as described above, the disclosed methods are also applicable to other types of immunotherapy.

*Sample Preparation and Stimulation*

Before vaccine treatment (the protocol for which is described below), peripheral blood was drawn and stimulated in triplicate at 37°C for 4 hours with phytohemagglutinin-L (PHA, general T-cell activator), heat aggregated IgG (HAG, classic model of immune complex to activate Fcγ receptors), zymosan (toll like receptor (TLR)-2 agonist as an innate immunity activator), recombinant human interleukin 2 (rIL2), recombinant human interferon α2β (rIFN), mouse monoclonal antibody against α/β chain (antigen recognition unit) of human T cell receptor (aTCR, TCR agonist), picibanil (OK432, immune activator clinically approved in Japan), and phosphate buffered saline (PBS).

Specifically, blood was drawn into a 4 mL heparin container, and 60 μL each of whole blood was immediately applied to three 8-well strips (total 24 well, 1.44 mL in total), where 1.2 μL of PHA (2 mg/mL) (Sigma-Aldrich, St. Louis, MO), HAG (10 mg/mL) (Sigma-Aldrich), zymosan (75 mg/mL) (Sigma-Aldrich), rIL2 (5 μg/mL) (BioLegend, San Diego, CA), rIFN (105 units/mL) (MDS, Tokyo, Japan), aTCR (50 μg/mL) (BioLegend), picibanil (0.05 KE/mL) (Chugai Pharmaceutical Co. Ltd., Tokyo, Japan), phosphate buffer saline (PBS) (Invitrogen, Carlsbad, CA). After 4 hours incubation at 37°C, strips were frozen at -80°C until analysis.

Seventeen different mRNAs were quantified: (β-action (ACTB) and β2 microglobulin (B2M) as controls; interferon γ (IFNG), tumor necrosis factor superfamily (TNFSF)-1, 2, and 5 as markers of cytotoxic activity; interleukin (IL)-10, transforming growth factor-β1 (TGFB), CTL-associated protein 4 (CTLA4), programmed cell death 1 (PDCD1 or PD-1), and forkhead box P3 (FOXP3) for suppressor function; granulocyte macrophage colony-stimulating factor (GMCSF) for antigen presentation, vascular endothelial growth factor (VEGF) for angiogenesis, IL8, CCL chemokine-8 (CCL8), and CXCL chemokine-3 (CXCL3) for chemotaxis, and IL2 (T-cell promoter).

For quantification, frozen blood samples were first thawed (1 min at 37°C). Thawing multiple samples under the same conditions assists in equalizing condition among the 96 wells). 96-well filterplates comprising leukocyte reduction membranes placed over oligo(dT)-immobilized collection plates were prepared and 150 μL of 5 mmol/L Tris (pH 7.4) was applied to wet the filter membranes. After centrifugation at 120g for 1 min at 4 °C to remove the Tris solution from the membranes, 50 μL of the stimulated whole blood samples was applied to each well and immediately centrifuged at 120g for 2 min at 4 °C. The wells were then washed once with 300 μL of phosphate-buffered saline. After centrifugation at 2000g for 5 min at 4 °C to remove the saline solution, 60 μL of stock lysis buffer [5 g/L *N*-lauroylsarcosine, 4x standard saline citrate, 10 mmol/L Tris-HCl (pH 7.4), 1 mmol/L EDTA, 1 mL/L IGEPAL CA-630 (substitute of NP-40), 1.79 mol/L guanidine thiocyanate (all from Sigma)], supplemented with 10 mL/L 2-mercaptoethanol (Bio-Rad), 0.5 g/L proteinase K (Pierce), 0.1 g/L salmon sperm DNA (5 Prime Eppendorf/Brinkman), 0.1 g/L *Escherichia coli* tRNA (Sigma), 5 nmol/L each of the specific reverse primers, and $10^{10}$ molecules/L of synthetic RNA34 (as external control), was added to each well of the filterplates. The plates were then incubated at 37 °C for 10 min, placed over oligo(dT)-immobilized collection microplates (GenePlate; RNAture), and centrifuged at 2000g for 5 min at 4 °C. After overnight storage at 4 °C, the microplates were washed 3 times with 100 μL of plain lysis buffer and then 3 times with 150 μL of wash buffer [0.5 mol/L NaCl, 10 mmol/L Tris (pH 7.4) 1 mmol/L EDTA] at 4 °C.

cDNA was synthesized directly in each well by addition of 30 μL of buffer containing 1x reverse transcription buffer [50 mM KCl, 10 mM Tris-HCl (pH 8.3), 5.5 mM MgCl$_2$, 1 nL/μL Tween 20], 1.25 mM each deoxynucleoside triphosphate, 4 units of rRNasin, and 80 U of MMLV reverse transcriptase (Promega; without primers) and incubation at 37 °C for 2 h. From each 30-μL reaction, 4 μL of cDNA was transferred directly to 384-well PCR plates, and 5 μL of TaqMan universal master mixture (Applied Biosystems) and 1 μL of 5 μM of forward primers were added.

For IL2, IFNG, and GMCSF, 2 μL of undiluted cDNA were used for real time PCR using SYBR green (iTaq, BioRad, Hercules, CA) in the final volume of 5 μL in a 384-well plate. For the other mRNAs, cDNA was diluted 1:1 with nuclease-free water, and 2 μL was used for PCR. Primer sequences are displayed in Table 2. Each gene was amplified individually. As triplicate whole blood aliquots were used as starting materials, a single PCR reaction was performed for each cDNA. In order to minimize false amplification, PCR conditions were optimized for high stringency: 1 cycle of 95°C for 10 min, followed by 50 cycles of 1 min 95°C denature and 1 min 65°C annealing/extension in PRISM7900 (Applied Biosystem, Foster City, CA). In some embodiments, other conditions may be used, depending on the marker of interest.

Primer sequences were designed with the same stringency, thus the PCR protocol was applicable to all mRNAs. Melting curve analysis was performed every time to confirm that the amplification was derived from a single peak. The cycle threshold (Ct) was determined by the analytical software (SDS, Applied Biosystem).

Although the number of patients was 26, the number of mRNA/cDNA preparations was 624 (26 patients x 8 stimulations x 3 (triplicate)), and the number of PCR was 5,928 (=76 mRNAs/stimulations x 26 patients x 3 (triplicate)) (Table 4).

*Vaccine Therapy*

DC vaccine therapy was conducted independently from mRNA analysis. In brief, Peripheral blood mononuclear leukocytes were collected by leukapheresis (**COM.TEC**, Fresenius HemoCare GmbH, Bad Homburg, Germany) (an average 2.8 x $10^9$ cells was collected from 5,000 ml blood volume), suspended in AIM-V (Gibco-BRL, Grand Island, NY) and incubated in sterile culture dishes for 2 hours in an incubator with 5% CO2:95% air. Non-adherent cells were removed by aspiration, and adherent cells were re-suspended in AIM-V supplemented with rGMCSF (final 50 ng/mL) (Gentaur, Kobe, Japan) and rIL4 (50 ng/mL) (R&D Systems, Minneapolis, MN). The culture was continued for 6 days according to established methods. Immature DC were further incubated with rTNFα (50 ng/mL) (*CellGenix* Technologie Transfer GmbH, Freiburg, Germany) supplemented with WT1 peptide (HLA-A2402 (CMTWNQMNL) or HLA-A0201 (CYTWNQMNL) (Neomps Inc, San Diego, CA) dependent on each patient's HLA typing and/or MUC-1 peptide (TRPAPGSTAPPAHGVTSAPDTRPAPGSTAP) (Thermo Fisher Scientific, Bremen, Germany) for an additional 4 days.

Mature DC ($1x10^8$ cells) were harvested and injected into either axillary or inguinal lymph nodes. Administration was performed every other week for a total of 5 administrations. The efficacy of the treatment was determined as partial or minor response (including complete response (CR)) (PR), stable disease (SD), and progressive disease (PD), according to the criteria of Response Evaluation Criteria In Solid Tumors (RECIST, version 1.1.) using CT scan images taken before and 2 months after the completion of the treatment. CT scans were performed every 2-3 months to monitor the status of the cancer.

*Results*

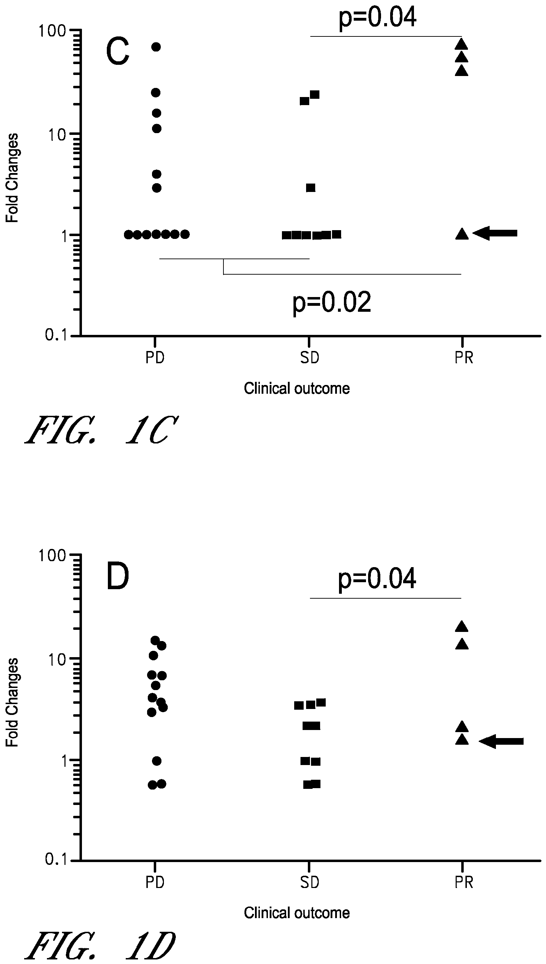

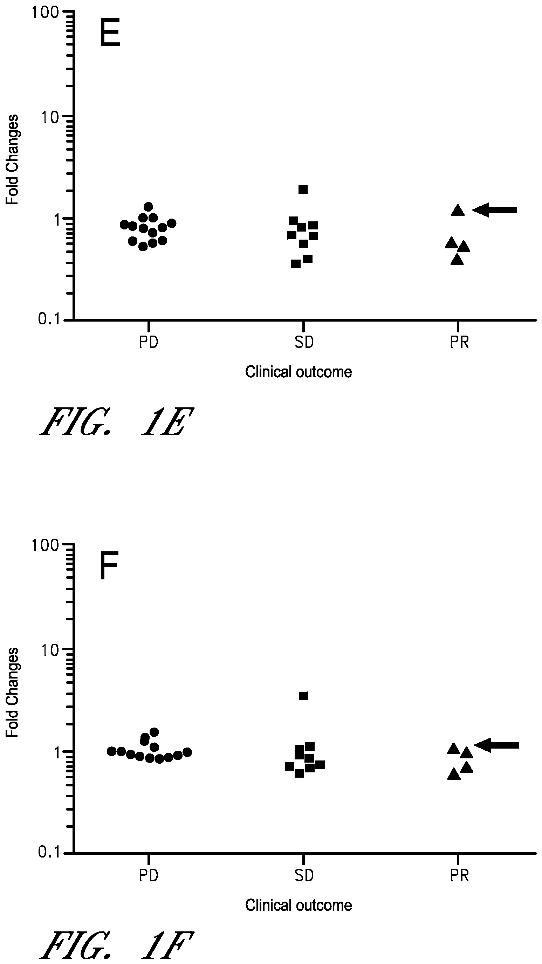

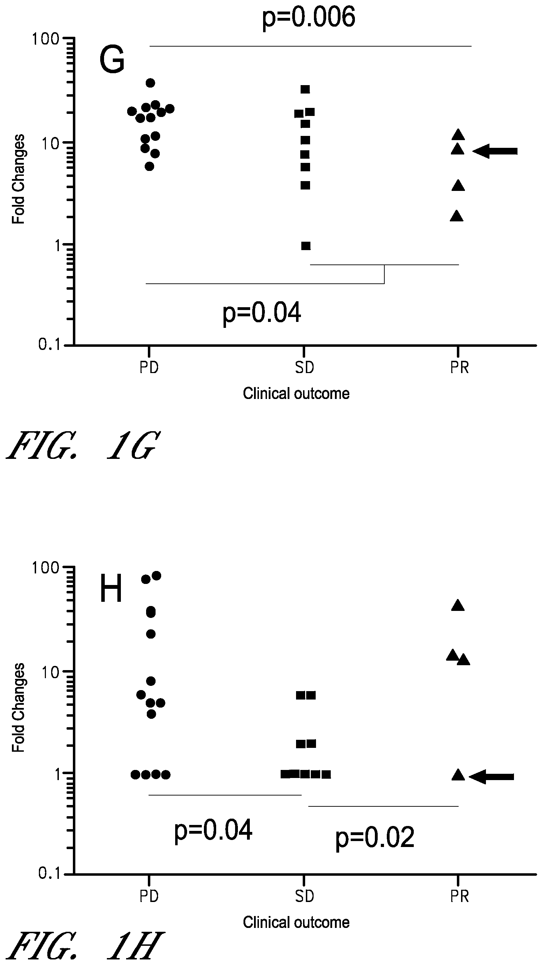

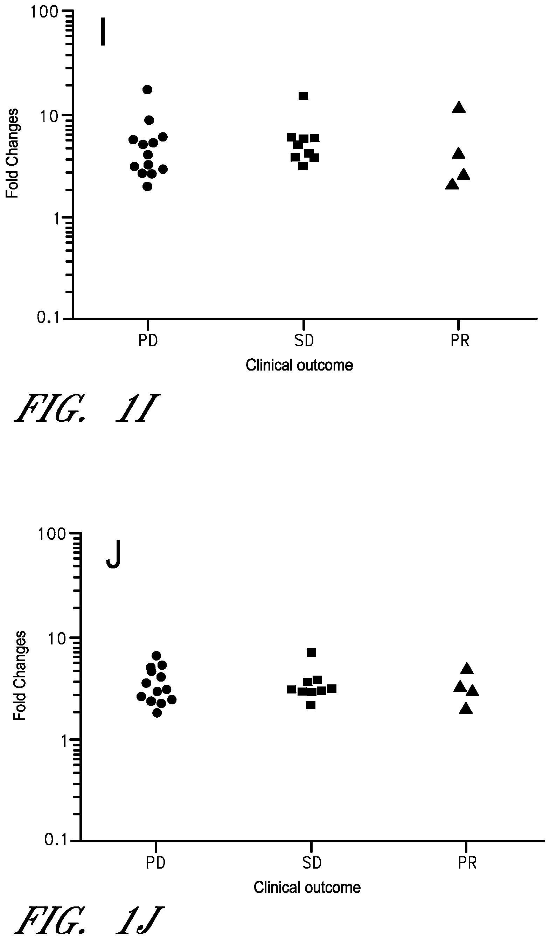

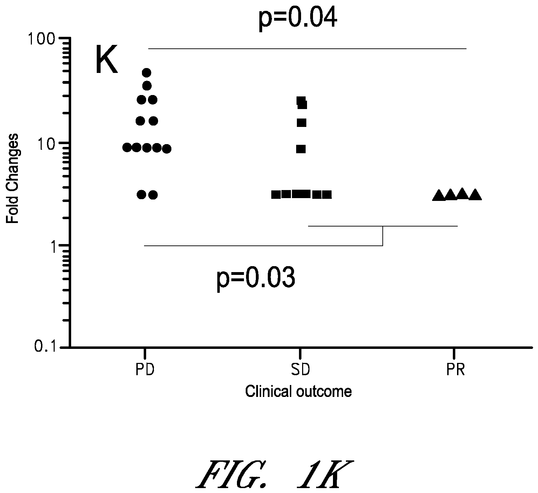

When individual mRNA data were compared among PD, SD, and PR (including 1 case of CR), zymosan-induced IFNG (Fig. 1C) and IL10 (Fig. 1D) were significantly higher in PR than SD (p=0.04) or PD and SD combined (p=0.02). In contrast, PHA- (Fig. 1G) and rIL2-induced TNFSF2 (=TNFα) (Fig. 1K) were significantly (p=0.006 for PHA, p=0.04 for rIL2) higher in PD than PR or SD and PR combined. PHA-induced IL10 (Fig. 1H) in SD was significantly lower than PD (p=0.04) or PR (p=0.02), respectively. The fold increase of 2 control genes (ACTB and B2M) were not different among 3 groups (Fig. 1A, B, E, F, I, J). Evaluation of induction of other mRNAs did not reveal any significant differences among the three groups (see Table 4). Even though certain statistically significant changes were identified in Fig. 1, the large overlap of values among PD, SD, and PR make the results difficult to apply in a diagnostic context.

Interestingly, one non-responsive individual (fold change=1) in zymosan-induced IFNG in PR (Fig. 1C, arrow) is also the second highest responder (fold increase =8.76) in PHA-induced TNFSF2 (Fig. 1G, arrow). Similarly, all patients showed the induction of at least one mRNA (Table 4), confirming that the leukocytes in the blood samples were not only alive, but also physiologically functional. As shown in Figs. 1A, B, E, F, I, J, the ACTB and B2M control genes were in the presence or absence of stimulation. Advantageously, this eliminates the need for normalization (which is required for standard RT-PCR), which can be problematic if the control genes show variability in response to the experimental variables. In some embodiments, however, normalization is optionally used during characterization of the likelihood that an individual will respond to a certain therapy. Thus, the minimized technical variations associated with the disclosed methods, and consistency of the control genes (ACTB and B2M), the high and low responders to the various stimuli (based on mRNA induction) shown in Fig. 1 and Table 4 are representative of individuals with variations in leukocyte function that are not due technical artifacts.

*Analysis of Two mRNAs*

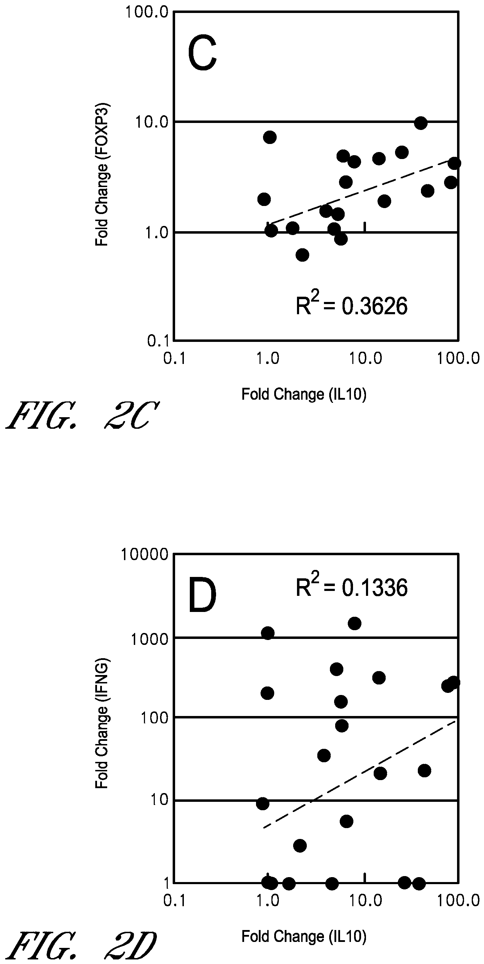

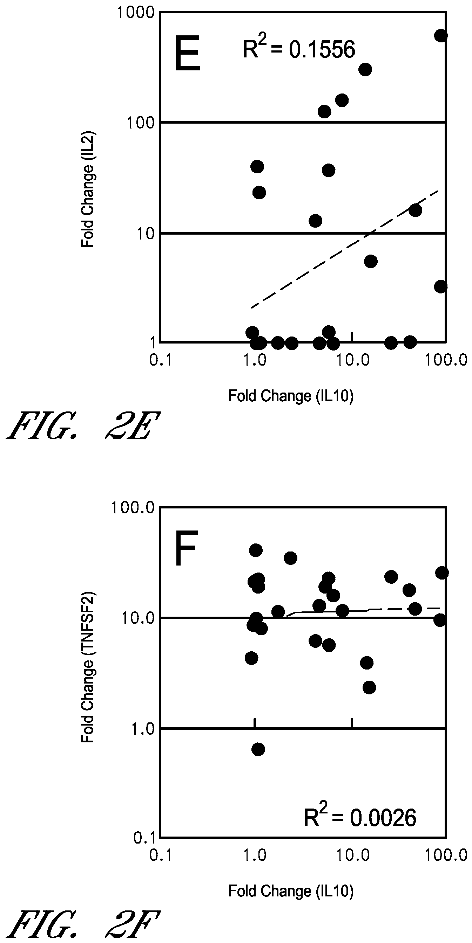

CTLA4, FOXP3, PD-1, and IL10 are markers of immune suppression that were analyzed after stimulation. As shown in Fig. 2, PHA-induced IL10 was correlated with the results of PHA-induced CTLA4 (Fig. 2A), PD-1 (Fig. 2B), and FOXP3 (Fig. 2C) among the 26 patients. However, relatively large individual-to-individual variation in the results exists and renders conclusive prediction of responders challenging. Moreover, stimulation of blood samples does not reveal correlation between all combinations of leukocyte-function associated genes. For example, when PHA-induced IL10 was compared with other immune function markers, such as IFNG (Fig. 2D), IL2 (Fig. 2E), and TNFSF2 (Fig. 2F), no correlation was identified. All other combinations of 2 mRNAs are summarized in Table 5, which was used in the subsequent multivariate analysis. The complexities are likely due to the varied functions of the different markers (e.g.,

certain markers are likely more responsive to certain classes of stimulant agents).

*Multivariate Analysis*

Whether addressing a healthy or diseased context, a variety of immune functions (such as those discussed above) interact with each other. For example, even though CTL activity is strongly maintained, cancer eradication may not occur if an immune suppressor function neutralizes (e.g., outweighs) these killing functions. Similarly, if the CTL fail to accumulate at the cancer lesion site due to weak chemotactic activities, cancer eradication will not likely occur. To achieve a greater degree of predictability of responsiveness to cancer immunotherapy, these multiple immune functions were taken into account by performing a multivariate analysis.

As shall be appreciated by one of ordinary skill in the art, multivariate analysis is designed to predict group membership based on a set of predictor variables. In the context of the methods disclosed herein, the classifications are based on the status of a patient's cancer after cancer immunotherapy (e.g., PD, SD, and PR). The values of the fold change in mRNA levels shown in Fig. 1 and reported in Table 4 were derived from 6 wells of whole blood (3 wells of stimulants and 3 wells of PBS). Each fold increase was calculated as 2^-(mean Ct of PBS - each Ct of the stimulant). The mean values were reported in Fig. 1 and Table 4.

In separate feasibility experiments using serial dilution of pooled cDNA, Ct values for all genes evaluated were linear up to 32 cycles, but Ct of 33 or more showed either no amplification or unacceptably large variation. Thus, Ct=32 was used as the baseline for the calculation of fold increase. When fold increase was analyzed by Student's t-test (Fig. 1) and multivariate discriminant analysis (Minitab 16, State College, PA), the values of fold increase were converted to log(10), to make the distribution normal.

Although the prediction calculation is described in detail above, in brief, after calculating fold change for each gene of interest and for each stimulant agent, discriminant analysis was used to generate a parameter for each combination of gene of interest and stimulant, for each of the disease status groups (PR, SD, PD). Thereafter, a Predictor Value was calculated for each individual, based on the parameter values and the fold change data. An example of the calculation of the Predictor Value is described above. The Predictor Value is then used to characterize the potential responsiveness of each individual to cancer immunotherapy, by determining the disease status group having the largest Predictor Value.

This multivariate approach is advantageous for predicting responsiveness because, even though all mRNAs were analyzed together, the analysis of each stimulant alone resulted in the prediction of PR, SD, and PD at less than 100% (e.g., there are false positives and/or false negatives) (see Table 6 and 6A). This is, however, a greater accuracy in prediction as compared to the natural course of disease progression/regression. In other words, without any prediction of the efficacy of cancer immunotherapy, the chance of SD and PR for each patient is 35%, and 15% respectively. However, as discussed below, the chance of SD or PR becomes 100% if the disclosed prediction methods are used.

As shown in Table 6, stimulation with aTCR, PHA, or Zymosan yields better predictive results of responsiveness to cancer immunotherapy (as measured by correlation to actual clinical outcome) than do than HAG, rIFN or picibanil (Table 6). For example, combination of rIL2 with any other stimulant (except picibanil) yields a correct prediction rate of 100% without any false and negative reactions for all groups of PD, SD and PR (Table 6 and 6A). Similarly, the combination of PHA with any other stimulant (except picibanil) yields a correct prediction rate of 100% without any false and negative reactions for all groups of PD, SD and PR (Table 6). Finally, the combination of aTCR and zymosan also showed 100% prediction (Table 6).

Similarly, when all stimulants were combined, and each mRNA was analyzed individually, no specific mRNA showed 100% accuracy of prediction (Table 5). Even if two different mRNAs were combined, 100% accuracy of prediction was not achieved (Table 5).

However, six combinations (IFNG+GMCSF, IFNG+CXCL3, IFNG+CTLA4, TNFSF1+IL10, TNFSF1+TNFSF2, and CCL8+GMCSF) showed better accuracy with respect to predicting responsiveness to therapy as demonstrated by a less than 30% false positive and negative reaction for PD, SD, and PR (Table 7, boxed). When these combinations were further analyzed, the combination of three mRNAs, such as IFNG+ CXCL3 + CTLA4, IFNG+ GMCSF+TNFSF2, IFNG+GMCSF+ PDCD1, and TNFSF1+TNFSF2+CTLA4 showed 100% accuracy in prediction (e.g., the predictions mirrored the clinical results) without any false positive and negative reactions for all three groups of PD, SD and PR. Of note is that these combinations of mRNAs were each derived from different functional marker groups. Thus, in several embodiments the more diverse the mRNAs that are evaluated, the better accuracy of prediction is achieved.

While cancer antigen-specific CTL or immunoglobulins have been studied in the past, such immunity is acquired only after immunotherapy, thereby negating the value of such studies as information useful for predictive methods. In the Examples above, in order to predict whether each patient is responsive to immunotherapy (in other words, can the patient raise sufficient numbers of cells with the required immune profiles, deliver those cells to cancer sites, and eradicate cancer cells) antigen-presentation function in terms of GMCSF, T cell activation and maturation (IL2), chemotactic activities (CCL8, CXCL3, IL8), immune suppression (CTLA4, TGFB, FOXP3, IL10, PD-1), angiogenesis (VEGF), as well as cytotoxic functions (IFNG, TNFSF1, 2, 5) were analyzed.

The results discussed above indicate that a combination of different functional categories of immunological functions was required for the most accurate prediction of immunotherapy. Since immunology is very complex, and various factors are interacting with each other, the use of multivariate analysis shown in this study is understandable and enables accurate, rapid, and minimally invasive prediction of an individual's potential responsiveness to cancer immunotherapy.

Example 2- Additional Clinical Subject and *Ex vivo* stimulation of leukocyte-function associated mRNAs

As discussed above, cancer immunotherapy is expensive, and may not be efficacious in all cancer patients. Moreover, certain immunotherapies require labor-intensive preparation, adding time and additional costs. Thus, determining if a subject is likely to respond to a therapy is particularly advantageous. Using the methods disclosed herein additional subjects were tested, the subject's being advanced cancer patients (21 new subjects and total of 47 patients) with a variety of cancer types. Clinical outcomes (PD, SD, and PR) were determined by the RECIST criteria. The number of mRNA preparation/cDNA synthesis was 1,128 (8 stimulants x 3 (triplicate) x 47 (patients)), and the number of PCR was 18,048 (1,128 cDNAs x 16 mRNAs). The fold increase (FI) was calculated using the values of PBS. The fold increase of the control genes ACTB and B2M were not different among the three clinical groups. All subjects showed induction of at least 1 mRNA, indicating that the assay conditions were valid and blood samples were capable of induction. Significant differences were detected between PD (n=21) and PR (n=11) groups ($p<0.05$) with respect to PHA-induced CTLA4, PDCD1, IL8, HAG-induced IL8, CXCL3, Picibanil-induced IFNG, anti-TCR-induced IL8, and rIFNy2b-induced IL10. When the SD and PR groups were combined, only PHA- and HAG-induced IL8 were significant (p=0.03, 0.04, respectively). Additionally, using multivariate discriminant analysis, various prediction formulas were developed. When 24 parameters were used, which were derived from the fold increase of 13 PHA-, 5 ZA-, 3 HAG-, 1 rIL2-, and 2 TCR-induced mRNAs, the prediction rate of PD and SD+PR were 100% and 96% in the first set of 26 patients, and 100% and 95% in the second set of patients reported in this example (n=21). This unexpectedly high degree of prediction is a result of the methods disclosed herein, which advantageously allows for the screening of cancer patients for the likelihood that they will respond to a cancer immunotherapy. The disclosed methods allow an analysis of the combinations of diverse types of immune function which accurately reflects the fact that clinical outcome is dependent on the balance among various immune functions in each individual. Thus, the disclosed methods and procedures allow the identification of likely cancer immunotherapy responders, allow medical professionals to be armed with this knowledge and recommend cancer immunotherapy (if appropriate) and/or allow the treatment of cancer patients with an immunotherapy regime predicted to be successful.

Table 1 - Patient Characteristics

| Gender | Age | Cancer type | WBC | Stage | Treatment | Peptide | Concurrent Treatment | Clinical Outcome | Current Status |
|---|---|---|---|---|---|---|---|---|---|
| F | 55 | Pancreas | 3,900 | IV | >=3rd line | WT1+MUC-1 | chemo. | PD | Deceased |
| F | 54 | Colon | 3,700 | Relapse | >=3rd line | MUC-1 | none | SD | Alive |
| M | 66 | Pancreas | 5,700 | IV | 1st line | WT1 | chemo. | PD | Deceased |
| M | 56 | Stomach | 10,700 | Relapse | >=3rd line | WT1+MUC-1 | chemo. | PR | Deceased |
| F | 52 | Ovary | 7,400 | Relapse | 2nd line | WT1+MUC-1 | rad. | PD | Alive |
| F | 64 | Kidney | 4,600 | Relapse | 2nd line | WT1 | chemo. + LAK | SD | Deceased |
| M | 56 | Stomach | 3,600 | IV | 1st line | WT1 | chemo. + LAK | SD | Alive |
| F | 58 | Breast | 6,900 | Relapse | 2nd line | MUC-1 | none | PD | Deceased |
| M | 77 | Lung | 6,600 | IV | >=3rd line | WT1+MUC-1 | none | SD | Deceased |
| F | 66 | Ovary | 4,100 | Relapse | >=3rd line | WT1+MUC-1 | none | SD | Alive |
| F | 43 | Esophagus | 1,800 | Relapse | 2nd line | MUC-1 | chemo. + LAK | PD | Deceased |
| M | 55 | Prostate | 6,900 | Relapse | >=3rd line | WT1+MUC-1 | chemo. + LAK | PR | Alive |
| F | 63 | Colon | 4,700 | IV | >=3rd line | WT1+MUC-1 | chemo. + LAK | PD | Alive |
| F | 72 | Bladder | 5,000 | Relapse | 1st line | WT1 | none | PD | Alive |
| F | 80 | Pancreas | 7,300 | IV | 1st line | WT1+MUC-1 | chemo. + LAK | PD | Deceased |
| F | 63 | Lung | 5,600 | IV | 1st line | WT1 | rad. | SD | Alive |
| F | 63 | Pancreas | 3,300 | IV | 1st line | WT1+MUC-1 | chemo. + LAK | PR | Alive |
| M | 60 | Stomach | 24,600 | IV | >=3rd line | WT1 | none | PD | Deceased |
| F | 59 | Leiomyosarcoma | 3,700 | Relapse | >=3rd line | WT1 | none | SD | Alive |
| F | 67 | Kidney | 2,800 | IV | >=3rd line | WT1 | LAK | PD | Deceased |
| M | 57 | Biliary | 9,300 | IV | neoadjuvant | WT1+MUC-1 | chemo. | CR | Alive |
| M | 59 | Pancreas | 11,300 | IV | 2nd line | WT1+MUC-1 | chemo. | PD | Deceased |
| F | 51 | Breast | 5,000 | III | >=3rd line | WT1+MUC-1 | none | SD | Alive |
| M | 74 | Esophagus | 7,500 | IV | >=3rd line | WT1 | none | SD | Alive |
| F | 60 | Breast | 5,600 | Relapse | 1st line | WT1+MUC-1 | none | PD | Alive |

| Gender | Age | Cancer type | WBC | Stage | Treatment | Peptide | Concurrent Treatment | Clinical Outcome | Current Status |
|--------|-----|-------------|------|---------|----------|---------|---------------------|------------------|----------------|
| F | 72 | Hepatic | 5,200 | Relapse | 2nd line | WT1 | none | PD | Deceased |

Table 2: Primer Sequences for RT-PCR Amplification

| Target | FWD Sequence (5'-3') | REV Sequence (3'-5') |
| --- | --- | --- |
| B-Actin | CCTGGCACCCAGCACAAT | GCCGATCCACACGGAGTACT |
| B-2 microglobulin (B2M) | TGACTTTGTCACAGCCCAAGATA | AATGCGGCATCTTCAAACCT |
| TNFSF1 | CAGCTATCCACCCACACAGATG | CGAAGGCTCCAAAGAAGACAGT |
| TNFSF2 | CGAAGGCTCCAAAGAAGACAGT | CAGGGCAATGATCCCAAAGT |
| TNFSF5 | CCACAGTTCCGCCAAACCT | CACCTGGTTGCAATTCAAATACTC |
| CCL8 | AGAGCTACACAAGAATCACCAACATC | AGACCTCCTTGCCCCGTTT |
| CXCL3 | GGAATTCACCTCAAGAACATCCA | GTGGCTATGACTTCGGTTTGG |
| IL2 | GAACTAAAGGGATCTGAAACAACATTC | TGTTGAGATGATGCTTTGACAAAA |
| IL8 | TGCTAAAGAACTTAGATGTCAGTGCAT | TGGTCCACTCTCAATCACTCTCA |
| IL10 | GCCATGAGTGAGTTTGACATCTTC | GATTTTGGAGACCTCTAATTTATGTCCTA |
| FOXP3 | CACCTACGCCACGCTCATC | AAGGCAAACATGCGTGTGAA |
| CTLA4 | CATGCCTCCTCTTCTTCCTTGA | GGAGGGTGCCACCATGACTA |
| PDCD1 | CTCAGCCGTGCCTGTGTTC | CTCATACGGTGGTAACAGAAAGG |
| GM-CSF | GGCCCCTTGACCATGATG | TCTGGGTTGCACAGGAAGTTT |
| IFNγ | GGAGACCATCAAGGAAGACATGA | GCTTTGCGTTGGACATTCAA |
| TGFβ | CTGCTGAGGCTCAAGTTAAAAGTG | TGAGGTATCGCCAGGAATTGT |
| VEGF | CGCAGCTACTGCCATCCAAT | TGGCTTGAAGATGTACTCGATCTC |

Table 3: Sample Data and Calculation

| | | Log10(fold increase) derived from experiments | | | | | | |
| | | IFNG | | | | CTLA4 | | |
| # | Clinical outcome | PHA | Zymosan | rIL2 | | PHA | Zymosan | rIL2 |
|---|---|---|---|---|---|---|---|---|
| 1 | PD | 2.59386 | 1.83 | 0 | | 0.64718 | 0.29321 | 0.14314 |
| 2 | PD | 3.14716 | 0.41 | 0.01487 | | 0.77558 | 0.23213 | 0.02438 |
| 3 | PD | 2.44457 | 1.41 | 0.27553 | | 0.88073 | 0.39963 | 0.07748 |
| 4 | PD | 0 | 0.00 | 0 | | 0.35627 | 0.30814 | -0.09453 |
| 5 | PD | 1.5696 | 1.04 | 0.20982 | | 0.24854 | 0.3148 | 0.04765 |
| 6 | PD | 1.90065 | 0.63 | 0 | | 0.77716 | 0.32494 | -0.00696 |
| 7 | PD | 0 | 0.00 | 0 | | 0.94292 | 0.31213 | -0.04386 |
| 8 | PD | 0 | 0.00 | 0 | | 0.75445 | 0.15321 | -0.00117 |
| 9 | PD | 2.31869 | 0.13 | -0.20174 | | 0.18241 | -1.59173 | -1.59173 |
| 10 | PD | 0 | 0.00 | 0 | | 0.51001 | 0.21892 | -0.10774 |
| 11 | PD | 2.41469 | 1.22 | 0.29669 | | 0.892 | -0.11646 | -0.21358 |
| 12 | PD | 0 | 0.00 | 0 | | 0.97454 | 0.45399 | 0.04834 |
| 13 | PD | 0 | 0.00 | 0 | | 0.53601 | 0.08859 | -0.06361 |
| 14 | SD | 0 | 0.00 | 0 | | -0.14274 | -0.0171 | -0.06 |
| 15 | SD | 0 | 0.00 | 0 | | 0.5955 | 0.36672 | -0.08574 |
| 16 | SD | 0 | 0.00 | 0 | | 0.79429 | 0.5082 | 0.17716 |
| 17 | SD | 0.98169 | 0.00 | 0 | | 0.52569 | 0 | 0 |
| 18 | SD | 2.2165 | 1.31 | 0.71886 | | 0.5679 | 0.49243 | -0.0214 |
| 19 | SD | 0 | 0.00 | 0 | | 0.69136 | 0.18855 | -0.05779 |
| 20 | SD | 0.73081 | 0.52 | 0 | | 0.47786 | 0.23459 | 0.15888 |
| 21 | SD | 3.06564 | 1.39 | 0.17221 | | 0.97652 | 0.34833 | 0.05513 |
| 22 | SD | 0.44673 | 0.00 | 0 | | 0.64133 | 0.30615 | 0.0507 |
| 23 | PR | 1.3151 | 1.88 | 0.17282 | | 0.60558 | 0.44129 | 0.15681 |
| 24 | PR | 1.36578 | 1.77 | 0 | | 0.69113 | 0.32086 | -0.10329 |
| 25 | PR | 2.49932 | 1.63 | 0.35658 | | 0.88739 | 0.30833 | 0.0103 |
| 26 | PR | 0 | 0.00 | 0 | | 0.42547 | 0.17042 | 0.20365 |

Table 3 Continued:  Sample Data and Calculation

| | | Log10(fold increase) derived from experiments | | | | | |
|---|---|---|---|---|---|---|---|
| | | | TNFSF2 | | | CXCL3 | |
| # | Clinical outcome | PHA | Zymosan | rIL2 | PHA | Zymosan | rIL2 |
| 1 | PD | 1.26378 | 1.36481 | 0.68392 | -0.08845 | 0.16992 | -0.1108 |
| 2 | PD | 1.04586 | 1.29475 | 0.22243 | -0.69033 | 0.2646 | 0.09686 |
| 3 | PD | 1.38087 | 2.15622 | 0.48394 | 0.3904 | 1.83498 | 0.60935 |
| 4 | PD | 1.31822 | 2.31729 | 0.64595 | 0 | 1.33614 | 0.97772 |
| 5 | PD | 0.76658 | 1.71964 | 0.6297 | 0.73891 | 1.32753 | 0.5929 |
| 6 | PD | 1.33644 | 2.34909 | 0.28456 | 0.17581 | 0.87399 | 0.28745 |
| 7 | PD | 1.25266 | 2.37732 | 0.36109 | 1.95237 | 2.52595 | 1.47353 |
| 8 | PD | 1.32544 | 2.07627 | 0.36384 | 0.45202 | 1.87994 | 1.54121 |
| 9 | PD | 1.60153 | 1.65693 | -0.20159 | 0.67929 | 1.5536 | -0.25455 |
| 10 | PD | 0.88893 | 1.7822 | 0.19313 | 0.62669 | 1.62027 | -0.22278 |
| 11 | PD | 0.96687 | 1.90718 | -0.06657 | 0.82181 | 2.15306 | 0.65454 |
| 12 | PD | 1.35907 | 2.53137 | 0.41724 | 0.65883 | 1.49652 | 0.23095 |
| 13 | PD | 1.09172 | 2.6369 | 0.8035 | 0.22959 | 1.07788 | 0.39553 |
| 14 | SD | -0.20776 | -0.20776 | -0.0047 | 0 | 0.04219 | 0 |
| 15 | SD | 1.04828 | 1.30839 | -0.0753 | 0 | 0.48278 | 0 |
| 16 | SD | 1.29843 | 2.02084 | 0.58042 | 0 | 1.20545 | 0.91358 |
| 17 | SD | 0.6436 | 0.31687 | 0 | 0.07706 | 0.51232 | 0.02289 |
| 18 | SD | 0.745 | 1.83954 | 0.10331 | 0.27117 | 1.22303 | 0.14172 |
| 19 | SD | 0.92743 | 2.14227 | 0.15065 | 0.2405 | 1.89241 | 1.19377 |
| 20 | SD | 1.20304 | 1.7057 | 0.29834 | 0.82667 | 1.41595 | 0.46054 |
| 21 | SD | 1.3296 | 2.03202 | 0.17422 | 0 | 1.82054 | 0.84028 |
| 22 | SD | 1.54361 | 2.82295 | 0.49517 | 0.64712 | 1.15594 | -0.04144 |
| 23 | PR | 0.36999 | 1.58689 | 0.10169 | 0.31687 | 1.36758 | 0.46416 |
| 24 | PR | 1.06811 | 2.09982 | 0.03173 | 1.43461 | 1.83775 | 0.417 |
| 25 | PR | 0.58613 | 1.414 | -0.19651 | -0.42808 | 0.47911 | -0.24909 |
| 26 | PR | 0.94268 | 2.14992 | 0.11058 | 0.98497 | 2.19984 | 0.30371 |

Table 3 Continued: Sample Data and Calculation

| | | Log10(fold increase) derived from experiments | | | | | | |
|---|---|---|---|---|---|---|---|---|
| | | GMCSF | | | | IL10 | | |
| # | Clinical outcome | PHA | Zymosan | rIL2 | | PHA | Zymosan | rIL2 |
| 1 | PD | 1.53345 | 1.65994 | 0.37595 | | 0.71498 | 1.43314 | 0.40703 |
| 2 | PD | 2.25788 | 1.65479 | 0.19201 | | 0.88405 | 1.03463 | -0.10258 |
| 3 | PD | 2.20387 | 1.90396 | 0.57038 | | 1.93434 | 1.82988 | 0.23976 |
| 4 | PD | 0.11617 | 0.33608 | 0 | | 0 | 0 | 0 |
| 5 | PD | 1.07141 | 1.36071 | 0 | | 0.59705 | 1.30342 | 0.35022 |
| 6 | PD | 1.99136 | 2.43452 | 0.49976 | | 0.7504 | 1.13851 | 0.26659 |
| 7 | PD | 1.5343 | 2.10636 | 0 | | 1.58008 | 1.89604 | 0.62741 |
| 8 | PD | 1.17469 | 1.62305 | 0 | | 0 | 1.08976 | 0.3276 |
| 9 | PD | 1.66224 | 0 | 0 | | 0.00419 | 0.06473 | -0.19184 |
| 10 | PD | 1.38728 | 1.45376 | 0.11683 | | 0.03586 | 0.22295 | 0.04914 |
| 11 | PD | 1.3818 | 1.56813 | 0 | | 1.89151 | 1.71614 | 0.3287 |
| 12 | PD | 2.14903 | 2.45531 | 0.65248 | | 1.38886 | 1.43975 | 0.56904 |
| 13 | PD | 2.09375 | 2.19599 | -0.01215 | | 0.66215 | 0.9562 | 0.02415 |
| 14 | SD | 0 | 0 | 0 | | 0.02968 | 0.3871 | -0.02917 |
| 15 | SD | 1.03396 | 1.10424 | 0.06551 | | 0.21084 | 0.16419 | 0 |
| 16 | SD | 0.9986 | 1.26037 | 0 | | 0.00604 | 0.23203 | 0 |
| 17 | SD | 0.52143 | 0.14791 | 0 | | -0.05027 | 0.04623 | 0.76129 |
| 18 | SD | 0.99344 | 1.0937 | 0.00479 | | 0.74289 | 1.03922 | 0 |
| 19 | SD | 0 | 1.43073 | 0 | | 0 | 0.80865 | 0.10405 |
| 20 | SD | 2.28232 | 1.82244 | 0.59646 | | 0.79888 | 1.07335 | 0.35068 |
| 21 | SD | 2.19749 | 1.83997 | 0.11312 | | 0.00654 | 1.02183 | 0.0551 |
| 22 | SD | 1.74987 | 2.32539 | 0.39436 | | 0.34095 | 0.79189 | 0.23118 |
| 23 | PR | 1.32165 | 1.72354 | 0.13346 | | 1.17798 | 1.85999 | 0.26874 |
| 24 | PR | 1.64775 | 2.05519 | 0.04255 | | 1.6387 | 2.08658 | 0.53377 |
| 25 | PR | 1.57216 | 2.11385 | 0.18583 | | 1.13778 | 0.78403 | -0.14683 |
| 26 | PR | 1.52 | 1.68855 | 0.11701 | | 0 | 0.6313 | 0 |

Table 3 Continued: Sample Data and Calculation

| # | Clinical outcome | Prediction Values Using Parameters from Table 3A | | | | Prediction Group of the largest values |
| | | PD | PR | SD | | |
|---|---|---|---|---|---|---|
| 1 | PD | 17.8 | 0.4 | 15.7 | | PD |
| 2 | PD | 28.8 | 0.3 | 22.0 | | PD |
| 3 | PD | 37.2 | 4.7 | 26.5 | | PD |
| 4 | PD | 16.7 | -2.8 | 15.1 | | PD |
| 5 | PD | 32.3 | 2.1 | 23.8 | | PD |
| 6 | PD | 16.4 | 2.7 | 15.9 | | PD |
| 7 | PD | 19.5 | -4.1 | 15.1 | | PD |
| 8 | PD | 25.5 | 1.2 | 24.4 | | PD |
| 9 | PD | 27.5 | -0.7 | 20.0 | | PD |
| 10 | PD | 21.2 | 7.9 | 19.5 | | PD |
| 11 | PD | 17.5 | 11.8 | 14.7 | | PD |
| 12 | PD | 29.8 | 2.9 | 24.0 | | PD |
| 13 | PD | 28.8 | 7.9 | 20.1 | | PD |
| 14 | SD | -9.0 | -13.6 | -2.0 | | SD |
| 15 | SD | 4.6 | 3.0 | 12.5 | | SD |
| 16 | SD | 8.3 | -5.7 | 11.2 | | SD |
| 17 | SD | 7.8 | -6.7 | 12.3 | | SD |
| 18 | SD | 6.0 | -2.1 | 13.8 | | SD |
| 19 | SD | 15.9 | 2.9 | 16.5 | | SD |
| 20 | SD | 3.3 | -0.8 | 8.1 | | SD |
| 21 | SD | 10.9 | 8.3 | 16.2 | | SD |
| 22 | SD | 13.4 | 2.4 | 15.6 | | SD |
| 23 | PR | -10.8 | 18.1 | -0.4 | | PR |
| 24 | PR | 4.9 | 24.9 | 9.6 | | PR |
| 25 | PR | -8.0 | 9.6 | 0.9 | | PR |
| 26 | PR | -10.0 | 11.2 | 1.9 | | PR |
| | | | | | | |
| | | | | | | 100% match! |

Table 3 Continued:  Sample Data and Calculation

| Parameters derived from Multivariate Discriminant analysis | | | | |
|---|---|---|---|---|
| | PD | PR | SD | |
| Constant | -24.54 | -15.94 | -11.59 | |
| PHA-IFN | 4.03 | -3.35 | 1.17 | |
| Zymo-IFN | -19.83 | 7.64 | -11.85 | |
| rIL2-IFN | 21.94 | -19.56 | 20.67 | |
| PHA-CTLA4 | -69.07 | -32.81 | -44.66 | |
| Xymo-CTLA4 | 49.47 | 8.17 | 33.28 | |
| rIL2-CTLA4 | -82.09 | 1.52 | -48.68 | |
| PHA-T2 | 47.03 | 12.11 | 34.70 | |
| Zymo-T2 | -44.63 | 1.43 | -27.21 | |
| rIL2-T2 | 42.12 | -18.55 | 17.44 | |
| PHA-X3 | -46.28 | -21.76 | -30.35 | |
| Zymo-X3 | 44.52 | 16.56 | 27.49 | |
| rIL2-X3 | -15.31 | -10.44 | -10.69 | |
| PHA-GM | -10.19 | 2.15 | -5.30 | |
| Zymo-GM | 40.91 | 8.97 | 24.18 | |
| rIL2-GM | -34.12 | -30.34 | -23.12 | |
| PHA-IL10 | 14.08 | 6.81 | 4.91 | |
| Zymo-IL10 | 2.90 | 0.48 | 5.06 | |
| rIL2-IL10 | 38.74 | 16.90 | 26.53 | |

Table 4. Summary of the fold changes of mRNA induction

| | | | Each number is the fold increase derived from the Ct of 6 wells (3 wells of each stimulant and another 3 wells of PBS control). | | | | | | |
|---|---|---|---|---|---|---|---|---|---|
| | | | ACTB | | | | | | |
| # | Age | Cancer type | PHA | HAG | rIFN | Zymo. | rIL2 | aTCR | Pici. |
| 1 | 55 | Pancreas | 0.9* | 1.3 | 1.1 | 1.2 | 1.4 | 1.4 | 1.0 |
| 2 | 54 | Colon | 0.7 | 1.2 | 1.4 | 1.6 | 1.5 | 1.3 | 1.4 |
| 3 | 66 | Pancreas | 0.6 | 1.0 | 0.8 | 0.7 | 1.0 | 1.1 | 1.3 |
| 4 | 56 | Stomach | 0.4 | 1.3 | 1.0 | 1.0 | 1.2 | 1.1 | 1.4 |
| 5 | 52 | Ovary | 0.7 | 1.1 | 0.8 | 0.9 | 1.0 | 1.0 | 1.4 |
| 6 | 64 | Kidney | 0.8 | 1.4 | 0.9 | 1.8 | 1.4 | 1.3 | 2.0 |
| 7 | 56 | Stomach | 1.0 | 1.6 | 1.1 | 1.9 | 1.5 | 1.6 | 1.4 |
| 8 | 58 | Breast | 0.6 | 1.3 | 1.3 | 1.8 | 1.6 | 1.7 | 1.6 |
| 9 | 77 | Lung | 1.9 | 1.3 | 2.1 | 0.3 | 2.9 | 3.7 | 1.3 |
| 10 | 66 | Ovary | 0.7 | 1.5 | 1.0 | 2.1 | 1.2 | 1.3 | 2.0 |
| 11 | 43 | Esophagus | 0.5 | 1.9 | 0.8 | 1.4 | 1.4 | 1.5 | 2.6 |
| 12 | 55 | Prostate | 0.6 | 2.6 | 0.8 | 2.0 | 0.8 | 1.2 | 1.5 |
| 13 | 63 | Colon | 0.8 | 1.4 | 0.9 | 1.0 | 1.2 | 1.2 | 1.5 |
| 14 | 72 | Bladder | 1.0 | 1.7 | 0.8 | 1.1 | 0.9 | 1.1 | 2.1 |
| 15 | 80 | Pancreas | 0.8 | 1.1 | 1.0 | 0.9 | 1.0 | 1.4 | 1.7 |
| 16 | 63 | Lung | 0.4 | 1.1 | 0.8 | 0.6 | 1.0 | 1.5 | 1.1 |
| 17 | 63 | Pancreas | 0.5 | 1.5 | 1.0 | 0.5 | 0.9 | 1.1 | 0.8 |
| 18 | 60 | Stomach | 1.3 | 1.2 | 0.9 | 1.1 | 0.7 | 0.9 | 1.0 |
| 19 | 59 | Leiomyosarcoma | 0.4 | 1.3 | 1.1 | 1.1 | 1.5 | 1.5 | 1.9 |
| 20 | 67 | Kidney | 0.9 | 1.5 | 1.0 | 1.7 | 1.5 | 1.4 | 2.3 |
| 21 | 57 | Biliary | 1.2 | 1.5 | 1.1 | 2.9 | 2.4 | 2.6 | 3.6 |
| 22 | 59 | Pancreas | 0.8 | 1.8 | 0.9 | 0.9 | 0.9 | 1.0 | 1.3 |
| 23 | 51 | Breast | 0.6 | 1.2 | 0.9 | 1.2 | 1.2 | 1.6 | 1.6 |
| 24 | 74 | Esophagus | 0.9 | 2.3 | 0.8 | 2.0 | 1.2 | 1.4 | 2.1 |
| 25 | 60 | Breast | 1.0 | 1.4 | 1.0 | 1.5 | 2.0 | 2.4 | 4.1 |
| 26 | 72 | Hepatic | 0.6 | 1.3 | 1.7 | 1.8 | 3.1 | 4.5 | 7.5 |

Table 4 Continued. Summary of the fold changes of mRNA induction

| # | Age | Cancer type | B2M PHA | HAG | rIFN | Zymo. | rIL2 | aTCR | Pici. |
|---|-----|-------------|---------|-----|------|-------|------|------|-------|
| 1 | 55 | Pancreas | 0.9 | 1.0 | 1.1 | 2.4 | 1.1 | 1.2 | 0.9 |
| 2 | 54 | Colon | 0.6 | 0.9 | 1.0 | 1.1 | 1.0 | 0.8 | 0.6 |
| 3 | 66 | Pancreas | 1.0 | 0.9 | 0.8 | 1.3 | 0.8 | 0.9 | 1.0 |
| 4 | 56 | Stomach | 0.7 | 0.9 | 0.8 | 1.6 | 1.0 | 0.9 | 0.9 |
| 5 | 52 | Ovary | 0.8 | 0.9 | 0.7 | 1.7 | 1.0 | 0.8 | 0.8 |
| 6 | 64 | Kidney | 0.9 | 1.1 | 0.9 | 1.8 | 1.0 | 1.0 | 0.9 |
| 7 | 56 | Stomach | 1.1 | 1.1 | 1.0 | 2.2 | 1.0 | 1.1 | 1.0 |
| 8 | 58 | Breast | 1.0 | 1.1 | 1.1 | 2.0 | 1.4 | 1.3 | 1.0 |
| 9 | 77 | Lung | 3.6 | 1.0 | 1.3 | 0.7 | 1.7 | 2.9 | 1.6 |
| 10 | 66 | Ovary | 0.9 | 1.2 | 0.9 | 2.4 | 1.2 | 1.4 | 1.4 |
| 11 | 43 | Esophagus | 0.9 | 1.0 | 0.8 | 1.8 | 1.3 | 1.7 | 1.4 |
| 12 | 55 | Prostate | 0.7 | 1.0 | 0.9 | 2.8 | 0.8 | 1.3 | 1.0 |
| 13 | 63 | Colon | 1.3 | 1.2 | 1.0 | 1.9 | 1.2 | 1.3 | 1.1 |
| 14 | 72 | Bladder | 1.4 | 1.0 | 0.8 | 1.5 | 0.8 | 1.1 | 1.1 |
| 15 | 80 | Pancreas | 1.5 | 1.1 | 0.8 | 1.3 | 0.8 | 1.0 | 1.2 |
| 16 | 63 | Lung | 0.7 | 0.9 | 0.8 | 1.1 | 0.8 | 0.9 | 0.7 |
| 17 | 63 | Pancreas | 1.0 | 1.2 | 0.9 | 1.2 | 1.0 | 1.1 | 0.9 |
| 18 | 60 | Stomach | 0.9 | 0.9 | 0.7 | 0.8 | 0.7 | 0.9 | 0.9 |
| 19 | 59 | Leiomyosarcoma | 0.8 | 1.1 | 1.0 | 1.1 | 1.0 | 1.0 | 1.2 |
| 20 | 67 | Kidney | 1.0 | 1.2 | 0.9 | 1.0 | 1.0 | 0.7 | 1.3 |
| 21 | 57 | Biliary | 1.1 | 1.2 | 0.9 | 1.3 | 1.3 | 1.4 | 2.0 |
| 22 | 59 | Pancreas | 0.9 | 1.2 | 1.0 | 1.0 | 0.9 | 0.9 | 1.1 |
| 23 | 51 | Breast | 1.1 | 1.5 | 0.9 | 1.6 | 1.1 | 1.2 | 1.3 |
| 24 | 74 | Esophagus | 0.7 | 1.3 | 0.9 | 1.6 | 1.0 | 1.1 | 0.9 |
| 25 | 60 | Breast | 1.1 | 1.0 | 1.1 | 1.7 | 1.4 | 1.4 | 1.9 |
| 26 | 72 | Hepatic | 0.9 | 1.0 | 1.5 | 2.0 | 1.6 | 2.4 | 2.9 |

Table 4 Continued. Summary of the fold changes of mRNA induction

| # | Age | Cancer type | | IFNG | | | | | |
|---|-----|-------------|---|------|------|------|------|------|------|
|   |     |             |   | PHA | rIFN | Zymo. | rIL2 | aTCR | Pici. |
| 1 | 55 | Pancreas | | 392.5 | 1.1 | 67.8 | 1.0 | 2.2 | 7.6 |
| 2 | 54 | Colon | | 1.0 | 1.0 | 1.0 | 1.0 | 1.0 | 1.0 |
| 3 | 66 | Pancreas | | 1403.3 | 1.0 | 2.6 | 1.0 | 2.8 | 1.1 |
| 4 | 56 | Stomach | | 20.7 | 1.1 | 75.8 | 1.5 | 5.4 | 45.0 |
| 5 | 52 | Ovary | | 278.3 | 1.0 | 25.7 | 1.9 | 3.1 | 3.9 |
| 6 | 64 | Kidney | | 1.0 | 1.0 | 1.0 | 1.0 | 1.0 | 1.0 |
| 7 | 56 | Stomach | | 1.0 | 1.0 | 1.0 | 1.0 | 1.0 | 1.0 |
| 8 | 58 | Breast | | 1.0 | 1.0 | 1.0 | 1.0 | 1.0 | 1.0 |
| 9 | 77 | Lung | | 9.6 | 1.0 | 1.0 | 1.0 | 1.0 | 1.0 |
| 10 | 66 | Ovary | | 164.6 | 1.5 | 20.5 | 5.2 | 1.9 | 13.4 |
| 11 | 43 | Esophagus | | 37.1 | 1.2 | 11.1 | 1.6 | 1.6 | 15.2 |
| 12 | 55 | Prostate | | 23.2 | 1.0 | 59.3 | 1.0 | 2.4 | 110.5 |
| 13 | 63 | Colon | | 79.6 | 1.0 | 4.3 | 1.0 | 1.4 | 8.2 |
| 14 | 72 | Bladder | | 1.0 | 1.0 | 1.0 | 1.0 | 6.3 | 2.3 |
| 15 | 80 | Pancreas | | 1.0 | 1.0 | 1.0 | 1.0 | 1.0 | 1.1 |
| 16 | 63 | Lung | | 1.0 | 1.0 | 1.0 | 1.0 | 1.0 | 1.0 |
| 17 | 63 | Pancreas | | 315.7 | 1.2 | 42.7 | 2.3 | 9.2 | 10.6 |
| 18 | 60 | Stomach | | 208.3 | 0.6 | 1.4 | 0.6 | 423.0 | 1.5 |
| 19 | 59 | Leiomyosarcoma | | 5.4 | 1.0 | 3.3 | 1.0 | 4.7 | 4.8 |
| 20 | 67 | Kidney | | 1.0 | 1.0 | 1.0 | 1.0 | 1.2 | 1.0 |
| 21 | 57 | Biliary | | 1.0 | 1.0 | 1.0 | 1.0 | 1.0 | 4.6 |
| 22 | 59 | Pancreas | | 259.8 | 0.9 | 16.5 | 2.0 | 1.1 | 354.6 |
| 23 | 51 | Breast | | 1163.2 | 1.0 | 24.5 | 1.5 | 15.8 | 36.7 |
| 24 | 74 | Esophagus | | 2.8 | 1.0 | 1.0 | 1.0 | 34.8 | 1.0 |
| 25 | 60 | Breast | | 1.0 | 1.0 | 1.0 | 1.0 | 1.0 | 1.0 |
| 26 | 72 | Hepatic | | 1.0 | 1.0 | 1.0 | 1.0 | 1.0 | 1.0 |

Table 4 Continued. Summary of the fold changes of mRNA induction

| # | Age | Cancer type | | TNFSF1 | | | |
|---|-----|-------------|---|------|-------|------|------|
| | | | | PHA | Zymo. | rIL2 | aTCR |
| 1 | 55 | Pancreas | | 9.1 | 4.0 | 3.1 | 1.4 |
| 2 | 54 | Colon | | 1.0 | 1.0 | 1.0 | 1.0 |
| 3 | 66 | Pancreas | | 11.2 | 2.7 | 3.7 | 0.9 |
| 4 | 56 | Stomach | | 2.6 | 3.8 | 1.3 | 1.1 |
| 5 | 52 | Ovary | | 5.0 | 3.9 | 3.0 | 0.9 |
| 6 | 64 | Kidney | | 3.1 | 1.9 | 1.5 | 1.0 |
| 7 | 56 | Stomach | | 5.9 | 4.1 | 2.2 | 1.4 |
| 8 | 58 | Breast | | 1.0 | 1.6 | 1.4 | 1.1 |
| 9 | 77 | Lung | | 5.1 | 1.0 | 1.0 | 1.0 |
| 10 | 66 | Ovary | | 1.5 | 5.2 | 1.1 | 1.2 |
| 11 | 43 | Esophagus | | 2.0 | 2.4 | 1.5 | 1.0 |
| 12 | 55 | Prostate | | 5.3 | 7.7 | 3.0 | 1.3 |
| 13 | 63 | Colon | | 4.7 | 3.3 | 1.3 | 0.9 |
| 14 | 72 | Bladder | | 9.3 | 5.3 | 2.2 | 4.1 |
| 15 | 80 | Pancreas | | 4.1 | 3.9 | 1.8 | 1.6 |
| 16 | 63 | Lung | | 2.1 | 2.7 | 1.2 | 2.7 |
| 17 | 63 | Pancreas | | 9.5 | 4.2 | 1.9 | 1.3 |
| 18 | 60 | Stomach | | 0.9 | 1.4 | 0.9 | 1.2 |
| 19 | 59 | Leiomyosarcoma | | 2.4 | 3.1 | 0.8 | 1.5 |
| 20 | 67 | Kidney | | 2.4 | 1.9 | 1.0 | 1.1 |
| 21 | 57 | Biliary | | 2.3 | 2.2 | 1.4 | 1.8 |
| 22 | 59 | Pancreas | | 3.5 | 2.0 | 1.0 | 0.7 |
| 23 | 51 | Breast | | 6.0 | 2.3 | 1.0 | 1.1 |
| 24 | 74 | Esophagus | | 1.1 | 3.0 | 1.3 | 0.8 |
| 25 | 60 | Breast | | 6.1 | 5.4 | 3.0 | 1.6 |
| 26 | 72 | Hepatic | | 3.1 | 3.2 | 1.9 | 1.0 |

Table 4 Continued. Summary of the fold changes of mRNA induction

| # | Age | Cancer type | | TNFSF2 | | | | |
|---|-----|-------------|---|--------|-----|------|------|------|
| | | | | PHA | HAG | Zymo. | rIL2 | aTCR |
| 1 | 55 | Pancreas | | 18.4 | 2.1 | 23.2 | 4.8 | 4.6 |
| 2 | 54 | Colon | | 0.6 | 0.6 | 0.6 | 1.0 | 0.7 |
| 3 | 66 | Pancreas | | 11.1 | 1.1 | 19.7 | 1.7 | 1.1 |
| 4 | 56 | Stomach | | 2.3 | 0.8 | 38.6 | 1.3 | 1.2 |
| 5 | 52 | Ovary | | 24.0 | 4.9 | 143.3 | 3.0 | 2.2 |
| 6 | 64 | Kidney | | 11.2 | 1.5 | 20.3 | 0.8 | 1.6 |
| 7 | 56 | Stomach | | 19.9 | 1.6 | 104.9 | 3.8 | 3.5 |
| 8 | 58 | Breast | | 20.8 | 1.4 | 207.6 | 4.4 | 2.9 |
| 9 | 77 | Lung | | 4.4 | 1.0 | 2.1 | 1.0 | 1.0 |
| 10 | 66 | Ovary | | 5.6 | 0.8 | 69.1 | 1.3 | 1.7 |
| 11 | 43 | Esophagus | | 5.8 | 2.4 | 52.4 | 4.3 | 2.5 |
| 12 | 55 | Prostate | | 11.7 | 2.0 | 125.8 | 1.1 | 1.8 |
| 13 | 63 | Colon | | 21.7 | 3.3 | 223.4 | 1.9 | 1.0 |
| 14 | 72 | Bladder | | 17.9 | 2.0 | 238.4 | 2.3 | 10.5 |
| 15 | 80 | Pancreas | | 21.2 | 3.2 | 119.2 | 2.3 | 2.6 |
| 16 | 63 | Lung | | 8.5 | 3.3 | 138.8 | 1.4 | 2.9 |
| 17 | 63 | Pancreas | | 3.9 | 1.9 | 25.9 | 0.6 | 1.1 |
| 18 | 60 | Stomach | | 40.0 | 1.6 | 45.4 | 0.6 | 39.2 |
| 19 | 59 | Leiomyosarcoma | | 16.0 | 1.7 | 50.8 | 2.0 | 5.0 |
| 20 | 67 | Kidney | | 7.7 | 1.5 | 60.6 | 1.6 | 7.3 |
| 21 | 57 | Biliary | | 8.8 | 2.0 | 141.2 | 1.3 | 1.8 |
| 22 | 59 | Pancreas | | 9.3 | 1.8 | 80.8 | 0.9 | 0.9 |
| 23 | 51 | Breast | | 21.4 | 2.0 | 107.7 | 1.5 | 3.6 |
| 24 | 74 | Esophagus | | 35.0 | 4.0 | 665.2 | 3.1 | 7.7 |
| 25 | 60 | Breast | | 22.9 | 1.8 | 339.9 | 2.6 | 4.6 |
| 26 | 72 | Hepatic | | 12.4 | 1.0 | 433.4 | 6.4 | 9.9 |

Table 4 Continued. Summary of the fold changes of mRNA induction

| # | Age | Cancer type | | TNFSF5 | | |
| --- | --- | --- | --- | --- | --- | --- |
| | | | | PHA | rIL2 | aTCR |
| 1 | 55 | Pancreas | | 2.4 | 1.3 | 0.9 |
| 2 | 54 | Colon | | 0.7 | 0.7 | 1.2 |
| 3 | 66 | Pancreas | | 23.2 | 1.8 | 1.6 |
| 4 | 56 | Stomach | | 4.4 | 1.0 | 1.2 |
| 5 | 52 | Ovary | | 10.4 | 0.6 | 0.7 |
| 6 | 64 | Kidney | | 3.7 | 0.5 | 0.5 |
| 7 | 56 | Stomach | | 4.7 | 0.4 | 0.4 |
| 8 | 58 | Breast | | 0.8 | 0.8 | 0.8 |
| 9 | 77 | Lung | | 1.6 | 1.0 | 1.0 |
| 10 | 66 | Ovary | | 5.6 | 0.8 | 1.2 |
| 11 | 43 | Esophagus | | 3.8 | 1.4 | 1.3 |
| 12 | 55 | Prostate | | 3.6 | 0.7 | 1.0 |
| 13 | 63 | Colon | | 16.3 | 1.6 | 1.5 |
| 14 | 72 | Bladder | | 6.9 | 0.2 | 0.3 |
| 15 | 80 | Pancreas | | 2.6 | 0.2 | 0.2 |
| 16 | 63 | Lung | | 0.4 | 0.2 | 0.2 |
| 17 | 63 | Pancreas | | 28.4 | 1.1 | 1.3 |
| 18 | 60 | Stomach | | 2.4 | 0.6 | 0.4 |
| 19 | 59 | Leiomyosarcoma | | 8.5 | 1.1 | 1.9 |
| 20 | 67 | Kidney | | 9.0 | 1.5 | 2.7 |
| 21 | 57 | Biliary | | 6.6 | 3.5 | 3.3 |
| 22 | 59 | Pancreas | | 13.0 | 0.6 | 0.7 |
| 23 | 51 | Breast | | 14.7 | 0.8 | 1.1 |
| 24 | 74 | Esophagus | | 3.2 | 0.8 | 1.9 |
| 25 | 60 | Breast | | 13.6 | 2.0 | 1.4 |
| 26 | 72 | Hepatic | | 9.1 | 3.9 | 5.2 |

Table 4 Continued. Summary of the fold changes of mRNA induction

| # | Age | Cancer type | | IL10 PHA | HAG | rIFN | Zymo. | rIL2 | aTCR |
|---|-----|-------------|---|-----|-----|------|-------|------|------|
| 1 | 55 | Pancreas | | 5.2 | 1.5 | 1.3 | 27.1 | 2.6 | 2.4 |
| 2 | 54 | Colon | | 1.1 | 0.9 | 1.1 | 2.4 | 0.9 | 1.0 |
| 3 | 66 | Pancreas | | 7.7 | 0.6 | 0.8 | 10.8 | 0.8 | 0.8 |
| 4 | 56 | Stomach | | 15.1 | 0.9 | 0.6 | 72.4 | 1.9 | 2.2 |
| 5 | 52 | Ovary | | 86.0 | 2.1 | 0.8 | 67.6 | 1.7 | 1.0 |
| 6 | 64 | Kidney | | 1.6 | 1.0 | 1.0 | 1.5 | 1.0 | 1.0 |
| 7 | 56 | Stomach | | 1.0 | 1.0 | 1.0 | 1.7 | 1.0 | 1.0 |
| 8 | 58 | Breast | | 1.0 | 1.0 | 1.0 | 1.0 | 1.0 | 1.0 |
| 9 | 77 | Lung | | 0.9 | 2.2 | 2.8 | 1.1 | 5.8 | 3.6 |
| 10 | 66 | Ovary | | 5.5 | 3.2 | 1.6 | 10.9 | 1.0 | 1.6 |
| 11 | 43 | Esophagus | | 4.0 | 1.9 | 1.0 | 20.1 | 2.2 | 2.1 |
| 12 | 55 | Prostate | | 43.5 | 8.5 | 1.1 | 122.1 | 3.4 | 4.9 |
| 13 | 63 | Colon | | 5.6 | 0.6 | 1.7 | 13.8 | 1.8 | 1.3 |
| 14 | 72 | Bladder | | 38.0 | 3.1 | 1.0 | 78.7 | 4.2 | 3.5 |
| 15 | 80 | Pancreas | | 1.0 | 1.0 | 1.1 | 12.3 | 2.1 | 3.2 |
| 16 | 63 | Lung | | 1.0 | 1.0 | 1.0 | 6.4 | 1.3 | 2.7 |
| 17 | 63 | Pancreas | | 13.7 | 0.8 | 0.8 | 6.1 | 0.7 | 1.2 |
| 18 | 60 | Stomach | | 1.0 | 2.3 | 0.9 | 1.2 | 0.6 | 0.3 |
| 19 | 59 | Leiomyosarcoma | | 6.3 | 0.9 | 0.7 | 11.8 | 2.2 | 3.1 |
| 20 | 67 | Kidney | | 1.1 | 1.0 | 1.0 | 1.7 | 1.1 | 1.5 |
| 21 | 57 | Biliary | | 1.0 | 1.0 | 1.0 | 4.3 | 1.0 | 1.0 |
| 22 | 59 | Pancreas | | 77.9 | 3.0 | 1.2 | 52.0 | 2.1 | 1.5 |
| 23 | 51 | Breast | | 1.0 | 1.0 | 1.0 | 10.5 | 1.1 | 1.0 |
| 24 | 74 | Esophagus | | 2.2 | 3.2 | 0.9 | 6.2 | 1.7 | 1.0 |
| 25 | 60 | Breast | | 24.5 | 5.8 | 2.8 | 27.5 | 3.7 | 3.3 |
| 26 | 72 | Hepatic | | 4.6 | 1.6 | 0.9 | 9.0 | 1.1 | 0.4 |

Table 4 Continued. Summary of the fold changes of mRNA induction

| # | Age | Cancer type | | TGFB PHA |
|---|-----|-------------|---|-----|
| 1 | 55 | Pancreas | | 0.6 |
| 2 | 54 | Colon | | 3.1 |
| 3 | 66 | Pancreas | | 91.2 |
| 4 | 56 | Stomach | | 37.4 |
| 5 | 52 | Ovary | | 53.9 |
| 6 | 64 | Kidney | | 5.5 |
| 7 | 56 | Stomach | | 6.6 |
| 8 | 58 | Breast | | 1.9 |
| 9 | 77 | Lung | | 10.7 |
| 10 | 66 | Ovary | | 42.3 |
| 11 | 43 | Esophagus | | 36.5 |
| 12 | 55 | Prostate | | 84.9 |
| 13 | 63 | Colon | | 79.2 |
| 14 | 72 | Bladder | | 119.9 |
| 15 | 80 | Pancreas | | 25.7 |
| 16 | 63 | Lung | | 5.4 |
| 17 | 63 | Pancreas | | 0.8 |
| 18 | 60 | Stomach | | 0.7 |
| 19 | 59 | Leiomyosarcoma | | 1.3 |
| 20 | 67 | Kidney | | 2.9 |
| 21 | 57 | Biliary | | 2.2 |
| 22 | 59 | Pancreas | | 0.8 |
| 23 | 51 | Breast | | 1.0 |
| 24 | 74 | Esophagus | | 1.2 |
| 25 | 60 | Breast | | 3.0 |
| 26 | 72 | Hepatic | | 4.9 |

Table 4 Continued. Summary of the fold changes of mRNA induction

| # | Age | Cancer type | | CTLA4 | | | |
|---|-----|-------------|---|-------|------|-----|------|
| | | | | PHA | Zymo. | rIL2 | aTCR |
| 1 | 55 | Pancreas | | 4.4 | 2.0 | 1.4 | 1.0 |
| 2 | 54 | Colon | | 0.7 | 1.0 | 0.9 | 0.9 |
| 3 | 66 | Pancreas | | 6.0 | 1.7 | 1.1 | 1.0 |
| 4 | 56 | Stomach | | 4.0 | 2.8 | 1.4 | 1.3 |
| 5 | 52 | Ovary | | 7.6 | 2.5 | 1.2 | 1.2 |
| 6 | 64 | Kidney | | 3.9 | 2.3 | 0.8 | 1.3 |
| 7 | 56 | Stomach | | 6.2 | 3.2 | 1.5 | 1.6 |
| 8 | 58 | Breast | | 2.3 | 2.0 | 0.8 | 1.0 |
| 9 | 77 | Lung | | 3.4 | 1.0 | 1.0 | 1.0 |
| 10 | 66 | Ovary | | 3.7 | 3.1 | 1.0 | 1.4 |
| 11 | 43 | Esophagus | | 1.8 | 2.1 | 1.1 | 1.1 |
| 12 | 55 | Prostate | | 4.9 | 2.1 | 0.8 | 1.4 |
| 13 | 63 | Colon | | 6.0 | 2.1 | 1.0 | 1.0 |
| 14 | 72 | Bladder | | 8.8 | 2.1 | 0.9 | 1.7 |
| 15 | 80 | Pancreas | | 5.7 | 1.4 | 1.0 | 0.9 |
| 16 | 63 | Lung | | 4.9 | 1.5 | 0.9 | 2.0 |
| 17 | 63 | Pancreas | | 7.7 | 2.0 | 1.0 | 1.1 |
| 18 | 60 | Stomach | | 1.5 | 0.0 | 0.0 | 1.0 |
| 19 | 59 | Leiomyosarcoma | | 3.0 | 1.7 | 1.4 | 1.9 |
| 20 | 67 | Kidney | | 3.2 | 1.7 | 0.8 | 0.8 |
| 21 | 57 | Biliary | | 2.7 | 1.5 | 1.6 | 1.6 |
| 22 | 59 | Pancreas | | 7.8 | 0.8 | 0.6 | 0.6 |
| 23 | 51 | Breast | | 9.5 | 2.2 | 1.1 | 1.6 |
| 24 | 74 | Esophagus | | 4.4 | 2.0 | 1.1 | 1.4 |
| 25 | 60 | Breast | | 9.4 | 2.8 | 1.1 | 1.4 |
| 26 | 72 | Hepatic | | 3.4 | 1.2 | 0.9 | 1.3 |

Table 4 Continued. Summary of the fold changes of mRNA induction

| # | Age | Cancer type | | PD1 PHA | Zymo. | rIL2 | aTCR |
|---|-----|-------------|---|-----|-------|------|------|
| 1 | 55 | Pancreas | | 12.2 | 2.8 | 1.2 | 0.7 |
| 2 | 54 | Colon | | 1.0 | 1.0 | 1.0 | 1.0 |
| 3 | 66 | Pancreas | | 3.7 | 0.7 | 0.5 | 1.0 |
| 4 | 56 | Stomach | | 2.1 | 1.9 | 1.4 | 1.0 |
| 5 | 52 | Ovary | | 7.1 | 3.7 | 1.6 | 1.3 |
| 6 | 64 | Kidney | | 1.3 | 1.9 | 1.1 | 1.4 |
| 7 | 56 | Stomach | | 4.7 | 4.2 | 2.8 | 2.1 |
| 8 | 58 | Breast | | 1.0 | 2.8 | 1.3 | 1.5 |
| 9 | 77 | Lung | | 1.4 | 1.0 | 1.0 | 1.0 |
| 10 | 66 | Ovary | | 4.3 | 5.9 | 3.1 | 1.5 |
| 11 | 43 | Esophagus | | 1.0 | 1.8 | 1.0 | 1.3 |
| 12 | 55 | Prostate | | 4.1 | 3.5 | 2.0 | 1.6 |
| 13 | 63 | Colon | | 4.4 | 3.3 | 1.6 | 1.6 |
| 14 | 72 | Bladder | | 1.4 | 1.2 | 1.0 | 1.0 |
| 15 | 80 | Pancreas | | 1.0 | 1.0 | 1.0 | 1.0 |
| 16 | 63 | Lung | | 1.0 | 1.3 | 1.2 | 1.5 |
| 17 | 63 | Pancreas | | 5.5 | 1.4 | 1.3 | 1.4 |
| 18 | 60 | Stomach | | 2.8 | 1.2 | 0.9 | 2.1 |
| 19 | 59 | Leiomyosarcoma | | 1.6 | 1.4 | 1.0 | 1.1 |
| 20 | 67 | Kidney | | 1.6 | 2.7 | 2.5 | 1.7 |
| 21 | 57 | Biliary | | 1.0 | 1.0 | 1.0 | 1.0 |
| 22 | 59 | Pancreas | | 3.1 | 1.0 | 0.5 | 0.6 |
| 23 | 51 | Breast | | 3.3 | 1.8 | 0.8 | 1.5 |
| 24 | 74 | Esophagus | | 2.8 | 1.9 | 1.1 | 2.8 |
| 25 | 60 | Breast | | 4.1 | 3.8 | 3.1 | 2.4 |
| 26 | 72 | Hepatic | | 1.5 | 3.2 | 2.2 | 1.5 |

Table 4 Continued. Summary of the fold changes of mRNA induction

| # | Age | Cancer type | | FOXP3 | | | |
|---|-----|-------------|---|------|------|------|------|
| | | | | PHA | Zymo. | rIL2 | aTCR |
| 1 | 55 | Pancreas | | 1.4 | 3.9 | 2.5 | 0.7 |
| 2 | 54 | Colon | | 1.0 | 1.0 | 1.0 | 1.0 |
| 3 | 66 | Pancreas | | 4.4 | 4.0 | 3.3 | 1.4 |
| 4 | 56 | Stomach | | 1.8 | 4.5 | 5.0 | 2.0 |
| 5 | 52 | Ovary | | 4.0 | 3.7 | 6.9 | 2.2 |
| 6 | 64 | Kidney | | 1.0 | 1.4 | 1.0 | 1.2 |
| 7 | 56 | Stomach | | 1.0 | 3.4 | 3.1 | 1.6 |
| 8 | 58 | Breast | | 1.0 | 1.0 | 1.0 | 1.0 |
| 9 | 77 | Lung | | 1.9 | 1.0 | 1.0 | 1.0 |
| 10 | 66 | Ovary | | 0.8 | 4.3 | 2.2 | 1.4 |
| 11 | 43 | Esophagus | | 1.5 | 2.9 | 1.5 | 1.1 |
| 12 | 55 | Prostate | | 2.3 | 3.7 | 3.2 | 1.5 |
| 13 | 63 | Colon | | 4.8 | 4.8 | 4.0 | 1.6 |
| 14 | 72 | Bladder | | 9.3 | 4.3 | 12.2 | 4.9 |
| 15 | 80 | Pancreas | | 1.0 | 1.3 | 2.0 | 1.1 |
| 16 | 63 | Lung | | 1.0 | 1.9 | 1.9 | 1.0 |
| 17 | 63 | Pancreas | | 4.6 | 2.8 | 4.1 | 1.1 |
| 18 | 60 | Stomach | | 1.0 | 1.0 | 1.9 | 1.4 |
| 19 | 59 | Leiomyosarcoma | | 2.7 | 3.8 | 7.7 | 2.1 |
| 20 | 67 | Kidney | | 1.0 | 1.0 | 1.6 | 1.0 |
| 21 | 57 | Biliary | | 1.0 | 1.0 | 3.1 | 1.2 |
| 22 | 59 | Pancreas | | 2.8 | 1.3 | 3.1 | 0.7 |
| 23 | 51 | Breast | | 7.1 | 8.4 | 8.2 | 5.4 |
| 24 | 74 | Esophagus | | 0.6 | 1.1 | 2.0 | 1.3 |
| 25 | 60 | Breast | | 5.1 | 2.9 | 11.6 | 3.7 |
| 26 | 72 | Hepatic | | 1.0 | 1.3 | 4.8 | 2.3 |

Table 4 Continued. Summary of the fold changes of mRNA induction

| # | Age | Cancer type | | GMCSF | | | | | |
|---|-----|-------------|---|-------|-----|------|-------|------|------|
|   |     |             |   | PHA | HAG | rIFN | Zymo. | rIL2 | aTCR |
| 1 | 55 | Pancreas | | 34.2 | 1.0 | 1.0 | 45.7 | 2.4 | 1.2 |
| 2 | 54 | Colon | | 1.0 | 1.0 | 1.0 | 1.0 | 1.0 | 1.0 |
| 3 | 66 | Pancreas | | 181.1 | 1.1 | 1.0 | 45.2 | 1.6 | 1.4 |
| 4 | 56 | Stomach | | 21.0 | 0.8 | 0.8 | 52.9 | 1.4 | 0.9 |
| 5 | 52 | Ovary | | 159.9 | 2.7 | 0.8 | 80.2 | 3.7 | 0.8 |
| 6 | 64 | Kidney | | 10.8 | 1.0 | 1.0 | 12.7 | 1.2 | 1.0 |
| 7 | 56 | Stomach | | 10.0 | 1.0 | 1.0 | 18.2 | 1.0 | 1.0 |
| 8 | 58 | Breast | | 1.3 | 1.0 | 1.0 | 2.2 | 1.0 | 1.0 |
| 9 | 77 | Lung | | 3.3 | 1.0 | 1.0 | 1.4 | 1.0 | 1.0 |
| 10 | 66 | Ovary | | 9.9 | 1.2 | 1.0 | 12.4 | 1.0 | 1.8 |
| 11 | 43 | Esophagus | | 11.8 | 1.0 | 1.0 | 22.9 | 1.0 | 1.0 |
| 12 | 55 | Prostate | | 44.4 | 1.0 | 1.0 | 113.6 | 1.1 | 1.7 |
| 13 | 63 | Colon | | 98.0 | 1.7 | 1.0 | 272.0 | 3.2 | 1.0 |
| 14 | 72 | Bladder | | 34.2 | 1.0 | 1.0 | 127.8 | 1.0 | 12.9 |
| 15 | 80 | Pancreas | | 15.0 | 1.0 | 1.0 | 42.0 | 1.0 | 1.1 |
| 16 | 63 | Lung | | 1.0 | 1.0 | 1.0 | 27.0 | 1.0 | 1.0 |
| 17 | 63 | Pancreas | | 37.3 | 0.7 | 0.5 | 130.0 | 1.5 | 1.2 |
| 18 | 60 | Stomach | | 45.9 | 1.2 | 1.0 | 1.0 | 1.0 | 26.3 |
| 19 | 59 | Leiomyosarcoma | | 191.6 | 3.4 | 4.4 | 66.4 | 3.9 | 14.5 |
| 20 | 67 | Kidney | | 24.4 | 2.4 | 1.1 | 28.4 | 1.3 | 9.9 |
| 21 | 57 | Biliary | | 33.1 | 2.4 | 1.0 | 48.8 | 1.3 | 1.2 |
| 22 | 59 | Pancreas | | 24.1 | 1.1 | 1.0 | 37.0 | 1.0 | 1.0 |
| 23 | 51 | Breast | | 157.6 | 1.5 | 1.4 | 69.2 | 1.3 | 1.0 |
| 24 | 74 | Esophagus | | 56.2 | 2.2 | 1.0 | 211.5 | 2.5 | 2.9 |
| 25 | 60 | Breast | | 140.9 | 1.4 | 1.0 | 285.3 | 4.5 | 1.5 |
| 26 | 72 | Hepatic | | 124.1 | 1.0 | 1.7 | 157.0 | 1.0 | 1.9 |

Table 4 Continued. Summary of the fold changes of mRNA induction

| # | Age | Cancer type | | VEGF Zymo. | | IL8 PHA | HAG | Zymo. | aTCR |
|---|-----|-------------|---|------|---|-----|-----|-------|------|
| 1 | 55 | Pancreas | | 1.7 | | 3.9 | 4.4 | 27.6 | 3.5 |
| 2 | 54 | Colon | | 0.8 | | 42.8 | 13.6 | 171.2 | 2.0 |
| 3 | 66 | Pancreas | | 1.9 | | 0.9 | 3.4 | 19.3 | 1.5 |
| 4 | 56 | Stomach | | 12.8 | | 13.7 | 19.2 | 345.0 | 1.9 |
| 5 | 52 | Ovary | | 34.6 | | 15.8 | 14.2 | 550.1 | 2.1 |
| 6 | 64 | Kidney | | 26.9 | | 32.4 | 6.6 | 153.9 | 2.8 |
| 7 | 56 | Stomach | | 4.8 | | 10.0 | 6.5 | 155.6 | 3.9 |
| 8 | 58 | Breast | | 2.4 | | 5.7 | 4.1 | 97.7 | 4.3 |
| 9 | 77 | Lung | | 3.0 | | 9.6 | 3.7 | 9.8 | 2.1 |
| 10 | 66 | Ovary | | 7.8 | | 31.7 | 19.5 | 253.7 | 2.9 |
| 11 | 43 | Esophagus | | 7.0 | | 77.8 | 79.6 | 333.9 | 5.9 |
| 12 | 55 | Prostate | | 162.4 | | 43.2 | 22.1 | 479.6 | 2.5 |
| 13 | 63 | Colon | | 5.5 | | 3.6 | 5.8 | 75.3 | 0.9 |
| 14 | 72 | Bladder | | 22.0 | | 40.3 | 44.5 | 597.8 | 7.2 |
| 15 | 80 | Pancreas | | 17.6 | | 6.8 | 8.7 | 82.1 | 2.7 |
| 16 | 63 | Lung | | 10.6 | | 8.0 | 29.3 | 134.3 | 3.9 |
| 17 | 63 | Pancreas | | 4.9 | | 2.2 | 22.3 | 48.2 | 1.2 |
| 18 | 60 | Stomach | | 1.6 | | 5.3 | 4.9 | 68.9 | 2.4 |
| 19 | 59 | Leiomyosarcoma | | 2.0 | | 15.7 | 14.5 | 98.8 | 2.6 |
| 20 | 67 | Kidney | | 8.9 | | 30.0 | 14.9 | 410.5 | 3.8 |
| 21 | 57 | Biliary | | | | 135.0 | 51.5 | 6057.3 | 10.7 |
| 22 | 59 | Pancreas | | 35.4 | | 7.7 | 43.5 | 851.1 | 1.6 |
| 23 | 51 | Breast | | 21.7 | | 20.0 | 26.7 | 305.0 | 7.7 |
| 24 | 74 | Esophagus | | 3.3 | | 11.4 | 7.0 | 81.7 | 1.8 |
| 25 | 60 | Breast | | 17.1 | | 16.0 | 4.9 | 274.6 | 3.4 |
| 26 | 72 | Hepatic | | 22.5 | | 3.9 | 2.0 | 88.5 | 4.8 |

Table 4 Continued. Summary of the fold changes of mRNA induction

| # | Age | Cancer type | | CCL8 | | | | | |
|---|-----|-------------|---|------|-----|------|-------|------|------|
| | | | | PHA | HAG | rIFN | Zymo. | rIL2 | aTCR |
| 1 | 55 | Pancreas | | 1.0 | 1.0 | 1.0 | 1.0 | 1.0 | 1.1 |
| 2 | 54 | Colon | | 1.0 | 1.0 | 1.0 | 1.0 | 1.0 | 1.0 |
| 3 | 66 | Pancreas | | 1.0 | 1.0 | 1.0 | 1.0 | 1.0 | 1.0 |
| 4 | 56 | Stomach | | 1.0 | 1.0 | 1.0 | 1.0 | 1.0 | 1.0 |
| 5 | 52 | Ovary | | 1.0 | 1.0 | 1.0 | 1.0 | 1.0 | 1.0 |
| 6 | 64 | Kidney | | 1.0 | 1.0 | 1.0 | 1.0 | 1.0 | 1.0 |
| 7 | 56 | Stomach | | 1.0 | 1.0 | 1.0 | 1.0 | 1.0 | 1.0 |
| 8 | 58 | Breast | | 1.0 | 1.0 | 1.0 | 1.0 | 1.0 | 1.0 |
| 9 | 77 | Lung | | 1.0 | 1.0 | 1.0 | 1.0 | 1.0 | 1.0 |
| 10 | 66 | Ovary | | 1.0 | 1.0 | 1.0 | 1.0 | 1.0 | 1.0 |
| 11 | 43 | Esophagus | | 1.0 | 1.0 | 1.0 | 1.0 | 1.0 | 1.0 |
| 12 | 55 | Prostate | | 1.0 | 1.0 | 1.0 | 1.0 | 1.0 | 1.0 |
| 13 | 63 | Colon | | 1.0 | 1.0 | 1.0 | 1.0 | 1.0 | 1.0 |
| 14 | 72 | Bladder | | 1.0 | 1.0 | 1.0 | 1.0 | 1.0 | 1.0 |
| 15 | 80 | Pancreas | | 1.0 | 1.0 | 1.0 | 1.0 | 1.0 | 1.0 |
| 16 | 63 | Lung | | 1.0 | 1.0 | 1.0 | 1.0 | 1.0 | 1.0 |
| 17 | 63 | Pancreas | | 5.1 | 1.0 | 1.0 | 2.5 | 1.2 | 1.6 |
| 18 | 60 | Stomach | | 1.0 | 1.0 | 1.0 | 1.0 | 1.0 | 1.4 |
| 19 | 59 | Leiomyosarcoma | | 5.8 | 1.0 | 1.0 | 14.9 | 1.5 | 3.3 |
| 20 | 67 | Kidney | | 1.0 | 1.0 | 1.0 | 1.0 | 1.0 | 1.0 |
| 21 | 57 | Biliary | | 1.0 | 1.0 | 1.0 | 1.0 | 1.0 | 1.0 |
| 22 | 59 | Pancreas | | 136.2 | 2.6 | 1.0 | 1.0 | 7.7 | 1.0 |
| 23 | 51 | Breast | | 1.0 | 1.0 | 1.0 | 1.0 | 1.0 | 1.0 |
| 24 | 74 | Esophagus | | 1.0 | 1.0 | 1.0 | 1.0 | 1.0 | 1.0 |
| 25 | 60 | Breast | | 1.0 | 1.0 | 1.0 | 1.0 | 1.0 | 1.0 |
| 26 | 72 | Hepatic | | 1.0 | 1.0 | 1.0 | 1.0 | 1.0 | 1.0 |

Table 4 Continued. Summary of the fold changes of mRNA induction

| # | Age | Cancer type | | CXCL3 | | | | | IL2 | |
|---|---|---|---|---|---|---|---|---|---|---|
| | | | | PHA | HAG | Zymo. | rIL2 | aTCR | PHA | aTCR |
| 1 | 55 | Pancreas | | 0.8 | 3.4 | 1.5 | 0.8 | 0.5 | | 1.0 |
| 2 | 54 | Colon | | 1.0 | 1.0 | 1.1 | 1.0 | 1.0 | | 1.0 |
| 3 | 66 | Pancreas | | 0.2 | 2.6 | 1.8 | 1.2 | 1.6 | | 1.2 |
| 4 | 56 | Stomach | | 2.1 | 13.4 | 23.3 | 2.9 | 2.2 | | 1.0 |
| 5 | 52 | Ovary | | 2.5 | 14.9 | 68.4 | 4.1 | 3.4 | | 1.0 |
| 6 | 64 | Kidney | | 1.0 | 1.1 | 3.0 | 1.0 | 1.3 | | 1.0 |
| 7 | 56 | Stomach | | 1.0 | 2.8 | 16.0 | 8.2 | 7.0 | | 1.0 |
| 8 | 58 | Breast | | 1.0 | 1.0 | 21.7 | 9.5 | 10.7 | | 1.0 |
| 9 | 77 | Lung | | 1.2 | 1.7 | 3.3 | 1.1 | 1.0 | | 1.0 |
| 10 | 66 | Ovary | | 1.9 | 11.8 | 16.7 | 1.4 | 1.9 | | 1.0 |
| 11 | 43 | Esophagus | | 5.5 | 30.9 | 21.3 | 3.9 | 3.8 | | 1.0 |
| 12 | 55 | Prostate | | 27.2 | 38.5 | 68.8 | 2.6 | 5.3 | | 1.0 |
| 13 | 63 | Colon | | 1.5 | 2.7 | 7.5 | 1.9 | 1.5 | | 1.0 |
| 14 | 72 | Bladder | | 89.6 | 123.0 | 335.7 | 29.8 | 30.6 | | 1.0 |
| 15 | 80 | Pancreas | | 2.8 | 12.3 | 75.8 | 34.8 | 40.1 | | 1.0 |
| 16 | 63 | Lung | | 1.7 | 19.6 | 78.1 | 15.6 | 40.7 | | 1.0 |
| 17 | 63 | Pancreas | | 0.4 | 11.4 | 3.0 | 0.6 | 0.6 | | 1.0 |
| 18 | 60 | Stomach | | 4.8 | 4.0 | 35.8 | 0.6 | 1.5 | | 2.1 |
| 19 | 59 | Leiomyosarcoma | | 6.7 | 22.1 | 26.1 | 2.9 | 4.0 | | 1.0 |
| 20 | 67 | Kidney | | 4.2 | 9.5 | 41.7 | 0.6 | 1.9 | | 1.0 |
| 21 | 57 | Biliary | | 9.7 | 12.1 | 158.4 | 2.0 | 2.3 | | 1.0 |
| 22 | 59 | Pancreas | | 6.6 | 64.3 | 142.3 | 4.5 | 6.1 | | 1.0 |
| 23 | 51 | Breast | | 1.0 | 4.1 | 66.2 | 6.9 | 18.0 | | 1.0 |
| 24 | 74 | Esophagus | | 4.4 | 5.1 | 14.3 | 0.9 | 1.1 | | 1.0 |
| 25 | 60 | Breast | | 4.6 | 6.5 | 31.4 | 1.7 | 1.5 | | 1.0 |
| 26 | 72 | Hepatic | | 1.7 | 5.8 | 12.0 | 2.5 | 2.5 | | 1.0 |

Table 5: Fold Change of mRNAs by Stimulant Used - Beta Actin v. Beta Actin

| | | ACTB | | | | | | |
|---|---|---|---|---|---|---|---|---|
| | | PHA | HAG | rIFN | Zymo. | rIL2 | aTCR | Pici. |
| ACTB | PHA | | 0.00 | 0.10 | 0.00 | 0.07 | 0.06 | 0.00 |
| | HAG | | | 0.09 | 0.17 | 0.02 | 0.01 | 0.02 |
| | rIFN | | | | 0.01 | **0.61** | **0.54** | 0.05 |
| | Zymo. | | | | | 0.04 | 0.01 | **0.31** |
| | rIL2 | | | | | | **0.84** | **0.42** |
| | aTCR | | | | | | | **0.46** |
| | Pici. | | | | | | | |

Table 5 Continued: Fold Change of mRNAs by Stimulant Used - Beta Actin v. Beta-2 Microglobulin

|  |  | B2M |  |  |  |  |  |  |
|---|---|---|---|---|---|---|---|---|
|  |  | PHA | HAG | rIFN | Zymo. | rIL2 | aTCR | Pici. |
| ACTB | PHA | **0.45** | 0.01 | 0.06 | 0.07 | 0.04 | 0.08 | 0.06 |
|  | HAG | 0.04 | 0.10 | 0.00 | 0.12 | 0.00 | 0.02 | 0.01 |
|  | rIFN | 0.21 | 0.00 | **0.62** | 0.04 | **0.44** | **0.34** | 0.13 |
|  | Zymo. | 0.21 | 0.05 | 0.01 | **0.32** | 0.01 | 0.00 | 0.05 |
|  | rIL2 | 0.11 | 0.00 | **0.62** | 0.00 | **0.77** | **0.45** | **0.43** |
|  | aTCR | 0.16 | 0.00 | **0.60** | 0.00 | **0.67** | **0.62** | **0.57** |
|  | Pici. | 0.00 | 0.01 | 0.19 | 0.06 | **0.31** | 0.22 | **0.65** |

Table 5 Continued: Fold Change of mRNAs by Stimulant Used - Beta Actin v. Interferon Gamma

|  |  | IFNG |  |  |  |  |  |
|---|---|---|---|---|---|---|---|
|  |  | PHA | rIFN | Zymo. | rIL2 | aTCR | Pici. |
| ACTB | PHA | 0.02 | 0.12 | 0.16 | 0.07 | 0.00 | 0.09 |
|  | HAG | 0.00 | 0.03 | 0.06 | 0.02 | 0.00 | 0.22 |
|  | rIFN | 0.11 | 0.00 | 0.11 | 0.03 | 0.12 | 0.13 |
|  | Zymo. | 0.14 | 0.01 | 0.02 | 0.00 | 0.01 | 0.00 |
|  | rIL2 | 0.21 | 0.03 | 0.15 | 0.03 | 0.23 | 0.14 |
|  | aTCR | 0.21 | 0.02 | 0.16 | 0.04 | 0.18 | 0.13 |
|  | Pici. | 0.25 | 0.02 | 0.14 | 0.01 | 0.09 | 0.05 |

Table 5 Continued: Fold Change of mRNAs by Stimulant Used - Beta Actin v. TNFSF 1 and 2

|  |  | TNFSF1 |  |  |  | TNFSF2 |  |  |  |  |
|---|---|---|---|---|---|---|---|---|---|---|
|  |  | PHA | Zymo. | rIL2 | aTCR | PHA | HAG | Zymo. | rIL2 | aTCR |
| ACTB | PHA | 0.01 | 0.08 | 0.00 | 0.00 | 0.05 | 0.00 | 0.02 | 0.01 | 0.03 |
|  | HAG | 0.00 | 0.12 | 0.00 | 0.00 | 0.00 | 0.03 | 0.09 | 0.00 | 0.00 |
|  | rIFN | 0.01 | 0.22 | 0.07 | 0.02 | 0.11 | 0.26 | 0.17 | 0.02 | 0.01 |
|  | Zymo. | 0.17 | 0.03 | 0.00 | 0.00 | 0.02 | 0.01 | 0.13 | 0.09 | 0.07 |
|  | rIL2 | 0.01 | 0.09 | 0.02 | 0.01 | 0.03 | 0.11 | 0.01 | 0.14 | 0.00 |
|  | aTCR | 0.00 | 0.02 | 0.00 | 0.00 | 0.00 | 0.05 | 0.00 | 0.13 | 0.00 |
|  | Pici. | 0.02 | 0.02 | 0.00 | 0.01 | 0.01 | 0.01 | 0.20 | 0.21 | 0.09 |

Table 5 Continued: Fold Change of mRNAs by Stimulant Used Beta Actin v. TNFSF-5 and IL-10

|  |  | TNFSF5 |  |  | IL10 |  |  |  |  |  |
|---|---|---|---|---|---|---|---|---|---|---|
|  |  | PHA | rIL2 | aTCR | PHA | HAG | rIFN | Zymo. | rIL2 | aTCR |
| ACTB | PHA | 0.00 | 0.00 | 0.01 | 0.05 | 0.08 | **0.32** | 0.12 | 0.06 | 0.01 |
|  | HAG | 0.02 | 0.00 | 0.04 | 0.11 | **0.32** | 0.00 | 0.09 | 0.10 | 0.07 |

(continued)

|  |  | TNFSF5 |  |  | IL10 |  |  |  |  |  |
|---|---|---|---|---|---|---|---|---|---|---|
|  |  | PHA | rIL2 | aTCR | PHA | HAG | rIFN | Zymo. | rIL2 | aTCR |
|  | rIFN | 0.09 | 0.09 | 0.08 | 0.18 | 0.02 | 0.15 | 0.24 | 0.01 | 0.00 |
|  | Zymo. | 0.00 | 0.05 | 0.10 | 0.02 | 0.05 | 0.02 | 0.00 | 0.06 | 0.05 |
|  | rIL2 | 0.01 | **0.35** | **0.31** | 0.13 | 0.01 | 0.15 | 0.13 | 0.02 | 0.00 |
|  | aTCR | 0.01 | 0.25 | 0.22 | 0.11 | 0.00 | 0.18 | 0.08 | 0.04 | 0.00 |
|  | Pici. | 0.04 | 0.23 | 0.29 | 0.00 | 0.05 | 0.02 | 0.00 | 0.01 | 0.01 |

Table 5 Continued: Fold Change of mRNAs by Stimulant Used - Beta Actin v. TGF Beta, CTLA4 and PD1

|  |  | TGFB | CTLA4 |  |  |  | PD1 |  |  |  |
|---|---|---|---|---|---|---|---|---|---|---|
|  |  | PHA | PHA | Zymo. | rIL2 | aTCR | PHA | Zymo. | rIL2 | aTCR |
| ACTB | PHA | 0.01 | 0.00 | 0.11 | 0.08 | 0.08 | 0.00 | 0.01 | 0.00 | 0.00 |
|  | HAG | 0.00 | 0.01 | 0.04 | 0.01 | 0.01 | 0.01 | 0.07 | 0.03 | 0.05 |
|  | rIFN | 0.06 | 0.14 | 0.00 | 0.02 | 0.03 | 0.08 | 0.00 | 0.02 | 0.03 |
|  | Zymo. | 0.01 | 0.07 | 0.02 | 0.00 | 0.05 | 0.01 | 0.21 | 0.17 | 0.11 |
|  | rIL2 | 0.03 | 0.07 | 0.04 | 0.14 | 0.01 | 0.10 | 0.02 | 0.08 | 0.00 |
|  | aTCR | 0.01 | 0.01 | 0.03 | 0.10 | 0.04 | 0.11 | 0.01 | 0.09 | 0.01 |
|  | Pici. | 0.01 | 0.00 | 0.04 | 0.05 | 0.07 | 0.12 | 0.07 | 0.16 | 0.06 |

Table 5 Continued: Fold Change of mRNAs by Stimulant Used - Beta Actin v. FoxP3 and GMCSF

|  |  | FOXP3 |  |  |  | GMCSF |  |  |  |  |
|---|---|---|---|---|---|---|---|---|---|---|
|  |  | PHA | Zymo. | rIL2 | aTCR | PHA | HAG | Zymo. | rIL2 | aTCR |
| ACTB | PHA | 0.01 | 0.17 | 0.05 | 0.01 | 0.00 | 0.00 | 0.08 | 0.01 | 0.01 |
|  | HAG | 0.00 | 0.00 | 0.00 | 0.00 | 0.01 | 0.00 | 0.11 | 0.00 | 0.01 |
|  | rIFN | 0.07 | 0.17 | 0.09 | 0.02 | 0.11 | 0.02 | 0.19 | 0.04 | 0.02 |
|  | Zymo. | 0.18 | 0.01 | 0.00 | 0.01 | 0.00 | 0.07 | 0.01 | 0.00 | 0.01 |
|  | rIL2 | 0.06 | 0.10 | 0.01 | 0.00 | 0.01 | 0.02 | 0.01 | 0.00 | 0.04 |
|  | aTCR | 0.04 | 0.09 | 0.00 | 0.01 | 0.00 | 0.00 | 0.00 | 0.00 | 0.04 |
|  | Pici. | 0.02 | 0.03 | 0.06 | 0.16 | 0.07 | 0.07 | 0.11 | 0.00 | 0.01 |

Table 5 Continued: Fold Change of mRNAs by Stimulant Used - Beta Actin v. VEGF, IL8 and CCL8

|  |  | VEGF | IL8 |  |  |  | CCL8 |  |  |  |  |  |
|---|---|---|---|---|---|---|---|---|---|---|---|---|
|  |  | Zymo. | PHA | HAG | Zymo. | aTCR | PHA | HAG | rIFN | Zymo. | rIL2 | aTCR |
| ACTB | PHA | 0.01 | 0.01 | 0.07 | 0.00 | 0.01 | 0.02 | 0.00 | 0 | 0.18 | 0.00 | 0.12 |
|  | HAG | 0.13 | 0.17 | 0.16 | 0.13 | 0.00 | 0.05 | 0.06 | 0 | 0.00 | 0.06 | 0.00 |
|  | rIFN | 0.13 | 0.01 | 0.22 | 0.13 | 0.01 | 0.01 | 0.02 | 0 | 0.00 | 0.01 | 0.00 |
|  | Zymo. | 0.03 | **0.30** | 0.01 | **0.31** | 0.22 | 0.05 | 0.01 | 0 | 0.03 | 0.02 | 0.04 |
|  | rIL2 | 0.03 | 0.03 | 0.11 | 0.00 | 0.13 | 0.05 | 0.04 | 0 | 0.00 | 0.04 | 0.00 |

(continued)

|  |  | VEGF | IL8 |  |  |  | CCL8 |  |  |  |  |  |
|---|---|---|---|---|---|---|---|---|---|---|---|---|
|  |  | Zymo. | PHA | HAG | Zymo. | aTCR | PHA | HAG | rIFN | Zymo. | rIL2 | aTCR |
|  | aTCR | 0.00 | 0.01 | 0.11 | 0.01 | 0.17 | 0.06 | 0.05 | 0 | 0.00 | 0.06 | 0.01 |
|  | Pici. | 0.10 | 0.14 | 0.01 | 0.14 | 0.26 | 0.04 | 0.02 | 0 | 0.00 | 0.02 | 0.02 |

Table 5 Continued: Fold Change of mRNAs by Stimulant Used - Beta Actin v. CXCL3 and IL2

|  |  | CXCL3 |  |  |  |  | IL2 |  |
|---|---|---|---|---|---|---|---|---|
|  |  | PHA | HAG | Zymo. | rIL2 | aTCR | PHA | aTCR |
| ACTB | PHA | 0.02 | 0.06 | 0.00 | 0.05 | 0.06 | 0.03 | 0.07 |
|  | HAG | 0.32 | 0.19 | 0.06 | 0.01 | 0.01 | 0.01 | 0.05 |
|  | rIFN | 0.08 | 0.24 | 0.15 | 0.02 | 0.05 | 0.11 | 0.03 |
|  | Zymo. | 0.10 | 0.00 | 0.04 | 0.00 | 0.00 | 0.08 | 0.01 |
|  | rIL2 | 0.02 | 0.14 | 0.05 | 0.02 | 0.07 | 0.24 | 0.12 |
|  | aTCR | 0.00 | 0.06 | 0.01 | 0.00 | 0.01 | 0.20 | 0.10 |
|  | Pici. | 0.11 | 0.01 | 0.06 | 0.01 | 0.00 | 0.23 | 0.06 |

Table 5 Continued: Fold Change of mRNAs by Stimulant Used - Beta-2 Microglobulin v. Beta-2 Microglobulin

|  |  | B2M |  |  |  |  |  |  |
|---|---|---|---|---|---|---|---|---|
|  |  | PHA | HAG | rIFN | Zymo. | rIL2 | aTCR | Pici. |
| B2M | PHA |  | 0.02 | 0.10 | 0.11 | 0.16 | 0.28 | 0.14 |
|  | HAG |  |  | 0.03 | 0.01 | 0.03 | 0.00 | 0.05 |
|  | rIFN |  |  |  | 0.02 | **0.61** | **0.52** | **0.33** |
|  | Zymo. |  |  |  |  | 0.03 | 0.03 | 0.01 |
|  | rIL2 |  |  |  |  |  | **0.64** | **0.45** |
|  | aTCR |  |  |  |  |  |  | **0.51** |
|  | Pici. |  |  |  |  |  |  |  |

Table 5 Continued: Fold Change of mRNAs by Stimulant Used - Beta-2 Microglobulin v. Interferon Gamma

|  |  | IFNG |  |  |  |  |  |
|---|---|---|---|---|---|---|---|
|  |  | PHA | rIFN | Zymo. | rIL2 | aTCR | Pici. |
| B2M | PHA | 0.00 | 0.00 | 0.09 | 0.02 | 0.03 | 0.05 |
|  | HAG | 0.01 | 0.05 | 0.01 | 0.10 | 0.00 | 0.08 |
|  | rIFN | 0.06 | 0.02 | 0.03 | 0.01 | 0.15 | 0.01 |
|  | Zymo. | 0.00 | 0.23 | 0.14 | 0.06 | 0.05 | 0.05 |
|  | rIL2 | 0.03 | 0.13 | 0.02 | 0.02 | 0.18 | 0.02 |
|  | aTCR | 0.01 | 0.06 | 0.01 | 0.00 | 0.06 | 0.00 |
|  | Pici. | 0.03 | 0.01 | 0.03 | 0.00 | 0.06 | 0.00 |

Table 5 Continued: Fold Change of mRNAs by Stimulant Used - Beta-2 Microglobulin v. TNFSF1 and 2

|  |  | TNFSF1 |  |  |  | TNFSF2 |  |  |  |  |
|---|---|---|---|---|---|---|---|---|---|---|
|  |  | PHA | Zymo. | rIL2 | aTCR | PHA | HAG | Zymo. | rIL2 | aTCR |
| B2M | PHA | 0.15 | 0.05 | 0.00 | 0.01 | 0.02 | 0.00 | 0.03 | 0.00 | 0.01 |
|  | HAG | 0.00 | 0.00 | 0.12 | 0.02 | 0.04 | 0.05 | 0.08 | 0.01 | 0.00 |
|  | rIFN | 0.00 | 0.03 | 0.01 | 0.05 | 0.00 | 0.10 | 0.00 | 0.13 | 0.00 |
|  | Zymo. | 0.03 | **0.41** | 0.27 | 0.00 | 0.04 | 0.01 | 0.17 | 0.29 | 0.00 |
|  | rIL2 | 0.00 | 0.03 | 0.00 | 0.04 | 0.01 | 0.05 | 0.00 | 0.16 | 0.02 |
|  | aTCR | 0.01 | 0.00 | 0.00 | 0.00 | 0.00 | 0.03 | 0.00 | 0.08 | 0.00 |
|  | Pici. | 0.02 | 0.02 | 0.00 | 0.00 | 0.04 | 0.01 | 0.13 | 0.08 | 0.05 |

Table 5 Continued: Fold Change of mRNAs by Stimulant Used - Beta-2 Microglobulin v. TNFSF5 and IL10

|  |  | TNFSF5 |  |  | IL10 |  |  |  |  |  |
|---|---|---|---|---|---|---|---|---|---|---|
|  |  | PHA | rIL2 | aTCR | PHA | HAG | rIFN | Zymo. | rIL2 | aTCR |
| B2M | PHA | 0.00 | 0.00 | 0.03 | 0.05 | 0.00 | **0.38** | 0.06 | 0.19 | 0.06 |
|  | HAG | 0.16 | 0.00 | 0.04 | 0.02 | 0.01 | 0.02 | 0.01 | 0.01 | 0.00 |
|  | rIFN | 0.00 | 0.22 | 0.22 | 0.03 | 0.01 | 0.28 | 0.05 | 0.06 | 0.00 |
|  | Zymo. | 0.01 | 0.01 | 0.02 | 0.07 | 0.05 | 0.00 | 0.15 | 0.00 | 0.01 |
|  | rIL2 | 0.01 | **0.40** | **0.33** | 0.03 | 0.00 | 0.26 | 0.05 | 0.03 | 0.00 |
|  | aTCR | 0.00 | 0.19 | 0.13 | 0.01 | 0.07 | **0.30** | 0.01 | 0.13 | 0.01 |
|  | Pici. | 0.15 | **0.34** | 0.27 | 0.00 | 0.05 | 0.14 | 0.00 | 0.05 | 0.00 |

Table 5 Continued: Fold Change of mRNAs by Stimulant Used - Beta-2 Microglobulin v. TGF Beta, CTLA4 and PD1

|  |  | TGFB | CTLA4 |  |  |  | PD1 |  |  |  |
|---|---|---|---|---|---|---|---|---|---|---|
|  |  | PHA | PHA | Zymo. | rIL2 | aTC R | PHA | Zymo. | rIL2 | aTC R |
| B2M | PHA | 0.02 | 0.05 | 0.00 | 0.00 | 0.03 | 0.01 | 0.05 | 0.01 | 0.03 |
|  | HAG | 0.13 | 0.11 | 0.06 | 0.05 | 0.00 | 0.01 | 0.02 | 0.00 | 0.01 |
|  | rIFN | 0.11 | 0.01 | 0.02 | 0.06 | 0.00 | 0.00 | 0.06 | 0.07 | 0.00 |
|  | Zymo. | 0.10 | 0.05 | **0.34** | 0.16 | 0.09 | 0.15 | **0.56** | 0.26 | 0.04 |
|  | rIL2 | 0.01 | 0.01 | 0.10 | 0.16 | 0.00 | 0.01 | 0.07 | 0.09 | 0.00 |
|  | aTCR | 0.01 | 0.00 | 0.01 | 0.03 | 0.02 | 0.02 | 0.03 | 0.04 | 0.00 |
|  | Pici. | 0.00 | 0.02 | 0.01 | 0.02 | 0.02 | 0.02 | 0.04 | 0.09 | 0.00 |

Table 5 Continued: Fold Change of mRNAs by Stimulant Used - Beta-2 Microglobulin v. FoxP3 and GMCSF

|  |  | FOXP3 |  |  |  | GMCSF |  |  |  |  |
|---|---|---|---|---|---|---|---|---|---|---|
|  |  | PHA | Zymo. | rIL2 | aTCR | PHA | HAG | Zymo. | rIL2 | aTCR |
| B2M | PHA | 0.06 | 0.01 | 0.01 | 0.00 | 0.00 | 0.01 | 0.03 | 0.01 | 0.00 |
|  | HAG | 0.02 | 0.01 | 0.01 | 0.02 | 0.04 | 0.11 | 0.08 | 0.01 | 0.00 |

(continued)

| | | FOXP3 | | | | GMCSF | | | | |
|---|---|---|---|---|---|---|---|---|---|---|
| | | PHA | Zymo. | rIL2 | aTCR | PHA | HAG | Zymo. | rIL2 | aTCR |
| | rIFN | 0.03 | 0.05 | 0.01 | 0.00 | 0.00 | 0.00 | 0.00 | 0.00 | 0.03 |
| | Zymo. | 0.00 | 0.24 | 0.04 | 0.04 | 0.03 | 0.02 | 0.16 | 0.02 | 0.12 |
| | rIL2 | 0.00 | 0.01 | 0.00 | 0.01 | 0.00 | 0.00 | 0.00 | 0.02 | 0.13 |
| | aTCR | 0.00 | 0.00 | 0.00 | 0.01 | 0.00 | 0.03 | 0.00 | 0.01 | 0.05 |
| | Pici. | 0.00 | 0.00 | 0.08 | 0.10 | 0.14 | 0.03 | 0.09 | 0.00 | 0.01 |

Table 5 Continued: Fold Change of mRNAs by Stimulant Used - Beta-2 Microglobulin v. VEGF, IL8, and CCL8

| | | VEGF | IL8 | | | | CCL8 | | | | | |
|---|---|---|---|---|---|---|---|---|---|---|---|---|
| | | Zymo. | PHA | HAG | Zymo. | aTCR | PHA | HAG | rIFN | Zymo. | rIL2 | aTCR |
| B2M | PHA | 0.00 | 0.01 | 0.05 | 0.11 | 0.00 | 0.01 | 0.00 | 0 | 0.02 | 0.01 | 0.02 |
| | HAG | 0.02 | 0.02 | 0.06 | 0.06 | 0.01 | 0.08 | 0.05 | 0 | 0.01 | 0.06 | 0.00 |
| | rIFN | 0.02 | 0.01 | 0.22 | 0.08 | 0.01 | 0.01 | 0.01 | 0 | 0.00 | 0.01 | 0.00 |
| | Zymo. | 0.12 | 0.02 | 0.00 | 0.03 | 0.03 | 0.09 | 0.05 | 0 | 0.04 | 0.07 | 0.07 |
| | rIL2 | 0.02 | 0.00 | 0.07 | 0.01 | 0.05 | 0.02 | 0.02 | 0 | 0.00 | 0.02 | 0.01 |
| | aTCR | 0.00 | 0.00 | 0.05 | 0.05 | 0.05 | 0.03 | 0.02 | 0 | 0.00 | 0.03 | 0.01 |
| | Pici. | 0.08 | 0.02 | 0.01 | 0.03 | 0.19 | 0.00 | 0.00 | 0 | 0.00 | 0.00 | 0.00 |

Table 5 Continued: Fold Change of mRNAs by Stimulant Used - Beta-2 Microglobulin v. CXCL3 and IL2

| | | CXCL3 | | | | | IL2 | |
|---|---|---|---|---|---|---|---|---|
| | | PHA | HAG | Zymo. | rIL2 | aTCR | PHA | aTCR |
| B2M | PHA | 0.00 | 0.02 | 0.00 | 0.02 | 0.00 | 0.02 | 0.00 |
| | HAG | 0.00 | 0.00 | 0.04 | 0.00 | 0.00 | 0.01 | 0.09 |
| | rIFN | 0.03 | 0.11 | 0.10 | 0.03 | 0.08 | 0.03 | 0.10 |
| | Zymo. | 0.00 | 0.00 | 0.00 | 0.02 | 0.00 | 0.00 | 0.15 |
| | rIL2 | 0.05 | 0.11 | 0.06 | 0.02 | 0.09 | 0.06 | 0.17 |
| | aTCR | 0.00 | 0.01 | 0.02 | 0.00 | 0.02 | 0.01 | 0.04 |
| | Pici. | 0.05 | 0.02 | 0.06 | 0.00 | 0.00 | 0.02 | 0.02 |

Table 5 Continued: Fold Change of mRNAs by Stimulant Used - IFNG v. IFNG

| | | IFNG | | | | | |
|---|---|---|---|---|---|---|---|
| | | PHA | rIFN | Zymo. | rIL2 | aTCR | Pici. |
| IFNG | PHA | | 0.00 | 0.53 | 0.20 | 0.17 | **0.33** |
| | rIFN | | | 0.13 | **0.50** | 0.20 | 0.03 |
| | Zymo. | | | | **0.34** | 0.03 | **0.67** |
| | rIL2 | | | | | 0.01 | 0.26 |
| | aTCR | | | | | | 0.01 |

(continued)

| | | IFNG | | | | | | |
|---|---|---|---|---|---|---|---|---|
| | | PHA | rIFN | Zymo. | rIL2 | aTCR | Pici. | |
| | Pici. | | | | | | | |

Table 5 Continued: Fold Change of mRNAs by Stimulant Used - IFNG v. TNFSF 1 and 2

| | | TNFSF1 | | | | TNFSF2 | | | | |
|---|---|---|---|---|---|---|---|---|---|---|
| | | PHA | Zymo. | rIL2 | aTCR | PHA | HAG | Zymo. | rIL2 | aTCR |
| IFNG | PHA | 0.11 | 0.01 | 0.02 | 0.16 | 0.01 | 0.00 | 0.02 | 0.06 | 0.04 |
| | rIFN | 0.03 | 0.17 | 0.02 | 0.00 | 0.17 | 0.03 | 0.00 | 0.03 | 0.23 |
| | Zymo. | 0.10 | 0.18 | 0.04 | 0.05 | 0.02 | 0.00 | 0.00 | 0.02 | 0.10 |
| | rIL2 | 0.00 | 0.09 | 0.00 | 0.03 | 0.10 | 0.02 | 0.00 | 0.02 | 0.17 |
| | aTCR | 0.03 | 0.00 | 0.03 | 0.00 | 0.12 | 0.02 | 0.01 | 0.07 | **0.31** |
| | Pici. | 0.03 | 0.09 | 0.00 | 0.04 | 0.02 | 0.00 | 0.00 | 0.07 | 0.12 |

Table 5 Continued: Fold Change of mRNAs by Stimulant Used - IFNG v. TNFSF5 and IL10

| | | TNFSF5 | | | IL10 | | | | | |
|---|---|---|---|---|---|---|---|---|---|---|
| | | PHA | rIL2 | aTCR | PHA | HAG | rIFN | Zymo. | rIL2 | aTCR |
| IFNG | PHA | 0.21 | 0.02 | 0.00 | 0.13 | 0.00 | 0.01 | 0.11 | 0.02 | 0.03 |
| | rIFN | 0.02 | 0.02 | 0.07 | 0.02 | 0.00 | 0.01 | 0.05 | 0.01 | 0.15 |
| | Zymo. | 0.10 | 0.01 | 0.01 | **0.31** | 0.02 | 0.03 | **0.39** | 0.01 | 0.04 |
| | rIL2 | 0.10 | 0.00 | 0.01 | 0.14 | 0.02 | 0.00 | 0.10 | 0.01 | 0.01 |
| | aTCR | 0.01 | 0.02 | 0.01 | 0.00 | 0.02 | 0.14 | 0.00 | 0.05 | 0.16 |
| | Pici. | 0.11 | 0.00 | 0.01 | 0.31 | 0.06 | 0.01 | **0.40** | 0.04 | 0.06 |

Table 5 Continued: Fold Change of mRNAs by Stimulant Used - IFNG v. TGFB, CTLA4, and PD1

| | | TGFB | CTLA4 | | | | PD1 | | | |
|---|---|---|---|---|---|---|---|---|---|---|
| | | PHA | PHA | Zymo. | rIL2 | aTCR | PHA | Zymo. | rIL2 | aTCR |
| IFNG | PHA | 0.00 | 0.07 | 0.02 | 0.03 | 0.08 | **0.44** | 0.00 | 0.11 | 0.05 |
| | rIFN | 0.10 | 0.03 | **0.64** | 0.54 | 0.05 | 0.01 | 0.13 | 0.13 | 0.01 |
| | Zymo. | 0.01 | 0.07 | 0.05 | 0.03 | 0.01 | **0.38** | 0.07 | 0.00 | 0.09 |
| | rIL2 | 0.03 | 0.05 | 0.15 | 0.08 | 0.00 | 0.09 | 0.09 | 0.03 | 0.03 |
| | aTCR | 0.07 | 0.00 | 0.35 | 0.41 | 0.01 | 0.06 | 0.03 | 0.07 | 0.11 |
| | Pici. | 0.00 | 0.07 | 0.01 | 0.01 | 0.02 | 0.11 | 0.01 | 0.03 | 0.14 |

Table 5 Continued: Fold Change of mRNAs by Stimulant Used - IFNG v. FoxP3 and GMCSF

|  |  | FOXP3 |  |  |  | GMCSF |  |  |  |  |
|---|---|---|---|---|---|---|---|---|---|---|
|  |  | PHA | Zymo. | rIL2 | aTCR | PHA | HAG | Zymo. | rIL2 | aTCR |
| IFNG | PHA | 0.19 | 0.27 | 0.04 | 0.00 | 0.18 | 0.00 | 0.01 | 0.03 | 0.01 |
|  | rIFN | 0.00 | 0.15 | 0.00 | 0.00 | 0.02 | 0.02 | 0.08 | 0.00 | 0.19 |
|  | Zymo. | 0.11 | **0.39** | 0.08 | 0.00 | 0.08 | 0.03 | 0.08 | 0.03 | 0.07 |
|  | rIL2 | 0.01 | 0.13 | 0.01 | 0.00 | 0.00 | 0.01 | 0.02 | 0.00 | 0.10 |
|  | aTCR | 0.01 | 0.01 | 0.04 | 0.06 | 0.13 | 0.02 | 0.00 | 0.01 | **0.33** |
|  | Pici. | 0.12 | 0.20 | 0.08 | 0.00 | 0.05 | 0.01 | 0.08 | 0.00 | 0.04 |

Table 5 Continued: Fold Change of mRNAs by Stimulant Used - IFNG v. VEGF, IL8, and CCL8

|  |  | VEGF | IL8 |  |  |  | CCL8 |  |  |  |  |  |
|---|---|---|---|---|---|---|---|---|---|---|---|---|
|  |  | Zymo. | PHA | HAG | Zymo. | aTCR | PHA | HAG | rIFN | Zymo. | rIL2 | aTCR |
| IFNG | PHA | 0.00 | 0.15 | 0.01 | 0.04 | 0.14 | 0.07 | 0.05 | 0 | 0.00 | 0.05 | 0.01 |
|  | rIFN | 0.01 | 0.03 | 0.06 | 0.00 | 0.00 | 0.01 | 0.05 | 0 | 0.01 | 0.04 | 0.01 |
|  | Zymo. | 0.08 | 0.00 | 0.11 | 0.01 | 0.06 | 0.06 | 0.03 | 0 | 0.01 | 0.04 | 0.01 |
|  | rIL2 | 0.05 | 0.01 | 0.17 | 0.04 | 0.02 | 0.09 | 0.06 | 0 | 0.00 | 0.07 | 0.00 |
|  | aTCR | 0.04 | 0.03 | 0.00 | 0.02 | 0.02 | 0.00 | 0.02 | 0 | 0.02 | 0.01 | 0.10 |
|  | Pici. | 0.20 | 0.02 | **0.32** | 0.15 | 0.01 | 0.30 | 0.30 | 0 | 0.00 | 0.31 | 0.00 |

Table 5 Continued: Fold Change of mRNAs by Stimulant Used - IFNG v. CXCL3 and IL2

|  |  | CXCL3 |  |  |  |  | IL2 |  |
|---|---|---|---|---|---|---|---|---|
|  |  | PHA | HAG | Zymo. | rIL2 | aTCR | PHA | aTCR |
| IFNG | PHA | 0.10 | 0.01 | 0.03 | 0.08 | 0.07 | 0.73 | 0.09 |
|  | rIFN | 0.04 | 0.01 | 0.05 | 0.00 | 0.01 | 0.01 | **0.54** |
|  | Zymo. | 0.01 | 0.11 | 0.00 | 0.02 | 0.03 | 0.50 | 0.02 |
|  | rIL2 | 0.02 | 0.09 | 0.00 | 0.00 | 0.00 | 0.23 | 0.10 |
|  | aTCR | 0.02 | 0.01 | 0.01 | 0.06 | 0.02 | 0.08 | **0.51** |
|  | Pici. | 0.06 | 0.28 | 0.08 | 0.00 | 0.00 | **0.41** | 0.02 |

Table 5 Continued: Fold Change of mRNAs by Stimulant Used - TNFSF1, 2, and 5 v. TNFSF1, 2, and 5

|  |  | TNFSF1 |  |  |  | TNFSF2 |  |  |  |  | TNFSF5 |  |  |
|---|---|---|---|---|---|---|---|---|---|---|---|---|---|
|  |  | PHA | Zymo. | rIL2 | aTCR | PHA | HAG | Zymo. | rIL2 | aTCR | PHA | rIL2 | aTCR |
| TNFSF1 | PHA |  | 0.24 | **0.42** | 0.04 | 0.03 | 0.03 | 0.00 | 0.01 | 0.04 | **0.32** | 0.00 | 0.01 |
|  | Zymo. |  |  | **0.38** | 0.15 | 0.10 | 0.10 | **0.33** | 0.08 | 0.01 | 0.16 | 0.01 | 0.00 |
|  | rIL2 |  |  |  | 0.02 | 0.07 | 0.05 | 0.06 | 0.16 | 0.00 | 0.07 | 0.00 | 0.01 |
|  | aTCR |  |  |  |  | 0.02 | 0.03 | 0.06 | 0.00 | 0.12 | 0.04 | 0.17 | 0.18 |
| TNFSF2 | PHA |  |  |  |  |  | **0.40** | **0.52** | 0.15 | **0.38** | 0.05 | 0.01 | 0.04 |
|  | HAG |  |  |  |  |  |  | **0.33** | 0.07 | 0.05 | 0.01 | 0.07 | 0.08 |

(continued)

|  |  | TNFSF1 |  |  | TNFSF2 |  |  |  |  | TNFSF5 |  |  |
|---|---|---|---|---|---|---|---|---|---|---|---|---|
|  | Zymo. |  |  |  |  |  | 0.23 | 0.22 | 0.08 | 0.00 | 0.00 |
|  | rIL2 |  |  |  |  |  |  | 0.10 | 0.00 | 0.03 | 0.03 |
|  | aTCR |  |  |  |  |  |  |  | 0.00 | 0.00 | 0.01 |
| TNFSF5 | PHA |  |  |  |  |  |  |  |  | 0.17 | 0.18 |
|  | rIL2 |  |  |  |  |  |  |  |  |  | **0.83** |
|  | aTCR |  |  |  |  |  |  |  |  |  |  |

Table 5 Continued: Fold Change of mRNAs by Stimulant Used - TNFSF1, 2, and 5 v. IL10, TGFB and CTLA4

|  |  | IL10 |  |  |  |  |  | TGFB | CTLA4 |  |  |  |
|---|---|---|---|---|---|---|---|---|---|---|---|---|
|  |  | PHA | HAG | rIFN | Zymo. | rIL2 | aTCR | PHA | PHA | Zymo. | rIL2 | aTCR |
| TNFSF1 | PHA | 0.20 | 0.00 | 0.03 | 0.19 | 0.12 | 0.11 | 0.07 | **0.57** | 0.14 | 0.14 | 0.00 |
|  | Zymo. | **0.36** | 0.13 | 0.00 | 0.49 | 0.08 | 0.16 | 0.16 | **0.40** | 0.24 | 0.11 | 0.20 |
|  | rIL2 | 0.22 | 0.05 | 0.00 | 0.20 | 0.03 | 0.02 | 0.15 | 0.22 | 0.13 | 0.07 | 0.01 |
|  | aTCR | 0.00 | 0.01 | 0.00 | 0.03 | 0.06 | 0.19 | 0.02 | 0.04 | 0.01 | 0.00 | **0.40** |
| TNFSF2 | PHA | 0.00 | 0.05 | 0.00 | 0.00 | 0.01 | 0.02 | 0.01 | 0.20 | 0.02 | 0.06 | 0.05 |
|  | HAG | 0.02 | 0.01 | 0.01 | 0.07 | 0.04 | 0.02 | 0.00 | 0.19 | 0.01 | 0.00 | 0.04 |
|  | Zymo. | 0.06 | 0.07 | 0.01 | 0.11 | 0.00 | 0.00 | 0.00 | 0.25 | 0.04 | 0.00 | 0.15 |
|  | rIL2 | 0.00 | 0.00 | 0.00 | 0.02 | 0.04 | 0.00 | 0.01 | 0.01 | 0.16 | 0.16 | 0.03 |
|  | aTCR | 0.03 | 0.07 | 0.03 | 0.02 | 0.00 | 0.08 | 0.08 | 0.00 | 0.20 | 0.27 | 0.07 |
| TNFSF5 | PHA | 0.27 | 0.00 | 0.00 | 0.13 | 0.00 | 0.02 | 0.01 | **0.34** | 0.04 | 0.03 | 0.00 |
|  | rIL2 | 0.00 | 0.01 | 0.02 | 0.00 | 0.02 | 0.08 | 0.03 | 0.03 | 0.00 | 0.02 | 0.02 |
|  | aTCR | 0.00 | 0.00 | 0.00 | 0.00 | 0.01 | 0.05 | 0.02 | 0.03 | 0.04 | 0.09 | 0.00 |

Table 5 Continued: Fold Change of mRNAs by Stimulant Used - TNFSF1, 2, and 5 v. PD1 and FoxP3

|  |  | PD1 |  |  |  | FOXP3 |  |  |  |
|---|---|---|---|---|---|---|---|---|---|
|  |  | PHA | Zymo. | rIL2 | aTCR | PHA | Zymo. | rIL2 | aTCR |
| TNFSF1 | PHA | 0.25 | 0.00 | 0.00 | 0.08 | 0.50 | **0.32** | 0.29 | 0.08 |
|  | Zymo. | 0.25 | 0.26 | 0.22 | 0.03 | 0.10 | **0.41** | **0.40** | 0.15 |
|  | rIL2 | 0.25 | 0.07 | 0.04 | 0.00 | 0.09 | 0.12 | 0.11 | 0.02 |
|  | aTCR | 0.05 | 0.01 | 0.02 | 0.00 | 0.03 | 0.03 | 0.13 | 0.13 |
| TNFSF2 | PHA | 0.09 | 0.06 | 0.00 | 0.16 | 0.02 | 0.02 | 0.11 | 0.09 |
|  | HAG | 0.04 | 0.00 | 0.01 | 0.05 | 0.03 | 0.01 | 0.05 | 0.00 |
|  | Zymo. | 0.01 | 0.18 | 0.10 | 0.21 | 0.01 | 0.03 | 0.23 | 0.16 |
|  | rIL2 | 0.00 | 0.18 | 0.09 | 0.02 | 0.01 | 0.02 | 0.03 | 0.03 |
|  | aTCR | 0.00 | 0.03 | 0.03 | 0.22 | 0.02 | 0.01 | 0.05 | 0.11 |
| TNFSF5 | PHA | 0.23 | 0.01 | 0.00 | 0.00 | **0.39** | 0.22 | **0.43** | 0.17 |
|  | rIL2 | 0.02 | 0.03 | 0.03 | 0.00 | 0.00 | 0.00 | 0.01 | 0.00 |

(continued)

|  |  | PD1 |  |  |  | FOXP3 |  |  |  |
|---|---|---|---|---|---|---|---|---|---|
|  |  | PHA | Zymo. | rIL2 | aTCR | PHA | Zymo. | rIL2 | aTCR |
|  | aTCR | 0.00 | 0.03 | 0.04 | 0.01 | 0.00 | 0.00 | 0.02 | 0.00 |

Table 5 Continued: Fold Change of mRNAs by Stimulant Used - TNFSF1, 2, and 5 v. GMCSF, VEGF, and IL8

|  |  | GMCSF |  |  |  |  | VEGF | IL8 |  |  |  |
|---|---|---|---|---|---|---|---|---|---|---|---|
|  |  | PHA | HAG | Zymo. | rIL2 | aTCR | Zymo. | PHA | HAG | Zymo. | aTCR |
| TNFSF1 | PHA | 0.21 | 0.04 | 0.27 | 0.05 | 0.04 | 0.10 | 0.10 | 0.00 | 0.03 | 0.01 |
|  | Zymo. | 0.21 | 0.00 | **0.57** | 0.11 | 0.00 | 0.26 | 0.00 | 0.02 | 0.04 | 0.00 |
|  | rIL2 | 0.11 | 0.06 | 0.20 | 0.06 | 0.08 | 0.08 | 0.06 | 0.06 | 0.00 | 0.00 |
|  | aTCR | 0.01 | 0.01 | 0.03 | 0.01 | 0.10 | 0.02 | 0.07 | 0.10 | 0.06 | 0.27 |
| TNFSF2 | PHA | 0.26 | 0.14 | 0.12 | 0.13 | 0.09 | 0.03 | 0.06 | 0.09 | 0.00 | 0.02 |
|  | HAG | 0.08 | 0.16 | 0.25 | 0.17 | 0.00 | 0.08 | 0.00 | 0.04 | 0.02 | 0.00 |
|  | Zymo. | 0.18 | 0.08 | **0.43** | 0.06 | 0.02 | 0.22 | 0.00 | 0.00 | 0.12 | 0.07 |
|  | rIL2 | 0.02 | 0.02 | 0.10 | 0.06 | 0.01 | 0.00 | 0.01 | 0.08 | 0.01 | 0.12 |
|  | aTCR | 0.08 | 0.06 | 0.00 | 0.00 | **0.55** | 0.00 | 0.00 | 0.04 | 0.00 | 0.17 |
| TNFSF5 | PHA | 0.68 | 0.07 | **0.41** | 0.17 | 0.01 | 0.08 | 0.04 | 0.00 | 0.02 | 0.03 |
|  | rIL2 | 0.16 | 0.06 | 0.04 | 0.10 | 0.01 | 0.03 | 0.00 | 0.06 | 0.00 | 0.00 |
|  | aTCR | 0.15 | 0.14 | 0.08 | 0.09 | 0.00 | 0.01 | 0.01 | 0.01 | 0.01 | 0.00 |

Table 5 Continued: Fold Change of mRNAs by Stimulant Used - TNFSF1, 2, and 5 v. CCL8

|  |  | CCL8 |  |  |  |  |  |
|---|---|---|---|---|---|---|---|
|  |  | PHA | HAG | rIFN | Zymo. | rIL2 | aTCR |
| TNFSF1 | PHA | 0.01 | 0.00 | 0 | 0.00 | 0.00 | 0.00 |
|  | Zymo. | 0.00 | 0.02 | 0 | 0.01 | 0.01 | 0.00 |
|  | rIL2 | 0.06 | 0.03 | 0 | 0.08 | 0.06 | 0.09 |
|  | aTCR | 0.06 | 0.10 | 0 | 0.01 | 0.08 | 0.01 |
| TNFSF2 | PHA | 0.01 | 0.00 | 0 | 0.00 | 0.00 | 0.00 |
|  | HAG | 0.00 | 0.00 | 0 | 0.00 | 0.00 | 0.00 |
|  | Zymo. | 0.00 | 0.00 | 0 | 0.01 | 0.00 | 0.01 |
|  | rIL2 | 0.09 | 0.05 | 0 | 0.01 | 0.06 | 0.02 |
|  | aTCR | 0.06 | 0.06 | 0 | 0.00 | 0.06 | 0.03 |
| TNFSF5 | PHA | 0.09 | 0.03 | 0 | 0.04 | 0.06 | 0.03 |
|  | rIL2 | 0.00 | 0.01 | 0 | 0.01 | 0.01 | 0.00 |
|  | aTCR | 0.00 | 0.01 | 0 | 0.04 | 0.00 | 0.01 |

Table 5 Continued: Fold Change of mRNAs by Stimulant Used - TNFSF1, 2, and 5 v. CXCL3 and IL2

| | | CXCL3 | | | | | IL2 | |
|---|---|---|---|---|---|---|---|---|
| | | PHA | HAG | Zymo. | rIL2 | aTCR | PHA | aTCR |
| TNFSF1 | PHA | 0.01 | 0.04 | 0.00 | 0.02 | 0.00 | 0.11 | 0.07 |
| | Zymo. | 0.09 | 0.28 | 0.08 | 0.07 | 0.03 | 0.02 | 0.08 |
| | rIL2 | 0.00 | 0.00 | 0.01 | 0.01 | 0.00 | 0.03 | 0.03 |
| | aTCR | 0.23 | 0.19 | 0.22 | 0.27 | 0.28 | 0.10 | 0.00 |
| TNFSF2 | PHA | 0.05 | 0.00 | 0.14 | 0.06 | 0.07 | 0.01 | 0.08 |
| | HAG | 0.07 | 0.11 | 0.18 | 0.10 | 0.10 | 0.01 | 0.01 |
| | Zymo. | 0.16 | 0.16 | 0.39 | 0.16 | 0.17 | 0.03 | 0.01 |
| | rIL2 | 0.00 | 0.00 | 0.00 | 0.12 | 0.03 | 0.10 | 0.12 |
| | aTCR | 0.15 | 0.02 | 0.12 | 0.00 | 0.02 | 0.05 | 0.27 |
| TNFSF5 | PHA | 0.00 | 0.07 | 0.01 | 0.04 | 0.05 | 0.15 | 0.00 |
| | rIL2 | 0.05 | 0.06 | 0.10 | **0.36** | 0.39 | 0.01 | 0.00 |
| | aTCR | 0.01 | 0.01 | 0.06 | **0.30** | 0.31 | 0.00 | 0.06 |

Table 5 Continued: Fold Change of mRNAs by Stimulant Used - IL10, TGFB, and CTLA4 v. IL10, TGFB, and CTLA4

| | | IL10 | | | | | | TGFB | CTLA4 | | | |
|---|---|---|---|---|---|---|---|---|---|---|---|---|
| | | PHA | HAG | rIFN | Zymo. | rIL2 | aTCR | PHA | PHA | Zymo. | rIL2 | aTCR |
| IL10 | PHA | | 0.22 | 0.00 | 0.71 | 0.16 | 0.08 | 0.12 | 0.26 | 0.05 | 0.02 | 0.00 |
| | HAG | | | 0.14 | 0.17 | 0.29 | 0.09 | 0.01 | 0.02 | 0.01 | 0.04 | 0.01 |
| | rIFN | | | | 0.01 | 0.26 | 0.14 | 0.00 | 0.02 | 0.01 | 0.01 | 0.02 |
| | Zymo. | | | | | 0.26 | 0.22 | 0.19 | 0.25 | 0.11 | 0.09 | 0.02 |
| | rIL2 | | | | | | **0.63** | 0.09 | 0.09 | 0.06 | 0.09 | 0.01 |
| | aTCR | | | | | | | 0.11 | 0.09 | 0.24 | 0.24 | 0.05 |
| TGFB | PHA | | | | | | | | 0.03 | 0.12 | 0.05 | 0.01 |
| CTLA4 | PHA | | | | | | | | | 0.19 | 0.11 | 0.05 |
| | Zymo. | | | | | | | | | | **0.87** | 0.12 |
| | rIL2 | | | | | | | | | | | 0.00 |
| | aTCR | | | | | | | | | | | |

Table 5 Continued: Fold Change of mRNAs by Stimulant Used - IL10, TGFB, and CTLA4

| v. PD1 and FoxP3 | | | | | | | | | |
|---|---|---|---|---|---|---|---|---|---|
| | | PD1 | | | | FOXP3 | | | |
| | | PHA | Zymo. | rIL2 | aTCR | PHA | Zymo. | rIL2 | aTCR |
| IL10 | PHA | 0.24 | 0.04 | 0.00 | 0.05 | 0.36 | 0.21 | **0.38** | 0.07 |
| | HAG | 0.04 | 0.10 | 0.08 | 0.06 | 0.00 | 0.00 | 0.03 | 0.03 |
| | rIFN | 0.01 | 0.04 | 0.09 | 0.00 | 0.01 | 0.01 | 0.01 | 0.00 |
| | Zymo. | 0.14 | 0.02 | 0.00 | 0.08 | 0.28 | **0.37** | **0.43** | 0.12 |

(continued)

| v. PD1 and FoxP3 | | | | | | | | | |
|---|---|---|---|---|---|---|---|---|---|
| | | PD1 | | | | FOXP3 | | | |
| | | PHA | Zymo. | rIL2 | aTCR | PHA | Zymo. | rIL2 | aTCR |
| | rIL2 | 0.00 | 0.00 | 0.00 | 0.05 | 0.12 | 0.03 | 0.07 | 0.04 |
| | aTCR | 0.00 | 0.00 | 0.01 | 0.08 | 0.07 | 0.08 | 0.03 | 0.00 |
| TGFB | PHA | 0.00 | 0.02 | 0.03 | 0.00 | 0.07 | 0.16 | 0.02 | 0.07 |
| CTLA4 | PHA | 0.26 | 0.04 | 0.01 | 0.00 | **0.39** | **0.30** | **0.43** | 0.18 |
| | Zymo. | 0.02 | 0.16 | 0.12 | 0.00 | 0.04 | 0.21 | 0.06 | 0.04 |
| | rIL2 | 0.03 | 0.03 | 0.06 | 0.03 | 0.12 | 0.05 | 0.01 | 0.00 |
| | aTCR | 0.00 | 0.05 | 0.08 | 0.11 | 0.01 | 0.17 | 0.19 | 0.31 |

Table 5 Continued: Fold Change of mRNAs by Stimulant Used - IL10, TGFB, and CTLA4 v. GMCSF, VEGF, and IL8

| | | GMCSF | | | | | VEG F | IL8 | | | |
|---|---|---|---|---|---|---|---|---|---|---|---|
| | | PH A | HA G | Zymo | rIL2 | aTC R | Zymo | PH A | HA G | Zymo | aTC R |
| IL10 | PHA | 0.23 | 0.00 | **0.30** | 0.14 | 0.00 | 0.27 | 0.00 | 0.06 | 0.07 | 0.08 |
| | HAG | 0.01 | 0.00 | 0.02 | 0.00 | 0.03 | 0.21 | 0.11 | 0.01 | 0.06 | 0.01 |
| | rIFN | 0.02 | 0.01 | 0.00 | 0.02 | 0.05 | 0.00 | 0.00 | 0.06 | 0.04 | 0.00 |
| | Zymo | 0.21 | 0.00 | **0.43** | 0.10 | 0.01 | **0.33** | 0.01 | 0.16 | 0.12 | 0.00 |
| | rIL2 | 0.01 | 0.00 | 0.07 | 0.06 | 0.00 | 0.10 | 0.05 | 0.01 | 0.00 | 0.00 |
| | aTCR | 0.02 | 0.01 | 0.06 | 0.02 | 0.01 | 0.09 | 0.08 | 0.11 | 0.00 | 0.00 |
| TGFB | PHA | 0.00 | 0.03 | 0.04 | 0.00 | 0.02 | 0.13 | 0.02 | 0.01 | 0.01 | 0.01 |
| CTLA 4 | PHA | 0.25 | 0.00 | **0.50** | 0.11 | 0.03 | 0.31 | 0.08 | 0.00 | 0.00 | 0.01 |
| | Zymo. | 0.00 | 0.00 | 0.27 | 0.07 | 0.27 | 0.09 | 0.03 | 0.03 | 0.03 | 0.01 |
| | rIL2 | 0.01 | | 0.07 | **0.30** | 0.02 | 0.03 | 0.03 | 0.02 | 0.02 | 0.00 |
| | aTCR | 0.02 | 0.04 | 0.12 | 0.04 | 0.02 | 0.04 | 0.09 | 0.03 | 0.04 | 0.19 |

Table 5 Continued: Fold Change of mRNAs by Stimulant Used - IL10, TGFB, and CTLA4 v. CCL8, CXCL3, and IL2

| | | CCL8 | | | | | | CXCL3 | | | | | IL2 | |
|---|---|---|---|---|---|---|---|---|---|---|---|---|---|---|
| | | PHA | HAG | rIFN | Zymo. | rIL2 | aTCR | PHA | HAG | Zymo. | rIL2 | aTCR | PHA | aTCR |
| IL10 | PHA | 0.18 | 0.15 | 0 | 0.01 | 0.17 | 0.00 | 0.12 | **0.37** | 0.06 | 0.00 | 0.00 | 0.16 | 0.03 |
| | HAG | 0.01 | 0.04 | 0 | 0.04 | 0.02 | 0.03 | **0.38** | 0.17 | 0.13 | 0.00 | 0.00 | 0.01 | 0.01 |
| | rIFN | 0.00 | 0.00 | 0 | 0.06 | 0.00 | 0.08 | 0.00 | 0.04 | 0.02 | 0.01 | 0.03 | 0.01 | 0.03 |
| | Zymo. | 0.05 | 0.06 | 0 | 0.00 | 0.06 | 0.00 | 0.19 | **0.50** | 0.14 | 0.09 | 0.04 | 0.10 | 0.07 |
| | rIL2 | 0.00 | 0.01 | 0 | 0.00 | 0.01 | 0.00 | 0.27 | 0.15 | 0.05 | 0.07 | 0.02 | 0.03 | 0.12 |
| | aTCR | 0.00 | 0.00 | 0 | 0.03 | 0.00 | 0.00 | 0.14 | 0.20 | 0.05 | 0.12 | 0.08 | 0.01 | 0.29 |
| TGFB | PHA | 0.14 | 0.06 | 0 | 0.07 | 0.10 | 0.12 | 0.04 | 0.06 | 0.01 | 0.11 | 0.05 | 0.02 | 0.04 |
| CTLA4 | PHA | 0.05 | 0.04 | 0 | 0.00 | 0.04 | 0.01 | 0.00 | 0.13 | 0.10 | 0.11 | 0.08 | 0.06 | 0.09 |
| | Zymo. | 0.02 | 0.03 | 0 | 0.00 | 0.02 | 0.03 | 0.01 | 0.01 | 0.00 | 0.07 | 0.01 | 0.03 | 0.84 |
| | rIL2 | 0.01 | 0 | 0.02 | 0.00 | 0.01 | 0.02 | 0.01 | 0.01 | 0.06 | 0.00 | 0.03 | **0.88** | 0 |
| | aTCR | 0.13 | 0.24 | 0 | 0.09 | 0.18 | 0.06 | 0.07 | 0.05 | 0.09 | 0.10 | 0.10 | 0.15 | 0.04 |

Table 5 Continued: Fold Change of mRNAs by Stimulant Used - PD1, FoxP3, and GMCSF

| v. PD1 and FoxP3 | | PD1 | | | | FOXP3 | | | |
|---|---|---|---|---|---|---|---|---|---|
| | | PHA | Zymo. | rIL2 | aTCR | PHA | Zymo. | rIL2 | aTCR |
| PD1 | PHA | | 0.22 | 0.06 | 0.01 | 0.13 | **0.32** | 0.16 | 0.01 |
| | Zymo. | | | **0.73** | 0.25 | 0.01 | 0.11 | 0.03 | 0.04 |
| | rIL2 | | | | **0.33** | 0.04 | 0.01 | 0.02 | 0.04 |
| | aTCR | | | | | 0.04 | 0.00 | 0.00 | 0.08 |
| FOXP3 | PHA | | | | | | **0.43** | **0.54** | **0.36** |
| | Zymo. | | | | | | | **0.44** | **0.31** |
| | rIL2 | | | | | | | | **0.62** |
| | aTCR | | | | | | | | |
| GMCSF | PHA | | | | | | | | |
| | HAG | | | | | | | | |
| | rIFN | | | | | | | | |
| | Zymo. | | | | | | | | |
| | rIL2 | | | | | | | | |
| | aTCR | | | | | | | | |

Table 5 Continued: Fold Change of mRNAs by Stimulant Used - PD1, FoxP3, and GMCSF v. GMCSF, VEGF, and IL8

| | | GMCSF | | | | | VEGF | IL8 | | | |
|---|---|---|---|---|---|---|---|---|---|---|---|
| | | PHA | HAG | Zymo. | rIL2 | aTCR | Zymo. | PHA | HAG | Zymo. | aTCR |
| PD1 | PHA | 0.27 | 0.00 | 0.14 | 0.24 | 0.01 | 0.00 | 0.15 | 0.04 | 0.03 | 0.15 |
| | Zymo. | 0.02 | 0.01 | 0.07 | 0.07 | 0.01 | 0.09 | 0.01 | 0.03 | 0.02 | 0.00 |
| | rIL2 | 0.00 | 0.00 | 0.04 | 0.03 | 0.00 | 0.05 | 0.03 | 0.04 | 0.02 | 0.01 |
| | aTCR | 0.02 | 0.04 | 0.02 | 0.03 | 0.05 | 0.00 | 0.00 | 0.07 | 0.00 | 0.00 |
| FOXP3 | PHA | 0.28 | 0.00 | 0.22 | 0.15 | 0.00 | 0.08 | 0.02 | 0.04 | 0.00 | 0.01 |
| | Zymo. | 0.22 | 0.00 | 0.26 | 0.13 | 0.01 | 0.05 | 0.00 | 0.04 | 0.00 | 0.00 |
| | rIL2 | **0.54** | 0.06 | **0.52** | 0.26 | 0.04 | 0.14 | 0.00 | 0.03 | 0.08 | 0.01 |
| | aTCR | 0.27 | 0.04 | 0.19 | 0.07 | 0.06 | 0.12 | 0.03 | 0.00 | 0.06 | 0.11 |
| GMCSF | PHA | | 0.21 | **0.48** | **0.37** | 0.08 | 0.06 | 0.04 | 0.01 | 0.01 | 0.01 |
| | HAG | | | 0.06 | **0.36** | 0.14 | 0.01 | 0.07 | 0.01 | 0.09 | 0.01 |
| | rIFN | | | | 0.29 | 0.00 | 0.25 | 0.01 | 0.02 | 0.06 | 0.00 |
| | Zymo. | | | | 0.29 | 0.00 | 0.25 | 0.01 | 0.02 | 0.06 | 0.00 |
| | rIL2 | | | | | 0.00 | 0.01 | 0.02 | 0.04 | 0.00 | 0.09 |
| | aTCR | | | | | | 0.03 | 0.01 | 0.00 | 0.00 | 0.01 |

Table 5 Continued: Fold Change of mRNAs by Stimulant Used - PD1, FoxP3, and GMCSF v. CCL8

| | | CCL8 | | | | | |
|---|---|---|---|---|---|---|---|
| | | PHA | HAG | rIFN | Zymo. | rIL2 | aTCR |
| PD1 | PHA | 0.01 | 0.01 | 0 | 0.00 | 0.01 | 0.00 |
| | Zymo. | 0.07 | 0.05 | 0 | 0.02 | 0.06 | 0.03 |
| | rIL2 | 0.15 | 0.15 | 0 | 0.01 | 0.16 | 0.02 |
| | aTCR | 0.19 | 0.19 | 0 | 0.01 | 0.20 | 0.00 |
| FOXP3 | PHA | 0.04 | 0.01 | 0 | 0.03 | 0.02 | 0.02 |
| | Zymo. | 0.01 | 0.03 | 0 | 0.03 | 0.02 | 0.02 |
| | rIL2 | 0.02 | 0.00 | 0 | 0.08 | 0.01 | 0.06 |
| | aTCR | 0.07 | 0.08 | 0 | 0.01 | 0.07 | 0.00 |
| GMCSF | PHA | 0.01 | 0.00 | 0 | 0.07 | 0.00 | 0.08 |
| | HAG | 0.00 | 0.00 | 0 | 0.15 | 0.00 | 0.12 |
| | rIFN | 0.01 | 0.00 | 0 | 0.02 | 0.00 | 0.00 |
| | Zymo. | 0.01 | 0.00 | 0 | 0.02 | 0.00 | 0.00 |
| | rIL2 | 0.00 | 0.02 | 0 | 0.17 | 0.00 | 0.14 |
| | aTCR | 0.00 | 0.01 | 0 | 0.15 | 0.00 | 0.25 |

Table 5 Continued: Fold Change of mRNAs by Stimulant Used - PD1, FoxP3, and GMCSF v. CXCL3 and IL2

| | | CXCL3 | | | | | IL2 | |
|---|---|---|---|---|---|---|---|---|
| | | WPHA | HAG | Zymo. | rIL2 | aTCR | PHA | aTCR |
| PD1 | PHA | 0.06 | 0.00 | 0.05 | 0.11 | 0.16 | 0.32 | 0.01 |
| | Zymo. | 0.00 | 0.00 | 0.00 | 0.00 | 0.01 | 0.02 | 0.06 |
| | rIL2 | 0.00 | 0.00 | 0.00 | 0.01 | 0.02 | 0.13 | 0.05 |
| | aTCR | 0.00 | 0.05 | 0.00 | 0.03 | 0.01 | 0.13 | 0.05 |
| FOXP3 | PHA | 0.01 | 0.10 | 0.03 | 0.03 | 0.02 | 0.09 | 0.01 |
| | Zymo. | 0.00 | 0.07 | 0.00 | 0.04 | 0.02 | 0.11 | 0.04 |
| | rIL2 | 0.10 | 0.25 | 0.18 | 0.07 | 0.04 | 0.01 | 0.01 |
| | aTCR | 0.10 | 0.05 | 0.15 | 0.11 | 0.11 | 0.05 | 0.00 |
| GMCSF | PHA | 0.02 | 0.06 | 0.02 | 0.03 | 0.03 | 0.06 | 0.02 |
| | HAG | 0.07 | 0.01 | 0.08 | 0.02 | 0.00 | 0.10 | 0.00 |
| | rIFN | 0.06 | 0.23 | 0.08 | 0.02 | 0.01 | 0.00 | 0.18 |
| | Zymo. | 0.06 | 0.23 | 0.08 | 0.02 | 0.01 | 0.00 | 0.18 |
| | rIL2 | 0.00 | 0.00 | 0.01 | 0.06 | 0.11 | 0.01 | 0.02 |
| | aTCR | 0.25 | 0.08 | 0.08 | 0.02 | 0.00 | 0.01 | 0.30 |

Table 5 Continued: Fold Change of mRNAs by Stimulant Used - VEGF, IL8, CCL8, CXCL3 and IL2 v. VEGF, IL8, and CCL8

| | | VEGF | IL8 | | | | CCL8 | | | | | |
|---|---|---|---|---|---|---|---|---|---|---|---|---|
| | | Zymo. | PHA | HAG | Zymo. | aTCR | PHA | HAG | rIFN | Zymo. | rIL2 | aTCR |
| VEGF | Zymo. | | 0.12 | 0.13 | **0.42** | 0.05 | 0.02 | 0.06 | 0 | 0.06 | 0.04 | 0.11 |
| IL8 | PHA | | | **0.39** | **0.54** | **0.32** | 0.03 | 0.01 | 0 | 0.00 | 0.01 | 0.02 |
| | HAG | | | | **0.50** | 0.10 | 0.09 | 0.08 | 0 | 0.01 | 0.08 | 0.00 |
| | Zymo. | | | | | 0.25 | 0.02 | 0.07 | 0 | 0.02 | 0.05 | 0.04 |
| | aTCR | | | | | | 0.09 | 0.04 | 0 | 0.02 | 0.06 | 0.03 |
| CCL8 | PHA | | | | | | | **0.81** | 0 | 0.12 | **0.94** | 0.11 |
| | HAG | | | | | | | | 0 | 0.00 | **0.95** | 0.00 |
| | rIFN | | | | | | | | | 0 | 0 | 0 |
| | Zymo. | | | | | | | | | | 0.03 | **0.94** |
| | rIL2 | | | | | | | | | | | 0.02 |
| | aTCR | | | | | | | | | | | |
| CXCL3 | PHA | | | | | | | | | | | |
| | HAG | | | | | | | | | | | |
| | Zymo. | | | | | | | | | | | |
| | rIL2 | | | | | | | | | | | |
| | aTCR | | | | | | | | | | | |
| IL2 | PHA | | | | | | | | | | | |
| | **aTCR** | | | | | | | | | | | |

Table 5 Continued: Fold Change of mRNAs by Stimulant Used - VEGF, IL8, CCL8, CXCL3 and IL2 v. CXCL3 and IL2

| | | CXCL3 | | | | | IL2 | |
|---|---|---|---|---|---|---|---|---|
| | | PHA | HAG | Zymo. | rIL2 | aTCR | PHA | aTCR |
| VEGF | Zymo. | 0.22 | 0.30 | 0.38 | 0.15 | 0.18 | 0.00 | 0.09 |
| IL8 | PHA | **0.37** | 0.09 | 0.17 | 0.01 | 0.02 | 0.13 | 0.06 |
| | HAG | 0.25 | 0.51 | 0.31 | 0.09 | 0.14 | 0.04 | 0.05 |
| | Zymo. | **0.37** | 0.28 | 0.50 | 0.07 | 0.10 | 0.01 | 0.03 |
| | aTCR | 0.14 | 0.04 | 0.23 | 0.16 | 0.20 | 0.08 | 0.02 |
| CCL8 | PHA | 0.01 | 0.16 | 0.03 | 0.00 | 0.00 | 0.25 | 0.00 |
| | HAG | 0.02 | 0.12 | 0.07 | 0.01 | 0.01 | 0.22 | 0.00 |
| | rIFN | 0 | 0 | 0 | 0 | 0 | 0 | 0 |
| | Zymo. | 0.00 | 0.04 | 0.00 | 0.00 | 0.00 | 0.00 | 0.00 |
| | rIL2 | 0.03 | 0.15 | 0.06 | 0.01 | 0.01 | 0.21 | 0.00 |
| | aTCR | 0.00 | 0.02 | 0.00 | 0.03 | 0.02 | 0.00 | 0.03 |
| CXCL3 | PHA | | **0.52** | **0.56** | 0.11 | 0.13 | 0.06 | 0.00 |
| | HAG | | | **0.52** | 0.17 | 0.19 | 0.04 | 0.01 |
| | Zymo. | | | | **0.39** | **0.51** | 0.02 | 0.00 |

(continued)

|  |  | CXCL3 |  |  |  |  | IL2 |  |
|---|---|---|---|---|---|---|---|---|
|  |  | PHA | HAG | Zymo. | rIL2 | aTCR | PHA | aTCR |
|  | rIL2 |  |  |  |  | **0.88** | 0.07 | 0.08 |
|  | aTCR |  |  |  |  |  | 0.05 | 0.02 |
| IL2 | PHA |  |  |  |  |  |  | 0.06 |
|  | **aTCR** |  |  |  |  |  |  |  |

Table 6. Summary of multivariate discrinant analysis with 1 or 2 combinations of stimulants.

| 2nd stimulant | FALSE (%)* | rIL2 PD | rIL2 SD | rIL2 PR | aTCR PD | aTCR SD | aTCR PR | PHA PD | PHA SD | PHA PR | Zymosan PD | Zymosan SD | Zymosan PR | HAG PD | HAG SD | HAG PR | rIFN PD | rIFN SD | rIFN PR | Picibanil PD | Picibanil SD | Picibanil PR |
|---|---|---|---|---|---|---|---|---|---|---|---|---|---|---|---|---|---|---|---|---|---|---|
| (-) | Positive | 0 | 10 | 0 | 0 | 18 | 0 | 9 | 0 | 50 | 15 | 22 | 0 | 25 | 43 | 57 | 50 | 50 | 50 | 40 | 57 | 57 |
|  | Negative | 8 | 0 | 0 | 15 | 0 | 0 | 23 | 22 | 0 | 15 | 22 | 0 | 31 | 56 | 25 | 38 | 67 | 50 | 77 | 33 | 25 |
| Picibanil | Positive | 0 | 11 | 20 | 0 | 10 | 0 | 0 | 10 | 20 | 15 | 22 | 0 | 29 | 50 | 50 | 38 | 63 | 40 |  |  |  |
|  | Negative | 8 | 11 | 0 | 8 | 0 | 0 | 15 | 0 | 0 | 15 | 22 | 0 | 23 | 67 | 25 | 38 | 67 | 25 |  |  |  |
| rIFN | Positive |  |  |  | 0 | 10 | 0 |  |  |  | 8 | 20 | 0 | 9 | 20 | 20 |  |  |  |  |  |  |
|  | Negative |  |  |  | 8 | 0 | 0 |  |  |  | 15 | 11 | 0 | 23 | 11 | 0 |  |  |  |  |  |  |
| HAG | Positive |  |  |  | 0 | 10 | 0 |  |  |  | 8 | 20 | 0 |  |  |  |  |  |  |  |  |  |
|  | Negative |  |  |  | 8 | 0 | 0 |  |  |  | 15 | 11 | 0 |  |  |  |  |  |  |  |  |  |
| Zymosan | Positive |  |  |  |  |  |  |  |  |  |  |  |  |  |  |  |  |  |  |  |  |  |
|  | Negative |  |  |  |  |  |  |  |  |  |  |  |  |  |  |  |  |  |  |  |  |  |
| PHA | Positive |  |  |  |  |  |  |  |  |  |  |  |  |  |  |  |  |  |  |  |  |  |
|  | Negative |  |  |  |  |  |  |  |  |  |  |  |  |  |  |  |  |  |  |  |  |  |
| aTCR | Positive |  |  |  |  |  |  |  |  |  |  |  |  |  |  |  |  |  |  |  |  |  |
|  | Negative |  |  |  |  |  |  |  |  |  |  |  |  |  |  |  |  |  |  |  |  |  |

*: False positive and negative for each of PR, SD, and PR were determined by the table below.

Table 6A

| Prediction | Clinical outcome PD | Clinical outcome SD | Clinical outcome PR | False positive |
|---|---|---|---|---|
| PD | a | b | c | (b+c)/(a+b+c) |
| SD | d | e | f | (d+f)/(d+e+f) |
| PR | g | h | i | (g+h)/(g+h+i) |
| False negative | (d+g)f(a+d+g) | (b+h)/(b+e+h) | (c+f)/(c+f+i) |  |

Table 7 - Predictive Responsiveness and Gene Combinations

(Box indicated the combinations of 2 mRNAs with false positive and negative values were all =<30%.)

| 2nd mRNA | FALSE (%)* | IFNG | | | IL2 | | | TNFSF1 | | | TNFSF2 | | |
|---|---|---|---|---|---|---|---|---|---|---|---|---|---|
| | | PD | SD | PR | PD | SD | PR | PD | SD | PR | PD | SD | PR |
| (-) | Positive | 38 | 63 | 40 | 0 | 59 | 75 | 14 | 44 | 70 | 31 | 50 | 43 |
| | Negative | 38 | 67 | 25 | 92 | 22 | 50 | 54 | 44 | 25 | 31 | 67 | 0 |
| GMCSF | Positive | 15 | 22 | 0 | 18 | 30 | 40 | 18 | 22 | 33 | 15 | 25 | 40 |
| | Negative | 15 | 22 | 0 | 31 | 22 | 25 | 31 | 22 | 0 | 15 | 33 | 25 |
| CCL8 | Positive | 31 | 33 | 0 | 38 | 59 | 0 | 18 | 46 | 50 | 18 | 33 | 33 |
| | Negative | 15 | 56 | 0 | 62 | 22 | 75 | 31 | 22 | 75 | 31 | 33 | 0 |
| CXCL3 | Positive | 8 | 20 | 0 | 14 | 36 | 50 | 22 | 27 | 50 | 27 | 50 | 40 |
| | Negative | 15 | 11 | 0 | 54 | 22 | 0 | 46 | 11 | 25 | 38 | 44 | 25 |
| IL8 | Positive | 21 | 25 | 0 | 30 | 44 | 43 | 25 | 30 | 50 | 21 | 29 | 40 |
| | Negative | 15 | 33 | 0 | 46 | 44 | 0 | 54 | 22 | 0 | 15 | 44 | 25 |
| PD-1 | Positive | 10 | 38 | 0 | 20 | 30 | 67 | 20 | 33 | 43 | 20 | 44 | 43 |
| | Negative | 31 | 11 | 25 | 38 | 22 | 50 | 38 | 33 | 0 | 38 | 44 | 0 |
| FOXP3 | Positive | 18 | 30 | 20 | 0 | 27 | 50 | 25 | 33 | 67 | 0 | 13 | 43 |
| | Negative | 31 | 22 | 0 | 46 | 11 | 0 | 54 | 11 | 50 | 15 | 22 | 0 |

EP 2 734 233 B1

54

Table 7 Continued- Predictive Responsiveness and Gene Combinations

| 2nd mRNA | FALSE (%)* | TNFSF5 | | | CTLA4 | | | TGFB | | |
|---|---|---|---|---|---|---|---|---|---|---|
| | | PD | SD | PR | PD | SD | PR | PD | SD | PR |
| (-) | Positive | 30 | 40 | # | 10 | 33 | # | 100 | 60 | # |
| | Negative | 46 | 33 | # | 31 | 33 | # | 100 | 33 | # |
| GMCSF | Positive | 0 | 33 | # | 0 | 11 | # | 15 | 25 | # |
| | Negative | 38 | 11 | 0 | 23 | 11 | 0 | 15 | 33 | # |
| CCL8 | Positive | 38 | 45 | # | 9 | 38 | 0 | 33 | 46 | 0 |
| | Negative | 38 | 33 | # | 23 | 11 | # | 38 | 22 | # |
| CXCL3 | Positive | 20 | 20 | # | 0 | 27 | # | 33 | 42 | # |
| | Negative | 38 | 11 | # | 31 | 11 | # | 69 | 22 | # |
| IL8 | Positive | 22 | 30 | # | 10 | 27 | # | 22 | 36 | # |
| | Negative | 46 | 22 | 0 | 31 | 11 | # | 46 | 22 | 0 |
| PD-1 | Positive | 20 | 29 | # | 11 | 25 | # | 29 | 44 | # |
| | Negative | 38 | 44 | # | 38 | 33 | # | 62 | 44 | # |
| FOXP3 | Positive | 14 | 42 | # | 15 | 13 | # | 14 | 33 | # |
| | Negative | 54 | 22 | # | 15 | 22 | # | 54 | 33 | 0 |

Table 7 Continued- Predictive Responsiveness and Gene Combinations

| 2nd mRNA | FALSE (%)* | IL10 | | | FOXP3 | | | PD-1 | | |
|---|---|---|---|---|---|---|---|---|---|---|
| | | PD | SD | PR | PD | SD | PR | PD | SD | PR |
| (-) | Positive | 10 | 45 | 40 | 13 | 30 | 50 | 50 | 57 | 73 |
| | Negative | 31 | 33 | 25 | 46 | 22 | 0 | 69 | 67 | 25 |
| GMCSF | Positive | 0 | 25 | 40 | 10 | 20 | 33 | 17 | 11 | 40 |
| | Negative | 31 | 0 | 25 | 31 | 11 | 0 | 23 | 11 | 25 |
| CCL8 | Positive | 0 | 31 | 0 | 29 | 22 | 33 | 41 | 38 | 0 |
| | Negative | 23 | 0 | 25 | 23 | 22 | 50 | 23 | 44 | 75 |
| CXCL3 | Positive | 10 | 33 | 43 | 29 | 30 | 56 | 25 | 36 | 57 |
| | Negative | 31 | 33 | 0 | 62 | 22 | 0 | 54 | 22 | 25 |
| IL8 | Positive | 10 | 40 | 33 | 0 | 25 | 33 | 30 | 25 | 50 |
| | Negative | 31 | 33 | 0 | 38 | 0 | 0 | 46 | 33 | 0 |
| PD-1 | Positive | 13 | 33 | 50 | 11 | 30 | 57 | | | |
| | Negative | 46 | 11 | 25 | 38 | 22 | 25 | | | |
| FOXP3 | Positive | 9 | 22 | 33 | | | | | | |
| | Negative | 23 | 22 | 0 | | | | | | |

Table 7 Continued- Predictive Responsiveness and Gene Combinations

| 2nd mRNA | FALSE (%)* | IL8 | | | CXCL3 | | | CCL8 | | | GMCSF | | |
|---|---|---|---|---|---|---|---|---|---|---|---|---|---|
| | | PD | SD | PR | PD | SD | PR | PD | SD | PR | PD | SD | PR |
| (-) | Positive | 25 | 14 | # | 43 | 50 | # | 46 | 0 | # | 17 | 30 | # |
| | Negative | 31 | 33 | 0 | 69 | 44 | # | 0 | 89 | # | 23 | 22 | # |
| GMCSF | Positive | 18 | 22 | # | 18 | 22 | # | 17 | 22 | # | | | |
| | Negative | 31 | 22 | 0 | 31 | 22 | 0 | 23 | 22 | 0 | | | |
| CCL8 | Positive | 21 | 14 | # | 31 | 33 | # | | | | | | |
| | Negative | 15 | 33 | 0 | 31 | 33 | # | | | | | | |
| CXCL3 | Positive | 27 | 30 | # | | | | | | | | | |
| | Negative | 38 | 22 | # | | | | | | | | | |
| IL8 | Positive | | | | | | | | | | | | |
| | Negative | | | | | | | | | | | | |
| PD-1 | Positive | | | | | | | | | | | | |
| | Negative | | | | | | | | | | | | |
| FOXP3 | Positive | | | | | | | | | | | | |
| | Negative | | | | | | | | | | | | |

Table 7 Continued- Predictive Responsiveness and Gene Combinations

| 2nd mRNA | FALSE (%)* | IFNG | | | IL2 | | |
|---|---|---|---|---|---|---|---|
| | | PD | SD | PR | PD | SD | PR |
| IL10 | Positive | 0 | 36 | 0 | 0 | 42 | 40 |
| | Negative | 31 | 0 | 25 | 31 | 22 | 25 |
| TGFB | Positive | 25 | 50 | 50 | 0 | 42 | 67 |
| | Negative | 54 | 22 | 50 | 62 | 22 | 25 |
| CTLA4 | Positive | 17 | 30 | 25 | 18 | 38 | 57 |
| | Negative | 23 | 22 | 25 | 31 | 44 | 25 |
| TNFSF5 | Positive | 27 | 33 | 33 | 33 | 50 | 70 |
| | Negative | 38 | 33 | 0 | 69 | 44 | 25 |
| TNFSF2 | Positive | 21 | 33 | 0 | 17 | 29 | 43 |
| | Negative | 15 | 33 | 25 | 23 | 44 | 0 |
| TNFSF1 | Positive | 18 | 11 | 50 | 14 | 36 | 63 |
| | Negative | 31 | 11 | 25 | 54 | 22 | 25 |
| IL2 | Positive | 36 | 43 | 40 | | | |
| | Negative | 31 | 56 | 25 | | | |

Table 7 Continued- Predictive Responsiveness and Gene Combinations

| 2nd mRNA | FALSE (%)* | TNFSF1 | | | TNFSF2 | | | TNFSF5 | | |
|---|---|---|---|---|---|---|---|---|---|---|
| | | PD | SD | PR | PD | SD | PR | PD | SD | PR |
| IL10 | Positive | 17 | 27 | 0 | 10 | 27 | # | 0 | 27 | # |
| | Negative | 23 | 11 | # | 31 | 11 | 0 | 38 | 11 | 0 |
| TGFB | Positive | 14 | 44 | # | 17 | 29 | # | 44 | 38 | # |
| | Negative | 54 | 44 | # | 23 | 44 | 0 | 62 | 44 | # |
| CTLA4 | Positive | 0 | 0 | # | 14 | 14 | # | 23 | 25 | # |
| | Negative | 23 | 22 | 0 | 8 | 33 | 0 | 23 | 33 | # |
| TNFSF5 | Positive | 22 | 25 | # | 9 | 30 | # | | | |
| | Negative | 46 | 33 | 0 | 23 | 22 | # | | | |
| TNFSF2 | Positive | 17 | 22 | # | | | | | | |
| | Negative | 23 | 22 | 0 | | | | | | |
| TNFSF1 | Positive | | | | | | | | | |
| | Negative | | | | | | | | | |
| IL2 | Positive | | | | | | | | | |
| | Negative | | | | | | | | | |

Table 7 Continued- Predictive Responsiveness and Gene Combinations

| 2nd mRNA | | FALSE (%)* | CTLA4 | | | TGFB | | |
|---|---|---|---|---|---|---|---|---|
| | | | PD | SD | PR | PD | SD | PR |
| IL10 | Positive | | 8 | 22 | # | | | |
| | Negative | | 8 | 22 | # | 31 | 22 | # |
| TGFB | Positive | | 30 | 27 | # | | | |
| | Negative | | 46 | 11 | # | 0 | 42 | # |
| CTLA4 | Positive | | | | | | | |
| | Negative | | | | | | | |
| TNFSF5 | Positive | | | | | | | |
| | Negative | | | | | | | |
| TNFSF2 | Positive | | | | | | | |
| | Negative | | | | | | | |
| TNFSF1 | Positive | | | | | | | |
| | Negative | | | | | | | |
| IL2 | Positive | | | | | | | |
| | Negative | | | | | | | |

## Claims

1. A method for enabling a medical professional to recommend a cancer immunotherapy to a subject comprising:

exposing a first sample of whole blood to a first leukocyte-activating agent in a solvent and exposing a second sample of whole blood to a second leukocyte-activating agent in said solvent, said exposing being for an amount of time sufficient for said leukocyte-activating agents to alter the expression of three or more leukocyte-function-associated mRNAs, wherein said first and second leukocyte-activating agents are associated with immune functions involved in cancer immunotherapy and

wherein said leukocyte-function-associated mRNA is selected from the group consisting of interferon-gamma, tumor necrosis factor superfamily-1, tumor necrosis factor superfamily-2, tumor necrosis factor superfamily-5, interleukin-10, transforming growth factor-beta, CTL-associated protein 4, programmed cell death 1, forkhead box P3, granulocyte macrophage-colony stimulating factor, vascular endothelial growth factor, interleukin-8, CCL chemokine-8, CXCL chemokine-3, and interleukin 2;

exposing a third sample of whole blood to said solvent without said first or second leukocyte-activating agents for said amount of time;

quantifying the expression of said three or more leukocyte-function-associated mRNAs by measuring the amount of mRNA encoding said three or more leukocyte-function-associated mRNAs in said first, second and third whole blood samples;

predetermining a parameter by multivariate analysis for each combination of said first and second leukocyte-activating agents and said three or more leukocyte-function-associated mRNAs using a plurality of patients in one or more of a plurality of groups with known clinical responsiveness to cancer immunotherapy;

calculating a predictor value for each individual for each of said clinical response groups; and

using said predetermined values as a comparative reference for said predictor values to categorize said subject within said clinical response group and enable said medical professional to recommend a cancer immunotherapy for said subject.

2. The method of claim 1, wherein said quantifying comprises

(i) amplifying said three or more leukocyte-function-associated mRNAs using RT-PCR, or
(ii) measuring the induction of said leukocyte-function-associated mRNAs using northern blot.

3. The method of claim 1 or 2, wherein said quantifying comprises amplifying said three or more leukocyte-function-associated mRNAs using real time RT-PCR with primers specifically designed for amplification said three or more leukocyte-function-associated mRNAs.

4. The method of any one of claims 1 to 3, wherein said three or more leukocyte-function-associated mRNAs are associated with different functional immune categories.

5. The method of any one of claims 1 to 4, wherein the cancer immunotherapy comprises a dendritic cell vaccine.

6. The method of any one of claims 1 to 5, wherein the whole blood samples are treated with an anti-coagulant.

7. The method of claim 6, wherein the anti-coagulant comprises heparin.

8. The method of any one of claims 1 to 7, wherein the exposing is performed

(i) at a temperature from about 30°C to about 42°C, or
(ii) at a temperature of about 37°C.

9. The method of any one of claims 1 to 8, wherein said amount of time is less than about 6 hours.

10. The method of any one of claims 1 to 9, wherein said amount of time is from about 1 to about 4 hours.

11. The method of any one of claims 1 to 10, wherein the groups with known clinical responsiveness to cancer immunotherapy are selected from the group consisting of stable disease, partial response, overall survival, and regression free survival.

12. The method of any one of claims 1 to 11, wherein said at least two different leukocyte-activating agents

(i) are selected from the group consisting of phytohemagglutinin, heat-aggregated IgG, zymosan, interleukin 2, interferon alpha-2-beta, a monoclonal antibody against the alpha/beta chain of the human T cell receptor, and picibanil,
(ii) comprise phytohemagglutinin in combination with one or more of interferon alpha-2-beta, heat-aggregated IgG, zymosan, and a monoclonal antibody against the alpha/beta chain of the human T cell receptor, or
(iii) comprise interleukin-2 in combination with one or more of interferon alpha-2-beta, heat-aggregated IgG, zymosan, phytohemagglutinin, and a monoclonal antibody against the alpha/beta chain of the human T cell receptor.

13. The method of claim 1, wherein the recommendation is cancer immunotherapy.

14. The method of any one of claims 1 to 13, wherein said multivariate analysis comprises multivariate discriminant analysis.

**Patentansprüche**

1. Verfahren, das es einem medizinischen Fachmann erlaubt, einem Individuum eine Krebsimmuntherapie zu empfehlen, umfassend:

Exponieren einer ersten Vollblutprobe einem ersten Leukozyten-aktivierenden Agens in einem Lösungsmittel und Exponieren einer zweiten Vollblutprobe gegenüber einem zweiten Leukozyten-aktivierenden Agens in dem Lösungsmittel, wobei das Exponieren für einen Zeitraum vorgenommen wird, der für die Leukozyten-aktivierenden Agenzien ausreichend ist, um die Expression von drei oder mehr Leukozytenfunktion-assoziierten mRNAs zu verändern,

wobei das erste und zweite Leukozyten-aktivierende Agens mit Immunfunktionen assoziiert sind, die in die Krebsimmuntherapie involviert sind und

wobei die Leukozytenfunktion-assoziierte mRNA ausgewählt ist aus der Gruppe, bestehend aus Interferon-gamma, Tumornekrosefaktor-Superfamilie-1, Tumornekrosefaktor-Superfamilie-2, Tumornekrosefaktor-Superfamilie-5, Interleukin-10, transformierendem Wachstumsfaktor-beta, CTL-assoziiertem Protein 4, programmiertem Zelltod 1, Forkhead-Box P3, Granulozyten-Makrophagen-Kolonie-stimulierendem Faktor, vaskulärem endothelialen Wachstumsfaktor, Interleukin-8, CCL Chemokin-8, CXCL Chemokin-3 und Interleukin 2;

Exponieren einer dritten Vollblutprobe dem Lösungsmittel ohne das erste oder zweite Leukozyten-aktivierende Agens für den Zeitraum;

Quantifizieren der Expression der drei oder mehr Leukozytenfunktion-assoziierten mRNAs durch Messung der mRNA-Menge, die die drei oder mehr Leukozytenfunktion-assoziierten mRNAs kodiert, in der ersten, zweiten und drittln Vollblutprobe;

Vorbestimmen eines Parameters durch multivariate Analyse für jede Kombination des ersten und zweiten Leukozyten-aktivierenden Agens und der drei oder mehr Leukozytenfunktion-assoziierten mRNAs unter Verwendung einer Mehrzahl von Patienten in einer oder mehreren einer Mehrzahl von Gruppen mit einem bekannten klinischen Antwortverhalten auf Krebsimmuntherapie;

Berechnen eines Vorhersagewerts für jedes Individuum für jede der klinischen Antwortgruppen; und

Verwenden der vorbestimmten Werte als eine Vergleichsreferenz für die Vorhersagewerte, um das Individuum innerhalb der klinischen Antwortgruppe einzustufen und dem medizinischen Fachmann zu erlauben, dem Individuum eine Krebsimmuntherapie zu empfehlen.

2. Verfahren nach Anspruch 1, wobei die Quantifizierung

(i) Amplifizieren der drei oder mehr Leukozytenfunktion-assoziierten mRNAs mittels RT-PCR, oder

(ii) Messung der Induktion der Leukozytenfunktion-assoziierten mRNAs mittels Northern Blotting

umfasst.

3. Verfahren nach Anspruch 1 oder 2, wobei die Quantifizierung die Amplifikation der drei oder mehr Leukozytenfunktion-assoziierten mRNAs mittels Echtzeit-RT-PCR mit Primern umfasst, die speziell entworfen wurden, um die drei oder mehr Leukozytenfunktion-assoziierten mRNAs zu amplifizieren.

4. Verfahren nach einem der Ansprüche 1 bis 3, wobei die drei oder mehr Leukozytenfunktion-assoziierten mRNAs mit verschiedenen funktionellen Immunkategorien assoziiert sind.

5. Verfahren nach einem der Ansprüche 1 bis 4, wobei die Krebsimmuntherapie ein dendritische Zellen-Vakzin umfasst.

6. Verfahren nach einem der Ansprüche 1 bis 5, wobei die Vollblutproben mit einem Antikoagulans behandelt werden.

7. Verfahren nach Anspruch 6, wobei das Antikoagulans Heparin umfasst.

8. Verfahren nach einem der Ansprüche 1 bis 7, wobei das Exponieren

(i) bei einer Temperatur von etwa 30°C bis etwa 42°C, oder

(ii) bei einer Temperatur von etwa 37°C

durchgeführt wird.

9. Verfahren nach einem der Ansprüche 1 bis 8, wobei der Zeitraum weniger als etwa 6 Stunden ist.

10. Verfahren nach einem der Ansprüche 1 bis 9, wobei der Zeitraum von etwa 1 bis etwa 4 Stunden ist.

11. Verfahren nach einem der Ansprüche 1 bis 10, wobei die Gruppen mit einem bekannten klinischen Antwortverhalten auf eine Krebsimmuntherapie ausgewählt sind aus der Gruppe bestehend aus stabiler Krankheit, teilweiser Antwort, Gesamtüberleben und regressionsfreiem Überleben.

12. Verfahren nach einem der Ansprüche 1 bis 11, wobei die mindestens zwei verschiedenen Leukozyten-aktivierenden Agenzien

(i) ausgewählt sind aus der Gruppe bestehend aus Phytohämagglutinin, Hitze-aggregiertem IgG, Zymosan, Interleukin-2, Interferon alpha-2-beta, einem monoklonalen Antikörper gegen die alpha/beta Kette des menschlichen T-Zell-Rezeptors und Picibanil,

(ii) Phytohämagglutinin in Kombination mit einer oder mehreren Substanzen aus Interferon alpha-2-beta, Hitze-aggregiertem IgG, Zymosan und einem monoklonalen Antikörper gegen die alpha/beta Kette des menschlichen T-Zell-Rezeptors umfassen, oder

(iii) Interleukin-2 in Kombination mit einer oder mehreren Substanzen aus Interferon alpha-2-beta, Hitze-aggregiertem IgG, Zymosan, Phytohämagglutinin und einem monoklonalen Antikörper gegen die alpha/beta Kette des menschlichen T-Zell-Rezeptors umfassen.

13. Verfahren nach Anspruch 1, wobei die Empfehlung Krebsimmuntherapie ist.

14. Verfahren nach einem der Ansprüche 1 bis 13, wobei die multivariate Analyse multivariate Diskriminanzanalyse umfasst.

## Revendications

1. Méthode permettant au corps médical de recommander une immunothérapie anticancéreuse à un sujet comprenant :

l'exposition d'un premier échantillon de sang entier à un premier agent d'activation leucocytaire dans un solvant et l'exposition d'un deuxième échantillon de sang entier à un second agent d'activation leucocytaire dans ledit solvant, ladite exposition étant mise en oeuvre pendant une durée suffisante pour que lesdits agents d'activation leucocytaire altèrent l'expression de trois ARNm ou plus associés aux fonctions leucocytaires, dans laquelle lesdits premier et second agents d'activation leucocytaire sont associés aux fonctions immunitaires impliquées en immunothérapie anticancéreuse et

dans laquelle ledit ARNm associé aux fonctions leucocytaires est choisi dans le groupe constitué par l'interféron gamma, la superfamille 1 du facteur de nécrose tumorale, la superfamille 2 du facteur de nécrose tumorale, la superfamille 5 du facteur de nécrose tumorale, l'interleukine 10, le facteur de croissance transformant bêta, la protéine 4 associée aux CTL, le gène de mort cellulaire programmée 1, le gène forkhead box P3, le facteur de stimulation des colonies de granulocytes-macrophages, le facteur de croissance endothélial vasculaire, l'interleukine 8, la chimiokine CCL8, la chimiokine CXCL3 et l'interleukine 2 ;

l'exposition d'un troisième échantillon de sang entier audit solvant en l'absence desdits premier ou second agents d'activation leucocytaire pendant ladite durée ;

la quantification de l'expression desdits trois ARNm ou plus associés aux fonctions leucocytaires par mesure de la quantité d'ARNm codant pour lesdits trois ARNm ou plus associés aux fonctions leucocytaires dans lesdits premier, deuxième et troisième échantillons de sang entier ;

la prédétermination d'un paramètre par une analyse à plusieurs variables pour chaque combinaison desdits premier et second agents d'activation leucocytaire et desdits trois ARNm ou plus associés aux fonctions leucocytaires à l'aide d'une pluralité de patients dans un ou plusieurs groupes d'une pluralité de groupes ayant une sensibilité clinique connue à l'immunothérapie anticancéreuse ;

le calcul d'une valeur de prédiction pour chaque individu dans chacun desdits groupes de réponses cliniques ; et

l'utilisation desdites valeurs prédéterminées à titre de référence de comparaison pour lesdites valeurs de prédiction dans le but de catégoriser ledit sujet dans ledit groupe de réponse clinique et de permettre audit corps médical de recommander une immunothérapie anticancéreuse pour ledit sujet.

2. Méthode selon la revendication 1, dans laquelle ladite quantification comprend

(i) l'amplification desdits trois ARNm ou plus associés aux fonctions leucocytaires par RT-PCR, ou

(ii) la mesure de l'induction desdits ARNm associés aux fonctions leucocytaires par transfert Northern (northern blot).

3. Méthode selon la revendication 1 ou 2, dans laquelle ladite quantification comprend l'amplification desdits trois ARNm ou plus associés aux fonctions leucocytaires par RT-PCR en temps réel à l'aide d'amorces spécifiquement conçues pour amplifier lesdits trois ARNm ou plus associés aux fonctions leucocytaires.

4. Méthode selon l'une quelconque des revendications 1 à 3, dans laquelle lesdits trois ARNm ou plus associés aux fonctions leucocytaires sont associés à des catégories fonctionnelles immunitaires différentes.

**5.** Méthode selon l'une quelconque des revendications 1 à 4, dans laquelle l'immunothérapie anticancéreuse comprend un vaccin à base de cellules dendritiques.

**6.** Méthode selon l'une quelconque des revendications 1 à 5, dans laquelle les échantillons de sang entier sont traités avec un anticoagulant.

**7.** Méthode selon la revendication 6, dans laquelle l'anticoagulant comprend l'héparine.

**8.** Méthode selon l'une quelconque des revendications 1 à 7, dans laquelle l'exposition est mise en oeuvre

(i) à une température d'environ 30 °C à environ 42 °C, ou
(ii) à une température d'environ 37 °C.

**9.** Méthode selon l'une quelconque des revendications 1 à 8, dans laquelle ladite durée est inférieure à environ 6 heures.

**10.** Méthode selon l'une quelconque des revendications 1 à 9, dans laquelle ladite durée est d'environ 1 à environ 4 heures.

**11.** Méthode selon l'une quelconque des revendications 1 à 10, dans laquelle les groupes ayant une sensibilité clinique connue à l'immunothérapie anticancéreuse sont choisis dans le groupe constitué par : maladie stable, réponse partielle, survie globale, et survie sans régression.

**12.** Méthode selon l'une quelconque des revendications 1 à 11, dans laquelle lesdits au moins deux agents d'activation leucocytaire différents

(i) sont choisis dans le groupe constitué par la phytohémagglutinine, les IgG thermo-agrégées, le zymosane, l'interleukine 2, l'interféron alpha-2-bêta, un anticorps monoclonal dirigé contre la chaîne alpha/bêta du récepteur humain des cellules T, et le picibanil,
(ii) comprennent la phytohémagglutinine en association avec un ou plusieurs agents parmi l'interféron alpha-2-bêta, les IgG thermo-agrégées, le zymosane, et un anticorps monoclonal dirigé contre la chaîne alpha/bêta du récepteur humain de cellules T, ou
(iii) comprennent l'interleukine 2 en association avec un ou plusieurs agents parmi l'interféron alpha-2-bêta, les IgG thermo-agrégées, le zymosane, la phytohémagglutinine, et un anticorps monoclonal dirigé contre la chaîne alpha/bêta du récepteur humain de cellules T.

**13.** Méthode selon la revendication 1, dans laquelle la recommandation est l'immunothérapie anticancéreuse.

**14.** Méthode selon l'une quelconque des revendications 1 à 13, dans laquelle ladite analyse à plusieurs variables comprend l'analyse discriminante à plusieurs variables.

*FIG. 1A*

*FIG. 1B*

FIG. 1C

FIG. 1D

*FIG. 1E*

*FIG. 1F*

**FIG. 1G**

**FIG. 1H**

FIG. 1I

FIG. 1J

FIG. 1K

FIG. 2A

FIG. 2B

FIG. 2C

FIG. 2D

FIG. 2E

FIG. 2F

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011084333 A
- WO 2006133399 A
- WO 2006116721 A
- WO 2009070442 A
- WO 2009083653 A
- US 7745180 B

- US 7968288 B
- US 7939300 B
- US 11376018 B
- US 11803594 B
- US 11803663 B

**Non-patent literature cited in the description**

- **MITSUHASHI.** *Journal of Immunological Methods,* 2010, vol. 363, 95-100